# EUROPEAN PATENT APPLICATION

(11) **EP 1 764 108 A1**
(43) Date of publication of application: **21.03.2007**
(21) Application number: 05020020.3
(22) Date of filing: 14.09.2005
(51) Int. Cl.: A61K 39/39

(54) **Compositions comprising immunostimulatory RNA oligonucleotides and methods for producing said RNA oligonucleotides**

(71) Applicant: Hartmann, Gunther, 81479 München (DE)
(72) Inventor: Hartmann, Gunther, 53347 Alfter (Impekoven) (DE); Hornung, Veit, Dr., 82049 Pullach (DE)
(74) Representative: Grünecker, Kinkeldey, Stockmair & Schwanhäusser Anwaltssozietät

(57) **Abstract**

The present invention provides 4-nucleotide (4mer) RNA motifs that confer immunostimulatory activity, in particular, IFN-α-inducing activity to a RNA oligonucleotide. The present invention also provides RNA oligonucleotides, including siRNA, with high or low immunostimulatory activity. The present invention further provides the use of the RNA oligonucleotides of the invention for therapeutic purposes.

### Annex to the application documents - subsequently filed sequences listing

SEQUENCE LISTING

<110> Hartmann, Gunther

<120> Compositions and methods for immunostimulatory RNA
oligonucleotides (IFN-alpha)

<130> EP36357FZ121

<140> EP 05020002.3
<141> 2005-14-09

<160> 390

<170> PatentIn version 3.3

<210> 1
<211> 19
<212> RNA
<213> Artificial

<220>
<223> randomized 4 mer motifs on poly A backbone

<400> 1
aaaaaaaaaa aaaaaaaaa 19

<210> 2
<211> 19
<212> RNA
<213> Artificial

<220>
<223> randomized 4 mer motifs on poly A backbone

<400> 2
aaaaaaaaca aaaaaaaaa 19

<210> 3
<211> 19
<212> RNA
<213> Artificial

<220>
<223> randomized 4 mer motifs on poly A backbone

<400> 3
aaaaaaaaga aaaaaaaaa 19

<210> 4
<211> 19
<212> RNA
<213> Artificial

<220>
<223> randomized 4 mer motifs on poly A backbone

<400> 4
aaaaaaaaua aaaaaaaaa 19

<210>  5
<211>  19
<212>  RNA
<213>  Artificial

<220>
<223>  randomized 4 mer motifs on poly A backbone

<400>  5
aaaaaaaacc aaaaaaaaa                                                    19


<210>  6
<211>  19
<212>  RNA
<213>  Artificial

<220>
<223>  randomized 4 mer motifs on poly A backbone

<400>  6
aaaaaaaacg aaaaaaaaa                                                    19


<210>  7
<211>  19
<212>  RNA
<213>  Artificial

<220>
<223>  randomized 4 mer motifs on poly A backbone

<400>  7
aaaaaaaacu aaaaaaaaa                                                    19


<210>  8
<211>  19
<212>  RNA
<213>  Artificial

<220>
<223>  randomized 4 mer motifs on poly A backbone

<400>  8
aaaaaaaagc aaaaaaaaa                                                    19


<210>  9
<211>  19
<212>  RNA
<213>  Artificial

<220>
<223>  randomized 4 mer motifs on poly A backbone

<400>  9
aaaaaaaagg aaaaaaaaa                                                    19


<210>  10
<211>  19
<212>  RNA
<213>  Artificial

<220>
<223>  randomized 4 mer motifs on poly A backbone

<400>  10
aaaaaaaagu aaaaaaaaa                                                    19


<210>  11
<211>  19
<212>  RNA
<213>  Artificial

<220>
<223>  randomized 4 mer motifs on poly A backbone

<400>  11
aaaaaaaauc aaaaaaaaa                                                    19


<210>  12
<211>  19
<212>  RNA
<213>  Artificial

<220>
<223>  randomized 4 mer motifs on poly A backbone

<400>  12
aaaaaaaaug aaaaaaaaa                                                    19


<210>  13
<211>  19
<212>  RNA
<213>  Artificial

<220>
<223>  randomized 4 mer motifs on poly A backbone

<400>  13
aaaaaaaauu aaaaaaaaa                                                    19


<210>  14
<211>  19
<212>  RNA
<213>  Artificial

<220>
<223>  randomized 4 mer motifs on poly A backbone

<400>  14
aaaaaaaaca caaaaaaaa                                                    19


<210>  15
<211>  19
<212>  RNA
<213>  Artificial

<220>
<223>  randomized 4 mer motifs on poly A backbone

<400>  15
aaaaaaaaca gaaaaaaaa                                                    19


<210>  16
<211>  19
<212>  RNA
<213>  Artificial

<220>
<223>  randomized 4 mer motifs on poly A backbone

<400>  16
aaaaaaaaca uaaaaaaaa                                                    19


<210>  17
<211>  19
<212>  RNA
<213>  Artificial

<220>
<223>  randomized 4 mer motifs on poly A backbone

<400>  17
aaaaaaaacc caaaaaaaa                                                    19


<210>  18
<211>  19
<212>  RNA
<213>  Artificial

<220>
<223>  randomized 4 mer motifs on poly A backbone

<400>  18
aaaaaaaacc gaaaaaaaa                                                    19


<210>  19
<211>  19
<212>  RNA
<213>  Artificial

<220>
<223>  randomized 4 mer motifs on poly A backbone

<400>  19
aaaaaaaacc uaaaaaaaa                                                    19


<210>  20
<211>  19
<212>  RNA
<213>  Artificial

<220>
<223>  randomized 4 mer motifs on poly A backbone

<400>  20
aaaaaaaacg caaaaaaaa                                                    19

<210>  21
<211>  19
<212>  RNA
<213>  Artificial

<220>
<223>  randomized 4 mer motifs on poly A backbone

<400>  21
aaaaaaaacg gaaaaaaaa                                                    19


<210>  22
<211>  19
<212>  RNA
<213>  Artificial

<220>
<223>  randomized 4 mer motifs on poly A backbone

<400>  22
aaaaaaaacg uaaaaaaaa                                                    19


<210>  23
<211>  19
<212>  RNA
<213>  Artificial

<220>
<223>  randomized 4 mer motifs on poly A backbone

<400>  23
aaaaaaaacu caaaaaaaa                                                    19


<210>  24
<211>  19
<212>  RNA
<213>  Artificial

<220>
<223>  randomized 4 mer motifs on poly A backbone

<400>  24
aaaaaaaacu gaaaaaaaa                                                    19


<210>  25
<211>  19
<212>  RNA
<213>  Artificial

<220>
<223>  randomized 4 mer motifs on poly A backbone

<400>  25
aaaaaaaacu uaaaaaaaa                                                    19


<210>  26
<211>  19
<212>  RNA
<213>  Artificial

<220>
<223>  randomized 4 mer motifs on poly A backbone

<400>  26
aaaaaaaaga caaaaaaaa                                                    19


<210>  27
<211>  19
<212>  RNA
<213>  Artificial

<220>
<223>  randomized 4 mer motifs on poly A backbone

<400>  27
aaaaaaaaga gaaaaaaaa                                                    19


<210>  28
<211>  19
<212>  RNA
<213>  Artificial

<220>
<223>  randomized 4 mer motifs on poly A backbone

<400>  28
aaaaaaaaga uaaaaaaaa                                                    19


<210>  29
<211>  19
<212>  RNA
<213>  Artificial

<220>
<223>  randomized 4 mer motifs on poly A backbone

<400>  29
aaaaaaaagc caaaaaaaa                                                    19


<210>  30
<211>  19
<212>  RNA
<213>  Artificial

<220>
<223>  randomized 4 mer motifs on poly A backbone

<400>  30
aaaaaaaagc gaaaaaaaa                                                    19


<210>  31
<211>  19
<212>  RNA
<213>  Artificial

<220>
<223>  randomized 4 mer motifs on poly A backbone

<400>  31
aaaaaaaagc uaaaaaaaa                                                    19


<210>  32
<211>  19
<212>  RNA
<213>  Artificial

<220>
<223>  randomized 4 mer motifs on poly A backbone

<400>  32
aaaaaaaagg caaaaaaaa                                                    19


<210>  33
<211>  19
<212>  RNA
<213>  Artificial

<220>
<223>  randomized 4 mer motifs on poly A backbone

<400>  33
aaaaaaaagg gaaaaaaaa                                                    19


<210>  34
<211>  19
<212>  RNA
<213>  Artificial

<220>
<223>  randomized 4 mer motifs on poly A backbone

<400>  34
aaaaaaaagg uaaaaaaaa                                                    19


<210>  35
<211>  19
<212>  RNA
<213>  Artificial

<220>
<223>  randomized 4 mer motifs on poly A backbone

<400>  35
aaaaaaaagu caaaaaaaa                                                    19


<210>  36
<211>  19
<212>  RNA
<213>  Artificial

<220>
<223>  randomized 4 mer motifs on poly A backbone

<400>  36
aaaaaaaagu gaaaaaaaa                                                    19

<210>  37
<211>  19
<212>  RNA
<213>  Artificial

<220>
<223>  randomized 4 mer motifs on poly A backbone

<400>  37
aaaaaaaagu uaaaaaaaa                                                    19


<210>  38
<211>  19
<212>  RNA
<213>  Artificial

<220>
<223>  randomized 4 mer motifs on poly A backbone

<400>  38
aaaaaaaaua caaaaaaaa                                                    19


<210>  39
<211>  19
<212>  RNA
<213>  Artificial

<220>
<223>  randomized 4 mer motifs on poly A backbone

<400>  39
aaaaaaaaua gaaaaaaaa                                                    19


<210>  40
<211>  19
<212>  RNA
<213>  Artificial

<220>
<223>  randomized 4 mer motifs on poly A backbone

<400>  40
aaaaaaaaua uaaaaaaaa                                                    19


<210>  41
<211>  19
<212>  RNA
<213>  Artificial

<220>
<223>  randomized 4 mer motifs on poly A backbone

<400>  41
aaaaaaaauc caaaaaaaa                                                    19


<210>  42
<211>  19
<212>  RNA
<213>  Artificial

<220>
<223>  randomized 4 mer motifs on poly A backbone

<400>  42
aaaaaaaauc gaaaaaaaa                                                    19


<210>  43
<211>  19
<212>  RNA
<213>  Artificial

<220>
<223>  randomized 4 mer motifs on poly A backbone

<400>  43
aaaaaaaauc uaaaaaaaa                                                    19


<210>  44
<211>  19
<212>  RNA
<213>  Artificial

<220>
<223>  randomized 4 mer motifs on poly A backbone

<400>  44
aaaaaaaaug caaaaaaaa                                                    19


<210>  45
<211>  19
<212>  RNA
<213>  Artificial

<220>
<223>  randomized 4 mer motifs on poly A backbone

<400>  45
aaaaaaaaug gaaaaaaaa                                                    19


<210>  46
<211>  19
<212>  RNA
<213>  Artificial

<220>
<223>  randomized 4 mer motifs on poly A backbone

<400>  46
aaaaaaaaug uaaaaaaaa                                                    19


<210>  47
<211>  19
<212>  RNA
<213>  Artificial

<220>
<223>  randomized 4 mer motifs on poly A backbone

<400>  47
aaaaaaaauu caaaaaaaa                                                    19


<210>  48
<211>  19
<212>  RNA
<213>  Artificial

<220>
<223>  randomized 4 mer motifs on poly A backbone

<400>  48
aaaaaaaauu gaaaaaaaa                                                    19


<210>  49
<211>  19
<212>  RNA
<213>  Artificial

<220>
<223>  randomized 4 mer motifs on poly A backbone

<400>  49
aaaaaaaauu uaaaaaaaa                                                    19


<210>  50
<211>  19
<212>  RNA
<213>  Artificial

<220>
<223>  randomized 4 mer motifs on poly A backbone

<400>  50
aaaaaaacaa caaaaaaaa                                                    19


<210>  51
<211>  19
<212>  RNA
<213>  Artificial

<220>
<223>  randomized 4 mer motifs on poly A backbone

<400>  51
aaaaaaacaa gaaaaaaaa                                                    19


<210>  52
<211>  19
<212>  RNA
<213>  Artificial

<220>
<223>  randomized 4 mer motifs on poly A backbone

<400>  52
aaaaaaacaa uaaaaaaaa                                                    19

<210>  53
<211>  19
<212>  RNA
<213>  Artificial

<220>
<223>  randomized 4 mer motifs on poly A backbone

<400>  53
aaaaaaacac caaaaaaaa                                                    19


<210>  54
<211>  19
<212>  RNA
<213>  Artificial

<220>
<223>  randomized 4 mer motifs on poly A backbone

<400>  54
aaaaaaacac gaaaaaaaa                                                    19


<210>  55
<211>  19
<212>  RNA
<213>  Artificial

<220>
<223>  randomized 4 mer motifs on poly A backbone

<400>  55
aaaaaaacac uaaaaaaaa                                                    19


<210>  56
<211>  19
<212>  RNA
<213>  Artificial

<220>
<223>  randomized 4 mer motifs on poly A backbone

<400>  56
aaaaaaacag caaaaaaaa                                                    19


<210>  57
<211>  19
<212>  RNA
<213>  Artificial

<220>
<223>  randomized 4 mer motifs on poly A backbone

<400>  57
aaaaaaacag gaaaaaaaa                                                    19


<210>  58
<211>  19
<212>  RNA
<213>  Artificial

<220>
<223>  randomized 4 mer motifs on poly A backbone

<400>  58
aaaaaaacag uaaaaaaaa                                                    19


<210>  59
<211>  19
<212>  RNA
<213>  Artificial

<220>
<223>  randomized 4 mer motifs on poly A backbone

<400>  59
aaaaaaacau caaaaaaaa                                                    19


<210>  60
<211>  19
<212>  RNA
<213>  Artificial

<220>
<223>  randomized 4 mer motifs on poly A backbone

<400>  60
aaaaaaacau gaaaaaaaa                                                    19


<210>  61
<211>  19
<212>  RNA
<213>  Artificial

<220>
<223>  randomized 4 mer motifs on poly A backbone

<400>  61
aaaaaaacau uaaaaaaaa                                                    19


<210>  62
<211>  19
<212>  RNA
<213>  Artificial

<220>
<223>  randomized 4 mer motifs on poly A backbone

<400>  62
aaaaaaacca caaaaaaaa                                                    19


<210>  63
<211>  19
<212>  RNA
<213>  Artificial

<220>
<223>  randomized 4 mer motifs on poly A backbone

<400>  63
aaaaaaacca gaaaaaaaa                                                    19


<210>  64
<211>  19
<212>  RNA
<213>  Artificial

<220>
<223>  randomized 4 mer motifs on poly A backbone

<400>  64
aaaaaaacca uaaaaaaaa                                                    19


<210>  65
<211>  19
<212>  RNA
<213>  Artificial

<220>
<223>  randomized 4 mer motifs on poly A backbone

<400>  65
aaaaaaaccc caaaaaaaa                                                    19


<210>  66
<211>  19
<212>  RNA
<213>  Artificial

<220>
<223>  randomized 4 mer motifs on poly A backbone

<400>  66
aaaaaaaccc gaaaaaaaa                                                    19


<210>  67
<211>  19
<212>  RNA
<213>  Artificial

<220>
<223>  randomized 4 mer motifs on poly A backbone

<400>  67
aaaaaaaccc uaaaaaaaa                                                    19


<210>  68
<211>  19
<212>  RNA
<213>  Artificial

<220>
<223>  randomized 4 mer motifs on poly A backbone

<400>  68
aaaaaaaccg caaaaaaaa                                                    19

<210>  69
<211>  19
<212>  RNA
<213>  Artificial

<220>
<223>  randomized 4 mer motifs on poly A backbone

<400>  69
aaaaaaaccg gaaaaaaaa                                                    19


<210>  70
<211>  19
<212>  RNA
<213>  Artificial

<220>
<223>  randomized 4 mer motifs on poly A backbone

<400>  70
aaaaaaaccg uaaaaaaaa                                                    19


<210>  71
<211>  19
<212>  RNA
<213>  Artificial

<220>
<223>  randomized 4 mer motifs on poly A backbone

<400>  71
aaaaaaaccu caaaaaaaa                                                    19


<210>  72
<211>  19
<212>  RNA
<213>  Artificial

<220>
<223>  randomized 4 mer motifs on poly A backbone

<400>  72
aaaaaaaccu gaaaaaaaa                                                    19


<210>  73
<211>  19
<212>  RNA
<213>  Artificial

<220>
<223>  randomized 4 mer motifs on poly A backbone

<400>  73
aaaaaaaccu uaaaaaaaa                                                    19


<210>  74
<211>  19
<212>  RNA
<213>  Artificial

<220>
<223>  randomized 4 mer motifs on poly A backbone

<400>  74
aaaaaaacga caaaaaaaa                                                    19


<210>  75
<211>  19
<212>  RNA
<213>  Artificial

<220>
<223>  randomized 4 mer motifs on poly A backbone

<400>  75
aaaaaaacga gaaaaaaaa                                                    19


<210>  76
<211>  19
<212>  RNA
<213>  Artificial

<220>
<223>  randomized 4 mer motifs on poly A backbone

<400>  76
aaaaaaacga uaaaaaaaa                                                    19


<210>  77
<211>  19
<212>  RNA
<213>  Artificial

<220>
<223>  randomized 4 mer motifs on poly A backbone

<400>  77
aaaaaaacgc caaaaaaaa                                                    19


<210>  78
<211>  19
<212>  RNA
<213>  Artificial

<220>
<223>  randomized 4 mer motifs on poly A backbone

<400>  78
aaaaaaacgc gaaaaaaaa                                                    19


<210>  79
<211>  19
<212>  RNA
<213>  Artificial

<220>
<223>  randomized 4 mer motifs on poly A backbone

<400>  79
aaaaaaacgc uaaaaaaaa                                                    19


<210>  80
<211>  19
<212>  RNA
<213>  Artificial

<220>
<223>  randomized 4 mer motifs on poly A backbone

<400>  80
aaaaaaacgg caaaaaaaa                                                    19


<210>  81
<211>  19
<212>  RNA
<213>  Artificial

<220>
<223>  randomized 4 mer motifs on poly A backbone

<400>  81
aaaaaaacgg gaaaaaaaa                                                    19


<210>  82
<211>  19
<212>  RNA
<213>  Artificial

<220>
<223>  randomized 4 mer motifs on poly A backbone

<400>  82
aaaaaaacgg uaaaaaaaa                                                    19


<210>  83
<211>  19
<212>  RNA
<213>  Artificial

<220>
<223>  randomized 4 mer motifs on poly A backbone

<400>  83
aaaaaaacgu caaaaaaaa                                                    19


<210>  84
<211>  19
<212>  RNA
<213>  Artificial

<220>
<223>  randomized 4 mer motifs on poly A backbone

<400>  84
aaaaaaacgu gaaaaaaaa                                                    19

<210>  85
<211>  19
<212>  RNA
<213>  Artificial

<220>
<223>  randomized 4 mer motifs on poly A backbone

<400>  85
aaaaaaacgu uaaaaaaaa                                                    19


<210>  86
<211>  19
<212>  RNA
<213>  Artificial

<220>
<223>  randomized 4 mer motifs on poly A backbone

<400>  86
aaaaaaacua caaaaaaaa                                                    19


<210>  87
<211>  19
<212>  RNA
<213>  Artificial

<220>
<223>  randomized 4 mer motifs on poly A backbone

<400>  87
aaaaaaacua gaaaaaaaa                                                    19


<210>  88
<211>  19
<212>  RNA
<213>  Artificial

<220>
<223>  randomized 4 mer motifs on poly A backbone

<400>  88
aaaaaaacua uaaaaaaaa                                                    19


<210>  89
<211>  19
<212>  RNA
<213>  Artificial

<220>
<223>  randomized 4 mer motifs on poly A backbone

<400>  89
aaaaaaacuc caaaaaaaa                                                    19


<210>  90
<211>  19
<212>  RNA
<213>  Artificial

<220>
<223>  randomized 4 mer motifs on poly A backbone

<400>  90
aaaaaaacuc gaaaaaaaa                                                    19


<210>  91
<211>  19
<212>  RNA
<213>  Artificial

<220>
<223>  randomized 4 mer motifs on poly A backbone

<400>  91
aaaaaaacuc uaaaaaaaa                                                    19


<210>  92
<211>  19
<212>  RNA
<213>  Artificial

<220>
<223>  randomized 4 mer motifs on poly A backbone

<400>  92
aaaaaaacug caaaaaaaa                                                    19


<210>  93
<211>  19
<212>  RNA
<213>  Artificial

<220>
<223>  randomized 4 mer motifs on poly A backbone

<400>  93
aaaaaaacug gaaaaaaaa                                                    19


<210>  94
<211>  19
<212>  RNA
<213>  Artificial

<220>
<223>  randomized 4 mer motifs on poly A backbone

<400>  94
aaaaaaacug uaaaaaaaa                                                    19


<210>  95
<211>  19
<212>  RNA
<213>  Artificial

<220>
<223>  randomized 4 mer motifs on poly A backbone

<400>  95
aaaaaaacuu caaaaaaaa                                                    19


<210>  96
<211>  19
<212>  RNA
<213>  Artificial

<220>
<223>  randomized 4 mer motifs on poly A backbone

<400>  96
aaaaaaacuu gaaaaaaaa                                                    19


<210>  97
<211>  19
<212>  RNA
<213>  Artificial

<220>
<223>  randomized 4 mer motifs on poly A backbone

<400>  97
aaaaaaacuu uaaaaaaaa                                                    19


<210>  98
<211>  19
<212>  RNA
<213>  Artificial

<220>
<223>  randomized 4 mer motifs on poly A backbone

<400>  98
aaaaaaagaa caaaaaaaa                                                    19


<210>  99
<211>  19
<212>  RNA
<213>  Artificial

<220>
<223>  randomized 4 mer motifs on poly A backbone

<400>  99
aaaaaaagaa gaaaaaaaa                                                    19


<210>  100
<211>  19
<212>  RNA
<213>  Artificial

<220>
<223>  randomized 4 mer motifs on poly A backbone

<400>  100
aaaaaaagaa uaaaaaaaa                                                    19

<210>  101
<211>  19
<212>  RNA
<213>  Artificial

<220>
<223>  randomized 4 mer motifs on poly A backbone

<400>  101
aaaaaaagac caaaaaaaa                                                    19


<210>  102
<211>  19
<212>  RNA
<213>  Artificial

<220>
<223>  randomized 4 mer motifs on poly A backbone

<400>  102
aaaaaaagac gaaaaaaaa                                                    19


<210>  103
<211>  19
<212>  RNA
<213>  Artificial

<220>
<223>  randomized 4 mer motifs on poly A backbone

<400>  103
aaaaaaagac uaaaaaaaa                                                    19


<210>  104
<211>  19
<212>  RNA
<213>  Artificial

<220>
<223>  randomized 4 mer motifs on poly A backbone

<400>  104
aaaaaaagag caaaaaaaa                                                    19


<210>  105
<211>  19
<212>  RNA
<213>  Artificial

<220>
<223>  randomized 4 mer motifs on poly A backbone

<400>  105
aaaaaaagag gaaaaaaaa                                                    19


<210>  106
<211>  19
<212>  RNA
<213>  Artificial

<220>
<223>  randomized 4 mer motifs on poly A backbone

<400>  106
aaaaaaagag uaaaaaaaa                                                    19


<210>  107
<211>  19
<212>  RNA
<213>  Artificial

<220>
<223>  randomized 4 mer motifs on poly A backbone

<400>  107
aaaaaaagau caaaaaaaa                                                    19


<210>  108
<211>  19
<212>  RNA
<213>  Artificial

<220>
<223>  randomized 4 mer motifs on poly A backbone

<400>  108
aaaaaaagau gaaaaaaaa                                                    19


<210>  109
<211>  19
<212>  RNA
<213>  Artificial

<220>
<223>  randomized 4 mer motifs on poly A backbone

<400>  109
aaaaaaagau uaaaaaaaa                                                    19


<210>  110
<211>  19
<212>  RNA
<213>  Artificial

<220>
<223>  randomized 4 mer motifs on poly A backbone

<400>  110
aaaaaaagca caaaaaaaa                                                    19


<210>  111
<211>  19
<212>  RNA
<213>  Artificial

<220>
<223>  randomized 4 mer motifs on poly A backbone

<400>  111
aaaaaaagca gaaaaaaaa                                                    19


<210>  112
<211>  19
<212>  RNA
<213>  Artificial

<220>
<223>  randomized 4 mer motifs on poly A backbone

<400>  112
aaaaaaagca uaaaaaaaa                                                    19


<210>  113
<211>  19
<212>  RNA
<213>  Artificial

<220>
<223>  randomized 4 mer motifs on poly A backbone

<400>  113
aaaaaaagcc caaaaaaaa                                                    19


<210>  114
<211>  19
<212>  RNA
<213>  Artificial

<220>
<223>  randomized 4 mer motifs on poly A backbone

<400>  114
aaaaaaagcc gaaaaaaaa                                                    19


<210>  115
<211>  19
<212>  RNA
<213>  Artificial

<220>
<223>  randomized 4 mer motifs on poly A backbone

<400>  115
aaaaaaagcc uaaaaaaaa                                                    19


<210>  116
<211>  19
<212>  RNA
<213>  Artificial

<220>
<223>  randomized 4 mer motifs on poly A backbone

<400>  116
aaaaaaagcg caaaaaaaa                                                    19

<210>  117
<211>  19
<212>  RNA
<213>  Artificial

<220>
<223>  randomized 4 mer motifs on poly A backbone

<400>  117
aaaaaaagcg gaaaaaaaa                                                    19


<210>  118
<211>  19
<212>  RNA
<213>  Artificial

<220>
<223>  randomized 4 mer motifs on poly A backbone

<400>  118
aaaaaaagcg uaaaaaaaa                                                    19


<210>  119
<211>  19
<212>  RNA
<213>  Artificial

<220>
<223>  randomized 4 mer motifs on poly A backbone

<400>  119
aaaaaaagcu caaaaaaaa                                                    19


<210>  120
<211>  19
<212>  RNA
<213>  Artificial

<220>
<223>  randomized 4 mer motifs on poly A backbone

<400>  120
aaaaaaagcu gaaaaaaaa                                                    19


<210>  121
<211>  19
<212>  RNA
<213>  Artificial

<220>
<223>  randomized 4 mer motifs on poly A backbone

<400>  121
aaaaaaagcu uaaaaaaaa                                                    19


<210>  122
<211>  19
<212>  RNA
<213>  Artificial

<220>
<223>  randomized 4 mer motifs on poly A backbone

<400>  122
aaaaaaagga caaaaaaaa                                                    19


<210>  123
<211>  19
<212>  RNA
<213>  Artificial

<220>
<223>  randomized 4 mer motifs on poly A backbone

<400>  123
aaaaaaagga gaaaaaaaa                                                    19


<210>  124
<211>  19
<212>  RNA
<213>  Artificial

<220>
<223>  randomized 4 mer motifs on poly A backbone

<400>  124
aaaaaaagga uaaaaaaaa                                                    19


<210>  125
<211>  19
<212>  RNA
<213>  Artificial

<220>
<223>  randomized 4 mer motifs on poly A backbone

<400>  125
aaaaaaaggc caaaaaaaa                                                    19


<210>  126
<211>  19
<212>  RNA
<213>  Artificial

<220>
<223>  randomized 4 mer motifs on poly A backbone

<400>  126
aaaaaaaggc gaaaaaaaa                                                    19


<210>  127
<211>  19
<212>  RNA
<213>  Artificial

<220>
<223>  randomized 4 mer motifs on poly A backbone

<400>  127
aaaaaaaggc uaaaaaaaa                                                    19


<210>  128
<211>  19
<212>  RNA
<213>  Artificial

<220>
<223>  randomized 4 mer motifs on poly A backbone

<400>  128
aaaaaaaggg caaaaaaaa                                                    19


<210>  129
<211>  19
<212>  RNA
<213>  Artificial

<220>
<223>  randomized 4 mer motifs on poly A backbone

<400>  129
aaaaaaaggg gaaaaaaaa                                                    19


<210>  130
<211>  19
<212>  RNA
<213>  Artificial

<220>
<223>  randomized 4 mer motifs on poly A backbone

<400>  130
aaaaaaaggg uaaaaaaaa                                                    19


<210>  131
<211>  19
<212>  RNA
<213>  Artificial

<220>
<223>  randomized 4 mer motifs on poly A backbone

<400>  131
aaaaaaaggu caaaaaaaa                                                    19


<210>  132
<211>  19
<212>  RNA
<213>  Artificial

<220>
<223>  randomized 4 mer motifs on poly A backbone

<400>  132
aaaaaaaggu gaaaaaaaa                                                    19

<210>  133
<211>  19
<212>  RNA
<213>  Artificial

<220>
<223>  randomized 4 mer motifs on poly A backbone

<400>  133
aaaaaaaggu uaaaaaaaa                                                    19


<210>  134
<211>  19
<212>  RNA
<213>  Artificial

<220>
<223>  randomized 4 mer motifs on poly A backbone

<400>  134
aaaaaaagua caaaaaaaa                                                    19


<210>  135
<211>  19
<212>  RNA
<213>  Artificial

<220>
<223>  randomized 4 mer motifs on poly A backbone

<400>  135
aaaaaaagua gaaaaaaaa                                                    19


<210>  136
<211>  19
<212>  RNA
<213>  Artificial

<220>
<223>  randomized 4 mer motifs on poly A backbone

<400>  136
aaaaaaagua uaaaaaaaa                                                    19


<210>  137
<211>  19
<212>  RNA
<213>  Artificial

<220>
<223>  randomized 4 mer motifs on poly A backbone

<400>  137
aaaaaaaguc caaaaaaaa                                                    19


<210>  138
<211>  19
<212>  RNA
<213>  Artificial

<220>
<223>  randomized 4 mer motifs on poly A backbone

<400>  138
aaaaaaaguc gaaaaaaaa                                                    19


<210>  139
<211>  19
<212>  RNA
<213>  Artificial

<220>
<223>  randomized 4 mer motifs on poly A backbone

<400>  139
aaaaaaaguc uaaaaaaaa                                                    19


<210>  140
<211>  19
<212>  RNA
<213>  Artificial

<220>
<223>  randomized 4 mer motifs on poly A backbone

<400>  140
aaaaaaagug caaaaaaaa                                                    19


<210>  141
<211>  19
<212>  RNA
<213>  Artificial

<220>
<223>  randomized 4 mer motifs on poly A backbone

<400>  141
aaaaaaagug gaaaaaaaa                                                    19


<210>  142
<211>  19
<212>  RNA
<213>  Artificial

<220>
<223>  randomized 4 mer motifs on poly A backbone

<400>  142
aaaaaaagug uaaaaaaaa                                                    19


<210>  143
<211>  19
<212>  RNA
<213>  Artificial

<220>
<223>  randomized 4 mer motifs on poly A backbone

<400>  143
aaaaaaaguu caaaaaaaa                                                    19


<210>  144
<211>  19
<212>  RNA
<213>  Artificial

<220>
<223>  randomized 4 mer motifs on poly A backbone

<400>  144
aaaaaaaguu gaaaaaaaa                                                    19


<210>  145
<211>  19
<212>  RNA
<213>  Artificial

<220>
<223>  randomized 4 mer motifs on poly A backbone

<400>  145
aaaaaaaguu uaaaaaaaa                                                    19


<210>  146
<211>  19
<212>  RNA
<213>  Artificial

<220>
<223>  randomized 4 mer motifs on poly A backbone

<400>  146
aaaaaaauaa caaaaaaaa                                                    19


<210>  147
<211>  19
<212>  RNA
<213>  Artificial

<220>
<223>  randomized 4 mer motifs on poly A backbone

<400>  147
aaaaaaauaa gaaaaaaaa                                                    19


<210>  148
<211>  19
<212>  RNA
<213>  Artificial

<220>
<223>  randomized 4 mer motifs on poly A backbone

<400>  148
aaaaaaauaa uaaaaaaaa                                                    19

<210> 149
<211> 19
<212> RNA
<213> Artificial

<220>
<223> randomized 4 mer motifs on poly A backbone

<400> 149
   aaaaaaauac caaaaaaaa 19


<210> 150
<211> 19
<212> RNA
<213> Artificial

<220>
<223> randomized 4 mer motifs on poly A backbone

<400> 150
   aaaaaaauac gaaaaaaaa 19


<210> 151
<211> 19
<212> RNA
<213> Artificial

<220>
<223> randomized 4 mer motifs on poly A backbone

<400> 151
   aaaaaaauac uaaaaaaaa 19


<210> 152
<211> 19
<212> RNA
<213> Artificial

<220>
<223> randomized 4 mer motifs on poly A backbone

<400> 152
   aaaaaaauag caaaaaaaa 19


<210> 153
<211> 19
<212> RNA
<213> Artificial

<220>
<223> randomized 4 mer motifs on poly A backbone

<400> 153
   aaaaaaauag gaaaaaaaa 19


<210> 154
<211> 19
<212> RNA
<213> Artificial

<220>
<223>  randomized 4 mer motifs on poly A backbone

<400>  154
aaaaaaauag uaaaaaaaa                                                    19


<210>  155
<211>  19
<212>  RNA
<213>  Artificial

<220>
<223>  randomized 4 mer motifs on poly A backbone

<400>  155
aaaaaaauau caaaaaaaa                                                    19


<210>  156
<211>  19
<212>  RNA
<213>  Artificial

<220>
<223>  randomized 4 mer motifs on poly A backbone

<400>  156
aaaaaaauau gaaaaaaaa                                                    19


<210>  157
<211>  19
<212>  RNA
<213>  Artificial

<220>
<223>  randomized 4 mer motifs on poly A backbone

<400>  157
aaaaaaauau uaaaaaaaa                                                    19


<210>  158
<211>  19
<212>  RNA
<213>  Artificial

<220>
<223>  randomized 4 mer motifs on poly A backbone

<400>  158
aaaaaaauca caaaaaaaa                                                    19


<210>  159
<211>  19
<212>  RNA
<213>  Artificial

<220>
<223>  randomized 4 mer motifs on poly A backbone

<400>  159
aaaaaaauca gaaaaaaaa                                                    19


<210>  160
<211>  19
<212>  RNA
<213>  Artificial

<220>
<223>  randomized 4 mer motifs on poly A backbone

<400>  160
aaaaaaauca uaaaaaaaa                                                    19


<210>  161
<211>  19
<212>  RNA
<213>  Artificial

<220>
<223>  randomized 4 mer motifs on poly A backbone

<400>  161
aaaaaaaucc caaaaaaaa                                                    19


<210>  162
<211>  19
<212>  RNA
<213>  Artificial

<220>
<223>  randomized 4 mer motifs on poly A backbone

<400>  162
aaaaaaaucc gaaaaaaaa                                                    19


<210>  163
<211>  19
<212>  RNA
<213>  Artificial

<220>
<223>  randomized 4 mer motifs on poly A backbone

<400>  163
aaaaaaaucc uaaaaaaaa                                                    19


<210>  164
<211>  19
<212>  RNA
<213>  Artificial

<220>
<223>  randomized 4 mer motifs on poly A backbone

<400>  164
aaaaaaaucg caaaaaaaa                                                    19

<210> 165
<211> 19
<212> RNA
<213> Artificial

<220>
<223> randomized 4 mer motifs on poly A backbone

<400> 165
aaaaaaaucg gaaaaaaaa 19


<210> 166
<211> 19
<212> RNA
<213> Artificial

<220>
<223> randomized 4 mer motifs on poly A backbone

<400> 166
aaaaaaaucg uaaaaaaaa 19


<210> 167
<211> 19
<212> RNA
<213> Artificial

<220>
<223> randomized 4 mer motifs on poly A backbone

<400> 167
aaaaaaaucu caaaaaaaa 19


<210> 168
<211> 19
<212> RNA
<213> Artificial

<220>
<223> randomized 4 mer motifs on poly A backbone

<400> 168
aaaaaaaucu gaaaaaaaa 19


<210> 169
<211> 19
<212> RNA
<213> Artificial

<220>
<223> randomized 4 mer motifs on poly A backbone

<400> 169
aaaaaaaucu uaaaaaaaa 19


<210> 170
<211> 19
<212> RNA
<213> Artificial

<220>
<223>  randomized 4 mer motifs on poly A backbone

<400>  170
aaaaaaauga caaaaaaaa                                                    19


<210>  171
<211>  19
<212>  RNA
<213>  Artificial

<220>
<223>  randomized 4 mer motifs on poly A backbone

<400>  171
aaaaaaauga gaaaaaaaa                                                    19


<210>  172
<211>  19
<212>  RNA
<213>  Artificial

<220>
<223>  randomized 4 mer motifs on poly A backbone

<400>  172
aaaaaaauga uaaaaaaaa                                                    19


<210>  173
<211>  19
<212>  RNA
<213>  Artificial

<220>
<223>  randomized 4 mer motifs on poly A backbone

<400>  173
aaaaaaaugc caaaaaaaa                                                    19


<210>  174
<211>  19
<212>  RNA
<213>  Artificial

<220>
<223>  randomized 4 mer motifs on poly A backbone

<400>  174
aaaaaaaugc gaaaaaaaa                                                    19


<210>  175
<211>  19
<212>  RNA
<213>  Artificial

<220>
<223>  randomized 4 mer motifs on poly A backbone

<400>  175
aaaaaaaugc uaaaaaaaa                                                    19


<210>  176
<211>  19
<212>  RNA
<213>  Artificial

<220>
<223>  randomized 4 mer motifs on poly A backbone

<400>  176
aaaaaaaugg caaaaaaaa                                                    19


<210>  177
<211>  19
<212>  RNA
<213>  Artificial

<220>
<223>  randomized 4 mer motifs on poly A backbone

<400>  177
aaaaaaaugg gaaaaaaaa                                                    19


<210>  178
<211>  19
<212>  RNA
<213>  Artificial

<220>
<223>  randomized 4 mer motifs on poly A backbone

<400>  178
aaaaaaaugg uaaaaaaaa                                                    19


<210>  179
<211>  19
<212>  RNA
<213>  Artificial

<220>
<223>  randomized 4 mer motifs on poly A backbone

<400>  179
aaaaaaaugu caaaaaaaa                                                    19


<210>  180
<211>  19
<212>  RNA
<213>  Artificial

<220>
<223>  randomized 4 mer motifs on poly A backbone

<400>  180
aaaaaaaugu gaaaaaaaa                                                    19

<210> 181
<211> 19
<212> RNA
<213> Artificial

<220>
<223> randomized 4 mer motifs on poly A backbone

<400> 181
aaaaaaaugu uaaaaaaaa 19


<210> 182
<211> 19
<212> RNA
<213> Artificial

<220>
<223> randomized 4 mer motifs on poly A backbone

<400> 182
aaaaaaauua caaaaaaaa 19


<210> 183
<211> 19
<212> RNA
<213> Artificial

<220>
<223> randomized 4 mer motifs on poly A backbone

<400> 183
aaaaaaauua gaaaaaaaa 19


<210> 184
<211> 19
<212> RNA
<213> Artificial

<220>
<223> randomized 4 mer motifs on poly A backbone

<400> 184
aaaaaaauua uaaaaaaaa 19


<210> 185
<211> 19
<212> RNA
<213> Artificial

<220>
<223> randomized 4 mer motifs on poly A backbone

<400> 185
aaaaaaauuc caaaaaaaa 19


<210> 186
<211> 19
<212> RNA
<213> Artificial

<220>
<223>  randomized 4 mer motifs on poly A backbone

<400>  186
aaaaaaauuc gaaaaaaaa                                                    19


<210>  187
<211>  19
<212>  RNA
<213>  Artificial

<220>
<223>  randomized 4 mer motifs on poly A backbone

<400>  187
aaaaaaauuc uaaaaaaaa                                                    19


<210>  188
<211>  19
<212>  RNA
<213>  Artificial

<220>
<223>  randomized 4 mer motifs on poly A backbone

<400>  188
aaaaaaauug caaaaaaaa                                                    19


<210>  189
<211>  19
<212>  RNA
<213>  Artificial

<220>
<223>  randomized 4 mer motifs on poly A backbone

<400>  189
aaaaaaauug gaaaaaaaa                                                    19


<210>  190
<211>  19
<212>  RNA
<213>  Artificial

<220>
<223>  randomized 4 mer motifs on poly A backbone

<400>  190
aaaaaaauug uaaaaaaaa                                                    19


<210>  191
<211>  19
<212>  RNA
<213>  Artificial

<220>
<223>  randomized 4 mer motifs on poly A backbone

<400>  191
aaaaaaauuu caaaaaaaa                                                    19


<210>  192
<211>  19
<212>  RNA
<213>  Artificial

<220>
<223>  randomized 4 mer motifs on poly A backbone

<400>  192
aaaaaaauuu gaaaaaaaa                                                    19


<210>  193
<211>  19
<212>  RNA
<213>  Artificial

<220>
<223>  randomized 4 mer motifs on poly A backbone

<400>  193
aaaaaaauuu uaaaaaaaa                                                    19


<210>  194
<211>  19
<212>  RNA
<213>  Artificial

<220>
<223>  different positions of the GUCA motif on poly A backbone

<400>  194
aagucaaaaa aaaaaaaaa                                                    19


<210>  195
<211>  19
<212>  RNA
<213>  Artificial

<220>
<223>  different positions of the GUCA motif on poly A backbone

<400>  195
aaaaaaguca aaaaaaaaa                                                    19


<210>  196
<211>  19
<212>  RNA
<213>  Artificial

<220>
<223>  different positions of the GUCA motif on poly A backbone

<400>  196
aaaaaaaagu caaaaaaaa                                                    19

<210>  197
<211>  19
<212>  RNA
<213>  Artificial

<220>
<223>  different positions of the GUCA motif on poly A backbone

<400>  197
   aaaaaaaaaa gucaaaaaa                                                    19


<210>  198
<211>  19
<212>  RNA
<213>  Artificial

<220>
<223>  different positions of the GUCA motif on poly A backbone

<400>  198
aaaaaaaaaa aaaagucaa                                                   19


<210>  199
<211>  19
<212>  RNA
<213>  Artificial

<220>
<223>  single base permutations to the 5' and the 3' end of GUCA motif
       on poly A backbone

<400>  199
   aaaaaaaagu caaaaaaaa                                                    19


<210>  200
<211>  19
<212>  RNA
<213>  Artificial

<220>
<223>  single base permutations to the 5' and the 3' end of GUCA motif
       on poly A backbone

<400>  200
   aaaaaaaagu cacaaaaaa                                                    19


<210>  201
<211>  19
<212>  RNA
<213>  Artificial

<220>
<223>  single base permutations to the 5' and the 3' end of GUCA motif
       on poly A backbone

<400>  201
   aaaaaaaagu cagaaaaaa                                                    19


<210>  202

<211>  19
<212>  RNA
<213>  Artificial

<220>
<223>  single base permutations to the 5' and the 3' end of GUCA motif
       on poly A backbone

<400>  202
aaaaaaaagu cauaaaaaa                                                    19


<210>  203
<211>  19
<212>  RNA
<213>  Artificial

<220>
<223>  single base permutations to the 5' and the 3' end of GUCA motif
       on poly A backbone

<400>  203
aaaaaaacgu caaaaaaaa                                                    19


<210>  204
<211>  19
<212>  RNA
<213>  Artificial

<220>
<223>  single base permutations to the 5' and the 3' end of GUCA motif
       on poly A backbone

<400>  204
aaaaaaacgu cacaaaaaa                                                    19


<210>  205
<211>  19
<212>  RNA
<213>  Artificial

<220>
<223>  single base permutations to the 5' and the 3' end of GUCA motif
       on poly A backbone

<400>  205
aaaaaaacgu cagaaaaaa                                                    19


<210>  206
<211>  19
<212>  RNA
<213>  Artificial

<220>
<223>  single base permutations to the 5' and the 3' end of GUCA motif
       on poly A backbone

<400>  206
aaaaaaacgu cauaaaaaa                                                    19

<210>  207
<211>  19
<212>  RNA
<213>  Artificial

<220>
<223>  single base permutations to the 5' and the 3' end of GUCA motif
       on poly A backbone

<400>  207
aaaaaaaggu caaaaaaaa                                                    19


<210>  208
<211>  19
<212>  RNA
<213>  Artificial

<220>
<223>  single base permutations to the 5' and the 3' end of GUCA motif
       on poly A backbone

<400>  208
aaaaaaaggu cacaaaaaa                                                    19


<210>  209
<211>  19
<212>  RNA
<213>  Artificial

<220>
<223>  single base permutations to the 5' and the 3' end of GUCA motif
       on poly A backbone

<400>  209
aaaaaaaggu cagaaaaaa                                                    19


<210>  210
<211>  19
<212>  RNA
<213>  Artificial

<220>
<223>  single base permutations to the 5' and the 3' end of GUCA motif
       on poly A backbone

<400>  210
aaaaaaaggu cauaaaaaa                                                    19


<210>  211
<211>  19
<212>  RNA
<213>  Artificial

<220>
<223>  single base permutations to the 5' and the 3' end of GUCA motif
       on poly A backbone

<400>  211
aaaaaaaugu caaaaaaaa                                                    19

<210>  212
<211>  19
<212>  RNA
<213>  Artificial

<220>
<223>  single base permutations to the 5' and the 3' end of GUCA motif
       on poly A backbone

<400>  212
aaaaaaaugu cacaaaaaa                                                    19


<210>  213
<211>  19
<212>  RNA
<213>  Artificial

<220>
<223>  single base permutations to the 5' and the 3' end of GUCA motif
       on poly A backbone

<400>  213
aaaaaaaugu cagaaaaaa                                                    19


<210>  214
<211>  19
<212>  RNA
<213>  Artificial

<220>
<223>  single base permutations to the 5' and the 3' end of GUCA motif
       on poly A backbone

<400>  214
aaaaaaaugu cauaaaaaa                                                    19


<210>  215
<211>  19
<212>  RNA
<213>  Artificial

<220>
<223>  sense strand of siRNA specific for human TLR9 with the starting
       base 1647 (siRNA9.2)

<400>  215
agcuuaaccu guccuucaa                                                    19


<210>  216
<211>  19
<212>  RNA
<213>  Artificial

<220>
<223>  poly A modification of the sense strand of siRNA specific for
       human TLR9 with the starting base 1647 (siRNA9.2)

<400>  216
aaaaaaaccu guccuucaa                                                    19

<210>  217
<211>  19
<212>  RNA
<213>  Artificial

<220>
<223>  poly A modification of the sense strand of siRNA specific for
       human TLR9 with the starting base 1647 (siRNA9.2)

<400>  217
aaaaaaaacu guccuucaa                                                    19


<210>  218
<211>  19
<212>  RNA
<213>  Artificial

<220>
<223>  poly A modification of the sense strand of siRNA specific for
       human TLR9 with the starting base 1647 (siRNA9.2)

<400>  218
aaaaaaaaau guccuucaa                                                    19


<210>  219
<211>  19
<212>  RNA
<213>  Artificial

<220>
<223>  poly A modification of the sense strand of siRNA specific for
       human TLR9 with the starting base 1647 (siRNA9.2)

<400>  219
aaaaaaaaaa guccuucaa                                                    19


<210>  220
<211>  19
<212>  RNA
<213>  Artificial

<220>
<223>  poly A modification of the sense strand of siRNA specific for
       human TLR9 with the starting base 1647 (siRNA9.2)

<400>  220
aaaaaaaaaa auccuucaa                                                    19


<210>  221
<211>  19
<212>  RNA
<213>  Artificial

<220>
<223>  poly A modification of the sense strand of siRNA specific for
       human TLR9 with the starting base 1647 (siRNA9.2)

<400>  221

aaaaaaaaaa aaccuucaa                                                19

<210>  222
<211>  19
<212>  RNA
<213>  Artificial

<220>
<223>  poly A modification of the sense strand of siRNA specific for
       human TLR9 with the starting base 1647 (siRNA9.2)

<400>  222
agcuuaaccu aaaaaaaaa                                                19

<210>  223
<211>  19
<212>  RNA
<213>  Artificial

<220>
<223>  poly A modification of the sense strand of siRNA specific for
       human TLR9 with the starting base 1647 (siRNA9.2)

<400>  223
agcuuaaccu gaaaaaaaa                                                19

<210>  224
<211>  19
<212>  RNA
<213>  Artificial

<220>
<223>  poly A modification of the sense strand of siRNA specific for
       human TLR9 with the starting base 1647 (siRNA9.2)

<400>  224
agcuuaaccu guaaaaaaa                                                19

<210>  225
<211>  19
<212>  RNA
<213>  Artificial

<220>
<223>  poly A modification of the sense strand of siRNA specific for
       human TLR9 with the starting base 1647 (siRNA9.2)

<400>  225
agcuuaaccu gucaaaaaa                                                19

<210>  226
<211>  19
<212>  RNA
<213>  Artificial

<220>
<223>  poly A modification of the sense strand of siRNA specific for
       human TLR9 with the starting base 1647 (siRNA9.2)

<400>  226
agcuuaaccu guccaaaaa                                                    19


<210>  227
<211>  19
<212>  RNA
<213>  Artificial

<220>
<223>  poly A modification of the sense strand of siRNA specific for
       human TLR9 with the starting base 1647 (siRNA9.2)

<400>  227
agcuuaaccu guccuaaaa                                                    19


<210>  228
<211>  19
<212>  RNA
<213>  Artificial

<220>
<223>  poly A modification of the sense strand of siRNA specific for
       human TLR9 with the starting base 1647 (siRNA9.2)

<400>  228
agcuuaaccu guccuuaaa                                                    19


<210>  229
<211>  20
<212>  RNA
<213>  Artificial

<220>
<223>  ssRNA oligonucleotide "RNA40" (Heil et al.)

<400>  229
gcccgucugu ugugugacuc                                                   20


<210>  230
<211>  19
<212>  RNA
<213>  Artificial

<220>
<223>  sense strand of siRNA specific for human beta-Galactosidase
       (Judge et al.)

<400>  230
uugauguguu uagucgcua                                                    19


<210>  231
<211>  19
<212>  RNA
<213>  Artificial

<220>
<223>  sense strand of siRNA specific for human TLR9 with the starting
       base 271

<400>  231
caucucuccc ugcucucug                                                    19


<210>  232
<211>  19
<212>  RNA
<213>  Artificial

<220>
<223>  antisense strand of siRNA specific for human TLR9 with the
       starting base 271

<400>  232
cagagagcag ggagagaug                                                    19


<210>  233
<211>  19
<212>  RNA
<213>  Artificial

<220>
<223>  sense strand of siRNA specific for human TLR9 with the starting
       base 1122

<400>  233
ucgccggccu gcaugcccu                                                    19


<210>  234
<211>  19
<212>  RNA
<213>  Artificial

<220>
<223>  antisense strand of siRNA specific for human TLR9 with the
       starting base 1122

<400>  234
agggcaugca ggccggcga                                                    19


<210>  235
<211>  19
<212>  RNA
<213>  Artificial

<220>
<223>  sense strand of siRNA specific for human TLR9 with the starting
       base 1949

<400>  235
gaggcagaug gaggggaga                                                    19


<210>  236
<211>  19
<212>  RNA
<213>  Artificial

<220>
<223>  antisense strand of siRNA specific for human TLR9 with the
       starting base 1949

<400>  236
ucuccccucc aucugccuc                                                    19


<210>  237
<211>  19
<212>  RNA
<213>  Artificial

<220>
<223>  sense strand of siRNA specific for human TLR9 with the starting
       base 1302

<400>  237
aguaucugcu guuguccua                                                    19


<210>  238
<211>  19
<212>  RNA
<213>  Artificial

<220>
<223>  antisense strand of siRNA specific for human TLR9 with the
       starting base 1302

<400>  238
uaggacaaca gcagauacu                                                    19


<210>  239
<211>  19
<212>  RNA
<213>  Artificial

<220>
<223>  sense strand of siRNA specific for human TLR9 with the starting
       base 1500

<400>  239
ugguguugaa ggacaguuc                                                    19


<210>  240
<211>  19
<212>  RNA
<213>  Artificial

<220>
<223>  antisense strand of siRNA specific for human TLR9 with the
       starting base 1500

<400>  240
gaacuguccu ucaacacca                                                    19


<210>  241
<211>  19
<212>  RNA
<213>  Artificial

<220>
<223>  sense strand of siRNA specific for human TLR9 with the starting

base 1019

<400> 241
aaccugagcu acaacaaca 19


<210> 242
<211> 19
<212> RNA
<213> Artificial

<220>
<223> antisense strand of siRNA specific for human TLR9 with the
starting base 1019

<400> 242
uguuguugua gcucagguu 19


<210> 243
<211> 21
<212> RNA
<213> Artificial

<220>
<223> example ssRNA oligonucleotide (hairpin sequence)

<400> 243
gaccuagccu aaaacuaggu c 21


<210> 244
<211> 17
<212> RNA
<213> Artificial

<220>
<223> example ssRNA oligonucleotide (palindromic sequence)

<400> 244
aaagauccgg aucaaaa 17


<210> 245
<211> 19
<212> RNA
<213> Artificial

<220>
<223> example ssRNA oligonucleotide (partially double stranded sequence
1 example 1)

<400> 245
aaaaguucaa agcucaaaa 19


<210> 246
<211> 11
<212> RNA
<213> Artificial

<220>
<223> example ssRNA oligonucleotide (partially double stranded sequence
2 example 1)

<400>  246
caaguuucga g                                                            11


<210>  247
<211>  33
<212>  RNA
<213>  Artificial

<220>
<223>  example ssRNA oligonucleotide (partially double stranded sequence
       1 example 2)

<400>  247
ucaaagucaa aagcucaaag uugaaaguuu aaa                                    33


<210>  248
<211>  41
<212>  RNA
<213>  Artificial

<220>
<223>  example ssRNA oligonucleotide (partially double stranded sequence
       2 example 2)

<400>  248
gacuugaaaa uuucaguuuu cgaguuuaag uugaaaacuc g                           41


<210>  249
<211>  26
<212>  RNA
<213>  Artificial

<220>
<223>  example ssRNA oligonucleotide (partially double stranded sequence
       1 example 3)

<400>  249
ucaaagucaa aagcucaaag uugaaa                                            26


<210>  250
<211>  31
<212>  RNA
<213>  Artificial

<220>
<223>  example ssRNA oligonucleotide (partially double stranded sequence
       2 example 3)

<400>  250
uuucaguuuu cgaguuuaag uugaaaacuc g                                      31


<210>  251
<211>  20
<212>  RNA
<213>  Artificial

<220>
<223>  example ssRNA oligonucleotide (20mer oligo to explain the

algorithm)

<400>  251
cagagcggga ugcguugguc                                                    20


<210>  252
<211>  19
<212>  RNA
<213>  Artificial

<220>
<223>  disclaimer ssRNA oligonucleotide (Judge et al. 1)

<400>  252
uugauguguu uagucgcua                                                     19


<210>  253
<211>  19
<212>  RNA
<213>  Artificial

<220>
<223>  disclaimer ssRNA oligonucleotide (Sioud et al. 1)

<400>  253
gcaccacuag uugguuguc                                                     19


<210>  254
<211>  19
<212>  RNA
<213>  Artificial

<220>
<223>  disclaimer ssRNA oligonucleotide (Sioud et al. 2)

<400>  254
guuguaguug uacuccagc                                                     19


<210>  255
<211>  20
<212>  RNA
<213>  Artificial

<220>
<223>  disclaimer ssRNA oligonucleotide "RNA40" (Heil et al. 1)

<400>  255
gcccgucugu ugugugacuc                                                    20


<210>  256
<211>  12
<212>  RNA
<213>  Artificial

<220>
<223>  disclaimer ssRNA oligonucleotide "RNA40" (Heil et al. 2)

<400>  256
gucuguugug ug                                                            12

<210>  257
<211>  18
<212>  RNA
<213>  Artificial

<220>
<223>  disclaimer ssRNA oligonucleotide (patent WO 03/086280)

<400>  257
guugugguug ugguugug                                                    18


<210>  258
<211>  19
<212>  RNA
<213>  Artificial

<220>
<223>  example ssRNA oligonucleotide (motif in different positions)

<400>  258
aaaaaaaguu caaaaaaaa                                                   19


<210>  259
<211>  19
<212>  RNA
<213>  Artificial

<220>
<223>  example ssRNA oligonucleotide (motif in different positions)

<400>  259
aaaguucaaa aaaaaaaaa                                                   19


<210>  260
<211>  19
<212>  RNA
<213>  Artificial

<220>
<223>  example ssRNA oligonucleotide (motif in different positions)

<400>  260
aaaaaaaaaa aaguucaaa                                                   19


<210>  261
<211>  19
<212>  RNA
<213>  Artificial

<220>
<223>  example ssRNA oligonucleotide (more than one motif)

<400>  261
aaaguucaaa aaguucaaa                                                   19


<210>  262
<211>  19

<212>  RNA
<213>  Artificial

<220>
<223>  example ssRNA oligonucleotide (more than one motif)

<400>  262
guucaaaguu caaaguuca                                                    19


<210>  263
<211>  19
<212>  RNA
<213>  Artificial

<220>
<223>  example ssRNA oligonucleotide (different motifs)

<400>  263
aguucaaagu caaaagcuc                                                    19


<210>  264
<211>  22
<212>  RNA
<213>  Artificial

<220>
<223>  example ssRNA oligonucleotide (different motifs)

<400>  264
aguucaguuc aagucaaagc uc                                                22


<210>  265
<211>  45
<212>  RNA
<213>  Artificial

<220>
<223>  example ssRNA oligonucleotide (different motifs)

<400>  265
aaaguucaaa gucaaaagcu caaaguugaa aguuuaaagg uuaaa                       45


<210>  266
<211>  17
<212>  RNA
<213>  Artificial

<220>
<223>  example ssRNA oligonucleotide (overlapping motifs)

<400>  266
aaaaguugcu caaaaaa                                                      17


<210>  267
<211>  10
<212>  RNA
<213>  Artificial

<220>

<223>  ssRNA oligonucleotide "RNA33" (Heil et al.)

<400>  267
guagugugug                                                            10


<210>  268
<211>  21
<212>  RNA
<213>  Artificial

<220>
<223>  ssRNA oligonucleotide "sense 27" (Sioud et al.)

<400>  268
guccgggcag gucuacuuuu u                                               21


<210>  269
<211>  21
<212>  RNA
<213>  Artificial

<220>
<223>  ssRNA oligonucleotide "sense 32" (Sioud et al.)

<400>  269
gcuggagauc cugaagaacu u                                               21


<210>  270
<211>  19
<212>  RNA
<213>  Artificial

<220>
<223>  ssRNA oligonucleotide "beta-Gal control sense" (Judge et al.)

<400>  270
uugauguguu uagucgcua                                                  19


<210>  271
<211>  19
<212>  RNA
<213>  Artificial

<220>
<223>  ssRNA oligonucleotide "beta-Gal control antisense" (Judge et al.)

<400>  271
uagcgacuaa acacaucaa                                                  19


<210>  272
<211>  19
<212>  RNA
<213>  Artificial

<220>
<223>  ssRNA oligonucleotide "5' UTR" (Barchet et al.)

<400>  272
aguagaaaca agguaguuu                                                  19

<210>  273
<211>  19
<212>  RNA
<213>  Artificial

<220>
<223>  ssRNA oligonucleotide "3' UTR" (Barchet et al.)

<400>  273
uuaacuaccu gcuuuugcu                                                    19


<210>  274
<211>  42
<212>  RNA
<213>  Artificial

<220>
<223>  ssRNA oligonucleotide "5' 3' UTR" (Barchet et al.)

<400>  274
aguagaaaca agguaguuuu uuguuaacua ccugcuuuug cu                          42


<210>  275
<211>  19
<212>  RNA
<213>  Artificial

<220>
<223>  ssRNA oligonucleotide "5' UTR U-C replacement" (Barchet et al.)

<400>  275
agcagaaaca aggcagccc                                                    19


<210>  276
<211>  19
<212>  RNA
<213>  Artificial

<220>
<223>  ssRNA oligonucleotide "5' UTR G-C replacement" (Barchet et al.)

<400>  276
acuacaaaca accuacuuu                                                    19


<210>  277
<211>  19
<212>  RNA
<213>  Artificial

<220>
<223>  example ssRNA oligonucleotide (Group A oligos - above threshold)

<400>  277
guuuguugcu uugauugcc                                                    19


<210>  278
<211>  19

<212>  RNA
<213>  Artificial

<220>
<223>  example ssRNA oligonucleotide (Group A oligos - above threshold)

<400>  278
uuguaguucg uugcuagug                                                    19


<210>  279
<211>  19
<212>  RNA
<213>  Artificial

<220>
<223>  example ssRNA oligonucleotide (Group A oligos - above threshold)

<400>  279
aguucauggu ggguuguac                                                    19


<210>  280
<211>  19
<212>  RNA
<213>  Artificial

<220>
<223>  example ssRNA oligonucleotide (Group A oligos - above threshold)

<400>  280
uguuuaaguu guucuaccc                                                    19


<210>  281
<211>  19
<212>  RNA
<213>  Artificial

<220>
<223>  example ssRNA oligonucleotide (Group A oligos - above threshold)

<400>  281
aaguuugau uuuucagua                                                     19


<210>  282
<211>  19
<212>  RNA
<213>  Artificial

<220>
<223>  example ssRNA oligonucleotide (Group A oligos - above threshold)

<400>  282
aggcguuugu guucggguu                                                    19


<210>  283
<211>  19
<212>  RNA
<213>  Artificial

<220>

<223>  example ssRNA oligonucleotide (Group A oligos - above threshold)

<400>  283
agauguugua ggguguuuu                                                    19


<210>  284
<211>  19
<212>  RNA
<213>  Artificial

<220>
<223>  example ssRNA oligonucleotide (Group A oligos - above threshold)

<400>  284
uagugugugu cagugugac                                                    19


<210>  285
<211>  19
<212>  RNA
<213>  Artificial

<220>
<223>  example ssRNA oligonucleotide (Group A oligos - above threshold)

<400>  285
gguugcgugu ggaguuguu                                                    19


<210>  286
<211>  19
<212>  RNA
<213>  Artificial

<220>
<223>  example ssRNA oligonucleotide (Group A oligos - above threshold)

<400>  286
uguaguuuug uuagaguca                                                    19


<210>  287
<211>  19
<212>  RNA
<213>  Artificial

<220>
<223>  example ssRNA oligonucleotide (Group A oligos - above threshold)

<400>  287
gugugguugc uguugucaa                                                    19


<210>  288
<211>  19
<212>  RNA
<213>  Artificial

<220>
<223>  example ssRNA oligonucleotide (Group B oligos - below threshold)

<400>  288
gggaccgaaa gaccagacc                                                    19

<210>  289
<211>  19
<212>  RNA
<213>  Artificial

<220>
<223>  example ssRNA oligonucleotide (Group B oligos - below threshold)

<400>  289
uaagacuaga agagacaga                                                    19


<210>  290
<211>  19
<212>  RNA
<213>  Artificial

<220>
<223>  example ssRNA oligonucleotide (Group B oligos - below threshold)

<400>  290
agauccgaac caccgacca                                                    19


<210>  291
<211>  19
<212>  RNA
<213>  Artificial

<220>
<223>  example ssRNA oligonucleotide (Group B oligos - below threshold)

<400>  291
gaaccagaaa auagagcag                                                    19


<210>  292
<211>  19
<212>  RNA
<213>  Artificial

<220>
<223>  example ssRNA oligonucleotide (Group B oligos - below threshold)

<400>  292
cauauaagaa gaccagcca                                                    19


<210>  293
<211>  19
<212>  RNA
<213>  Artificial

<220>
<223>  example ssRNA oligonucleotide (Group B oligos - below threshold)

<400>  293
uaagaaccaa cugcuagaa                                                    19


<210>  294
<211>  19

<212>  RNA
<213>  Artificial

<220>
<223>  example ssRNA oligonucleotide (Group B oligos - below threshold)

<400>  294
ccccuacaga cagaauacc                                                    19


<210>  295
<211>  19
<212>  RNA
<213>  Artificial

<220>
<223>  example ssRNA oligonucleotide (Group B oligos - below threshold)

<400>  295
cuggcagaua gauagaagc                                                    19


<210>  296
<211>  19
<212>  RNA
<213>  Artificial

<220>
<223>  example ssRNA oligonucleotide (Group B oligos - below threshold)

<400>  296
cuagaccaga acaaucucg                                                    19


<210>  297
<211>  19
<212>  RNA
<213>  Artificial

<220>
<223>  example ssRNA oligonucleotide (Group B oligos - below threshold)

<400>  297
uuagagacau aacaacauu                                                    19


<210>  298
<211>  19
<212>  RNA
<213>  Artificial

<220>
<223>  example ssRNA oligonucleotide (Group B oligos - below threshold)

<400>  298
ggaccaaacc ucucgacau                                                    19


<210>  299
<211>  19
<212>  RNA
<213>  Artificial

<220>

<223>  example ssRNA oligonucleotide (Group A siRNA oligos targeting
      human Cyclophilin B - sense strand)

<400>  299
uaacaaacuc cuaccaaca                                                    19


<210>  300
<211>  19
<212>  RNA
<213>  Artificial

<220>
<223>  example ssRNA oligonucleotide (Group A siRNA oligos targeting
      human Cyclophilin B - antisense strand)

<400>  300
uguugguagg aguuuguua                                                    19


<210>  301
<211>  19
<212>  RNA
<213>  Artificial

<220>
<223>  example ssRNA oligonucleotide (Group A siRNA oligos targeting
      human Cyclophilin B - sense strand)

<400>  301
aacaaacucc uaccaacac                                                    19


<210>  302
<211>  19
<212>  RNA
<213>  Artificial

<220>
<223>  example ssRNA oligonucleotide (Group A siRNA oligos targeting
      human Cyclophilin B - antisense strand)

<400>  302
guguugguag gaguuuguu                                                    19


<210>  303
<211>  19
<212>  RNA
<213>  Artificial

<220>
<223>  example ssRNA oligonucleotide (Group A siRNA oligos targeting
      human Cyclophilin B - sense strand)

<400>  303
uaccaacacu gaccaauaa                                                    19


<210>  304
<211>  19
<212>  RNA
<213>  Artificial

<220>
<223>  example ssRNA oligonucleotide (Group A siRNA oligos targeting
      human Cyclophilin B - antisense strand)

<400>  304
uuauugguca guguuggua                                                    19


<210>  305
<211>  19
<212>  RNA
<213>  Artificial

<220>
<223>  example ssRNA oligonucleotide (Group A siRNA oligos targeting
      human Cyclophilin B - sense strand)

<400>  305
cuaccaacac ugaccaaua                                                    19


<210>  306
<211>  19
<212>  RNA
<213>  Artificial

<220>
<223>  example ssRNA oligonucleotide (Group A siRNA oligos targeting
      human Cyclophilin B - antisense strand)

<400>  306
uauuggucag uguugguag                                                    19


<210>  307
<211>  19
<212>  RNA
<213>  Artificial

<220>
<223>  example ssRNA oligonucleotide (Group A siRNA oligos targeting
      human Cyclophilin B - sense strand)

<400>  307
accaacacug accaauaaa                                                    19


<210>  308
<211>  19
<212>  RNA
<213>  Artificial

<220>
<223>  example ssRNA oligonucleotide (Group A siRNA oligos targeting
      human Cyclophilin B - antisense strand)

<400>  308
uuuauugguc aguguuggu                                                    19


<210>  309
<211>  19
<212>  RNA
<213>  Artificial

<220>
<223>  example ssRNA oligonucleotide (Group A siRNA oligos targeting
      human Cyclophilin B - sense strand)

<400>  309
acaaacuccu accaacacu                                                    19


<210>  310
<211>  19
<212>  RNA
<213>  Artificial

<220>
<223>  example ssRNA oligonucleotide (Group A siRNA oligos targeting
      human Cyclophilin B - antisense strand)

<400>  310
aguguuggua ggaguuugu                                                    19


<210>  311
<211>  19
<212>  RNA
<213>  Artificial

<220>
<223>  example ssRNA oligonucleotide (Group A siRNA oligos targeting
      human Cyclophilin B - sense strand)

<400>  311
acuccuacca acacugacc                                                    19


<210>  312
<211>  19
<212>  RNA
<213>  Artificial

<220>
<223>  example ssRNA oligonucleotide (Group A siRNA oligos targeting
      human Cyclophilin B - antisense strand)

<400>  312
ggucaguguu gguaggagu                                                    19


<210>  313
<211>  19
<212>  RNA
<213>  Artificial

<220>
<223>  example ssRNA oligonucleotide (Group A siRNA oligos targeting
      human Cyclophilin B - sense strand)

<400>  313
ccuaccaaca cugaccaau                                                    19


<210>  314
<211>  19
<212>  RNA

<213>  Artificial

<220>
<223>  example ssRNA oligonucleotide (Group A siRNA oligos targeting
      human Cyclophilin B - antisense strand)

<400>  314
auuggucagu guugguagg                                                    19


<210>  315
<211>  19
<212>  RNA
<213>  Artificial

<220>
<223>  example ssRNA oligonucleotide (Group A siRNA oligos targeting
      human Cyclophilin B - sense strand)

<400>  315
uccuaccaac acugaccaa                                                    19


<210>  316
<211>  19
<212>  RNA
<213>  Artificial

<220>
<223>  example ssRNA oligonucleotide (Group A siRNA oligos targeting
      human Cyclophilin B - antisense strand)

<400>  316
uuggucagug uugguagga                                                    19


<210>  317
<211>  19
<212>  RNA
<213>  Artificial

<220>
<223>  example ssRNA oligonucleotide (Group A siRNA oligos targeting
      human Cyclophilin B - sense strand)

<400>  317
aaacuccuac caacacuga                                                    19


<210>  318
<211>  19
<212>  RNA
<213>  Artificial

<220>
<223>  example ssRNA oligonucleotide (Group A siRNA oligos targeting
      human Cyclophilin B - antisense strand)

<400>  318
ucaguguugg uaggaguuu                                                    19


<210>  319
<211>  19

<212>  RNA
<213>  Artificial

<220>
<223>  example ssRNA oligonucleotide (Group A siRNA oligos targeting
       human Cyclophilin B - sense strand)

<400>  319
ccaacacuga ccaauaaaa                                                19


<210>  320
<211>  19
<212>  RNA
<213>  Artificial

<220>
<223>  example ssRNA oligonucleotide (Group A siRNA oligos targeting
       human Cyclophilin B - antisense strand)

<400>  320
uuuuauuggu caguguugg                                                19


<210>  321
<211>  19
<212>  RNA
<213>  Artificial

<220>
<223>  example ssRNA oligonucleotide (Group A siRNA oligos targeting
       human Cyclophilin B - sense strand)

<400>  321
caaacuccua ccaacacug                                                19


<210>  322
<211>  19
<212>  RNA
<213>  Artificial

<220>
<223>  example ssRNA oligonucleotide (Group A siRNA oligos targeting
       human Cyclophilin B - antisense strand)

<400>  322
caguguuggu aggaguuug                                                19


<210>  323
<211>  19
<212>  RNA
<213>  Artificial

<220>
<223>  example ssRNA oligonucleotide (Group A siRNA oligos targeting
       human Cyclophilin B - sense strand)

<400>  323
aacuccuacc aacacugac                                                19


<210>  324

<211>  19
<212>  RNA
<213>  Artificial

<220>
<223>  example ssRNA oligonucleotide (Group A siRNA oligos targeting
       human Cyclophilin B - antisense strand)

<400>  324
gucaguguug guaggaguu                                                    19


<210>  325
<211>  19
<212>  RNA
<213>  Artificial

<220>
<223>  example ssRNA oligonucleotide (Group A siRNA oligos targeting
       human Cyclophilin B - sense strand)

<400>  325
aacacugacc aauaaaaaa                                                    19


<210>  326
<211>  19
<212>  RNA
<213>  Artificial

<220>
<223>  example ssRNA oligonucleotide (Group A siRNA oligos targeting
       human Cyclophilin B - antisense strand)

<400>  326
uuuuuuauug gucaguguu                                                    19


<210>  327
<211>  19
<212>  RNA
<213>  Artificial

<220>
<223>  example ssRNA oligonucleotide (Group A siRNA oligos targeting
       human Cyclophilin B - sense strand)

<400>  327
caacacugac caauaaaaa                                                    19


<210>  328
<211>  19
<212>  RNA
<213>  Artificial

<220>
<223>  example ssRNA oligonucleotide (Group A siRNA oligos targeting
       human Cyclophilin B - antisense strand)

<400>  328
uuuuuauugg ucaguguug                                                    19

<210>  329
<211>  19
<212>  RNA
<213>  Artificial

<220>
<223>  example ssRNA oligonucleotide (Group A siRNA oligos targeting
       human Cyclophilin B - sense strand)

<400>  329
gcuacaaaaa cagcaaauu                                                    19


<210>  330
<211>  19
<212>  RNA
<213>  Artificial

<220>
<223>  example ssRNA oligonucleotide (Group A siRNA oligos targeting
       human Cyclophilin B - antisense strand)

<400>  330
aauuugcugu uuuuguagc                                                    19


<210>  331
<211>  19
<212>  RNA
<213>  Artificial

<220>
<223>  example ssRNA oligonucleotide (Group A siRNA oligos targeting
       human Cyclophilin B - sense strand)

<400>  331
ggcuacaaaa acagcaaau                                                    19


<210>  332
<211>  19
<212>  RNA
<213>  Artificial

<220>
<223>  example ssRNA oligonucleotide (Group A siRNA oligos targeting
       human Cyclophilin B - antisense strand)

<400>  332
auuugcuguu uuuguagcc                                                    19


<210>  333
<211>  19
<212>  RNA
<213>  Artificial

<220>
<223>  example ssRNA oligonucleotide (Group A siRNA oligos targeting
       human Cyclophilin B - sense strand)

<400>  333
acacugacca auaaaaaaa                                                    19

<210>  334
<211>  19
<212>  RNA
<213>  Artificial

<220>
<223>  example ssRNA oligonucleotide (Group A siRNA oligos targeting
       human Cyclophilin B - antisense strand)

<400>  334
uuuuuuuauu ggucagugu                                                    19


<210>  335
<211>  19
<212>  RNA
<213>  Artificial

<220>
<223>  example ssRNA oligonucleotide (Group A siRNA oligos targeting
       human Cyclophilin B - sense strand)

<400>  335
uggcuacaaa aacagcaaa                                                    19


<210>  336
<211>  19
<212>  RNA
<213>  Artificial

<220>
<223>  example ssRNA oligonucleotide (Group A siRNA oligos targeting
       human Cyclophilin B - antisense strand)

<400>  336
uuugcuguuu uuguagcca                                                    19


<210>  337
<211>  19
<212>  RNA
<213>  Artificial

<220>
<223>  example ssRNA oligonucleotide (Group A siRNA oligos targeting
       human Cyclophilin B - sense strand)

<400>  337
guaacaaacu ccuaccaac                                                    19


<210>  338
<211>  19
<212>  RNA
<213>  Artificial

<220>
<223>  example ssRNA oligonucleotide (Group A siRNA oligos targeting
       human Cyclophilin B - antisense strand)

<400>  338
guugguagga guuuguuac                                                    19

<210>  339
<211>  19
<212>  RNA
<213>  Artificial

<220>
<223>  example ssRNA oligonucleotide (Group A siRNA oligos targeting
       human Cyclophilin B - sense strand)

<400>  339
cacugaccaa uaaaaaaaa                                                    19


<210>  340
<211>  19
<212>  RNA
<213>  Artificial

<220>
<223>  example ssRNA oligonucleotide (Group A siRNA oligos targeting
       human Cyclophilin B - antisense strand)

<400>  340
uuuuuuuuau uggucagug                                                    19


<210>  341
<211>  19
<212>  RNA
<213>  Artificial

<220>
<223>  example ssRNA oligonucleotide (Group A siRNA oligos targeting
       human Cyclophilin B - sense strand)

<400>  341
acugaccaau aaaaaaaaa                                                    19


<210>  342
<211>  19
<212>  RNA
<213>  Artificial

<220>
<223>  example ssRNA oligonucleotide (Group A siRNA oligos targeting
       human Cyclophilin B - antisense strand)

<400>  342
uuuuuuuuua uuggucagu                                                    19


<210>  343
<211>  19
<212>  RNA
<213>  Artificial

<220>
<223>  example ssRNA oligonucleotide (Group A siRNA oligos targeting
       human Cyclophilin B - sense strand)

<400>  343

uacaaaaaca gcaaauucc                                                    19

<210>  344
<211>  19
<212>  RNA
<213>  Artificial

<220>
<223>  example ssRNA oligonucleotide (Group A siRNA oligos targeting
       human Cyclophilin B - antisense strand)

<400>  344
ggaauuugcu guuuuugua                                                    19

<210>  345
<211>  19
<212>  RNA
<213>  Artificial

<220>
<223>  example ssRNA oligonucleotide (Group A siRNA oligos targeting
       human Cyclophilin B - sense strand)

<400>  345
cuacaaaaac agcaaauuc                                                    19

<210>  346
<211>  19
<212>  RNA
<213>  Artificial

<220>
<223>  example ssRNA oligonucleotide (Group A siRNA oligos targeting
       human Cyclophilin B - antisense strand)

<400>  346
gaauuugcug uuuuuguag                                                    19

<210>  347
<211>  19
<212>  RNA
<213>  Artificial

<220>
<223>  example ssRNA oligonucleotide (Group A siRNA oligos targeting
       human Cyclophilin B - sense strand)

<400>  347
aaaauguggg uuuuuuuuu                                                    19

<210>  348
<211>  19
<212>  RNA
<213>  Artificial

<220>
<223>  example ssRNA oligonucleotide (Group A siRNA oligos targeting
       human Cyclophilin B - antisense strand)

<400>  348
aaaaaaaaac ccacauuuu                                                    19


<210>  349
<211>  19
<212>  RNA
<213>  Artificial

<220>
<223>  example ssRNA oligonucleotide (Group A siRNA oligos targeting
       human Cyclophilin B - sense strand)

<400>  349
ugugguguuu ggcaaaguu                                                    19


<210>  350
<211>  19
<212>  RNA
<213>  Artificial

<220>
<223>  example ssRNA oligonucleotide (Group A siRNA oligos targeting
       human Cyclophilin B - antisense strand)

<400>  350
aacuuugcca aacaccaca                                                    19


<210>  351
<211>  19
<212>  RNA
<213>  Artificial

<220>
<223>  example ssRNA oligonucleotide (Group A siRNA oligos targeting
       human Cyclophilin B - sense strand)

<400>  351
aaaugugggu uuuuuuuuu                                                    19


<210>  352
<211>  19
<212>  RNA
<213>  Artificial

<220>
<223>  example ssRNA oligonucleotide (Group A siRNA oligos targeting
       human Cyclophilin B - antisense strand)

<400>  352
aaaaaaaaaa cccacauuu                                                    19


<210>  353
<211>  19
<212>  RNA
<213>  Artificial

<220>
<223>  example ssRNA oligonucleotide (Group A siRNA oligos targeting
       human Cyclophilin B - sense strand)

<400>  353
guuuuuuuu uuuuuaaua                                                    19


<210>  354
<211>  19
<212>  RNA
<213>  Artificial

<220>
<223>  example ssRNA oligonucleotide (Group A siRNA oligos targeting
       human Cyclophilin B - antisense strand)

<400>  354
uauuaaaaa aaaaaaaac                                                    19


<210>  355
<211>  19
<212>  RNA
<213>  Artificial

<220>
<223>  example ssRNA oligonucleotide (Group A siRNA oligos targeting
       human Cyclophilin B - sense strand)

<400>  355
aaugugggu uuuuuuuuu                                                    19


<210>  356
<211>  19
<212>  RNA
<213>  Artificial

<220>
<223>  example ssRNA oligonucleotide (Group A siRNA oligos targeting
       human Cyclophilin B - antisense strand)

<400>  356
aaaaaaaaa acccacauu                                                    19


<210>  357
<211>  19
<212>  RNA
<213>  Artificial

<220>
<223>  example ssRNA oligonucleotide (Group A siRNA oligos targeting
       human Cyclophilin B - sense strand)

<400>  357
gguuuuuuu uuuuuuaau                                                    19


<210>  358
<211>  19
<212>  RNA
<213>  Artificial

<220>
<223>  example ssRNA oligonucleotide (Group A siRNA oligos targeting

human Cyclophilin B - antisense strand)

<400> 358
auuaaaaaaa aaaaaaacc 19


<210> 359
<211> 19
<212> RNA
<213> Artificial

<220>
<223> example ssRNA oligonucleotide (Group A siRNA oligos targeting
human Cyclophilin B - sense strand)

<400> 359
ggguuuuuuu uuuuuuuaa 19


<210> 360
<211> 19
<212> RNA
<213> Artificial

<220>
<223> example ssRNA oligonucleotide (Group A siRNA oligos targeting
human Cyclophilin B - antisense strand)

<400> 360
uuaaaaaaaa aaaaaaccc 19


<210> 361
<211> 19
<212> RNA
<213> Artificial

<220>
<223> example ssRNA oligonucleotide (Group A siRNA oligos targeting
human Cyclophilin B - sense strand)

<400> 361
uggguuuuuu uuuuuuuua 19


<210> 362
<211> 19
<212> RNA
<213> Artificial

<220>
<223> example ssRNA oligonucleotide (Group A siRNA oligos targeting
human Cyclophilin B - antisense strand)

<400> 362
uaaaaaaaaa aaaaaccca 19


<210> 363
<211> 19
<212> RNA
<213> Artificial

<220>

<223>  example ssRNA oligonucleotide (Group A siRNA oligos targeting
      human Cyclophilin B - sense strand)

<400>  363
   auguggguuu uuuuuuuuu                                                19

<210>  364
<211>  19
<212>  RNA
<213>  Artificial

<220>
<223>  example ssRNA oligonucleotide (Group A siRNA oligos targeting
      human Cyclophilin B - antisense strand)

<400>  364
aaaaaaaaaa aacccacau                                                19

<210>  365
<211>  19
<212>  RNA
<213>  Artificial

<220>
<223>  example ssRNA oligonucleotide (Group A siRNA oligos targeting
      human Cyclophilin B - sense strand)

<400>  365
   guggguuuuu uuuuuuuuu                                                19

<210>  366
<211>  19
<212>  RNA
<213>  Artificial

<220>
<223>  example ssRNA oligonucleotide (Group A siRNA oligos targeting
      human Cyclophilin B - antisense strand)

<400>  366
aaaaaaaaaa aaaacccac                                                19

<210>  367
<211>  19
<212>  RNA
<213>  Artificial

<220>
<223>  example ssRNA oligonucleotide (Group A siRNA oligos targeting
      human Cyclophilin B - sense strand)

<400>  367
ugugggguuuu uuuuuuuu                                                19

<210>  368
<211>  19
<212>  RNA
<213>  Artificial

<220>
<223>  example ssRNA oligonucleotide (Group A siRNA oligos targeting
      human Cyclophilin B - antisense strand)

<400>  368
aaaaaaaaaa aaacccaca                                                    19


<210>  369
<211>  19
<212>  RNA
<213>  Artificial

<220>
<223>  example ssRNA oligonucleotide (Group B siRNA oligos targeting
      human Cyclophilin B - sense strand)


<400>  369
aagaucgagg uggagaagc                                                    19


<210>  370
<211>  19
<212>  RNA
<213>  Artificial

<220>
<223>  example ssRNA oligonucleotide (Group B siRNA oligos targeting
      human Cyclophilin B - antisense strand)

<400>  370
gcuucuccac cucgaucuu                                                    19


<210>  371
<211>  19
<212>  RNA
<213>  Artificial

<220>
<223>  example ssRNA oligonucleotide (Group B siRNA oligos targeting
      human Cyclophilin B - sense strand)

<400>  371
agaucgaggu ggagaagcc                                                    19


<210>  372
<211>  19
<212>  RNA
<213>  Artificial

<220>
<223>  example ssRNA oligonucleotide (Group B siRNA oligos targeting
      human Cyclophilin B - antisense strand)

<400>  372
ggcuucucca ccucgaucu                                                    19


<210>  373
<211>  19
<212>  RNA

<213>  Artificial

<220>
<223>  example ssRNA oligonucleotide (Group B siRNA oligos targeting
      human Cyclophilin B - sense strand)

<400>  373
ccgccgcccu caucgcggg                                                    19


<210>  374
<211>  19
<212>  RNA
<213>  Artificial

<220>
<223>  example ssRNA oligonucleotide (Group B siRNA oligos targeting
      human Cyclophilin B - antisense strand)

<400>  374
cccgcgauga gggcggcgg                                                    19


<210>  375
<211>  19
<212>  RNA
<213>  Artificial

<220>
<223>  example ssRNA oligonucleotide (Group B siRNA oligos targeting
      human Cyclophilin B - sense strand)

<400>  375
ccuucugcgg ccgaugaga                                                    19


<210>  376
<211>  19
<212>  RNA
<213>  Artificial

<220>
<223>  example ssRNA oligonucleotide (Group B siRNA oligos targeting
      human Cyclophilin B - antisense strand)

<400>  376
ucucaucggc cgcagaagg                                                    19


<210>  377
<211>  19
<212>  RNA
<213>  Artificial

<220>
<223>  example ssRNA oligonucleotide (Group B siRNA oligos targeting
      human Cyclophilin B - sense strand)

<400>  377
cgccgcccuc aucgcgggg                                                    19


<210>  378
<211>  19

<212>  RNA
<213>  Artificial

<220>
<223>  example ssRNA oligonucleotide (Group B siRNA oligos targeting
       human Cyclophilin B - antisense strand)

<400>  378
ccccgcgaug agggcggcg                                                    19


<210>  379
<211>  19
<212>  RNA
<213>  Artificial

<220>
<223>  example ssRNA oligonucleotide (Group B siRNA oligos targeting
       human Cyclophilin B - sense strand)

<400>  379
cuuccugcug cugccggga                                                    19


<210>  380
<211>  19
<212>  RNA
<213>  Artificial

<220>
<223>  example ssRNA oligonucleotide (Group B siRNA oligos targeting
       human Cyclophilin B - antisense strand)

<400>  380
ucccggcagc agcaggaag                                                    19


<210>  381
<211>  19
<212>  RNA
<213>  Artificial

<220>
<223>  example ssRNA oligonucleotide (Group B siRNA oligos targeting
       human Cyclophilin B - sense strand)

<400>  381
gagcgcuucc ccgaugaga                                                    19


<210>  382
<211>  19
<212>  RNA
<213>  Artificial

<220>
<223>  example ssRNA oligonucleotide (Group B siRNA oligos targeting
       human Cyclophilin B - antisense strand)

<400>  382
ucucaucggg gaagcgcuc                                                    19


<210>  383

<211>  19
<212>  RNA
<213>  Artificial

<220>
<223>  example ssRNA oligonucleotide (Group B siRNA oligos targeting
       human Cyclophilin B - sense strand)

<400>  383
gccgccgccc ucaucgcgg                                                    19


<210>  384
<211>  19
<212>  RNA
<213>  Artificial

<220>
<223>  example ssRNA oligonucleotide (Group B siRNA oligos targeting
       human Cyclophilin B - antisense strand)

<400>  384
ccgcgaugag ggcggcggc                                                    19


<210>  385
<211>  19
<212>  RNA
<213>  Artificial

<220>
<223>  example ssRNA oligonucleotide (Group B siRNA oligos targeting
       human Cyclophilin B - sense strand)

<400>  385
ggcaagaucg agguggaga                                                    19


<210>  386
<211>  19
<212>  RNA
<213>  Artificial

<220>
<223>  example ssRNA oligonucleotide (Group B siRNA oligos targeting
       human Cyclophilin B - antisense strand)

<400>  386
ucuccaccuc gaucuugcc                                                    19


<210>  387
<211>  19
<212>  RNA
<213>  Artificial

<220>
<223>  example ssRNA oligonucleotide (Group B siRNA oligos targeting
       human Cyclophilin B - sense strand)

<400>  387
ucuuccugcu gcugccggg                                                    19

<210>  388
<211>  19
<212>  RNA
<213>  Artificial

<220>
<223>  example ssRNA oligonucleotide (Group B siRNA oligos targeting
       human Cyclophilin B - antisense strand)

<400>  388
cccggcagca gcaggaaga                                                    19


<210>  389
<211>  19
<212>  RNA
<213>  Artificial

<220>
<223>  example ssRNA oligonucleotide (Group B siRNA oligos targeting
       human Cyclophilin B - sense strand)

<400>  389
ugccgccgcc cucaucgcg                                                    19


<210>  390
<211>  19
<212>  RNA
<213>  Artificial

<220>
<223>  example ssRNA oligonucleotide (Group B siRNA oligos targeting
       human Cyclophilin B - antisense strand)

<400>  390
cgcgaugagg gcggcggca                                                    19

## Description

### Field of the Invention

The present invention relates to the field of immunotherapy and drug discovery. The present invention provides a method for determining the immunostimulatory activity of a RNA oligonucleotide. The present invention also provides a method for predicting the immunostimulatory activity of a RNA oligonucleotide. The present invention further provides a method for preparing RNA oligonucleotides with high or low immunostimulatory activity. Moreover, the present invention provides RNA oligonucleotides with immunostimulatory activity and the therapeutic uses thereof. In addition, the present invention provides RNA oligonucleotides with gene silencing activity and with either high or low immunostimulatory activity, the methods of their preparation, and their therapeutic uses.

### Background of the Invention

The vertebrate immune system established different ways to detect invading pathogens based on certain characteristics of their microbial nucleic acids. Detection of microbial nucleic acids alerts the immune system to mount the appropriate type of immune response that is required for the defense against the respective type of pathogen detected. Detection of viral nucleic acids leads to the production of type I interferon (IFN), the key cytokine for anti-viral defence. An understanding of how nucleic acids interact with the vertebrate immune system is important for developing different nucleic acid-based therapeutic strategies for the immunotherapy of diseases (Rothenfusser S et al. 2003, Curr Opin Mol Ther 5:98-106) and for developing gene-specific therapeutic agents (Tuschl T et al. 2002, Mol Interv 2: 158-167).

For the recognition of long dsRNA, two detection modes are known, the serine threonine kinase PKR (Williams BR, 2001, Sci Signal Transduction Knowledge Enviroment 89: RE2; Meurs EF et al. 1992, J Virol 66: 5805-5814; Katze MG et al. 1991, Mol Cell Biol 11: 5497-5505) and Toll-like receptor (TLR) 3 (Alexopoulou L et al. 2001, Nature 413: 732-738). Whereas PKR is located in the cytosol, TLR3 is present in the endosomal compartment (Matsumoto M et al. 2003, J Immunol 171: 3154-3162). TLR3 is a member of the Toll-like receptor family that has evolved to detect pathogen-specific molecules (Takeda K et al. 2003, Annu Rev Immunol 21: 335-376).

A second characteristic feature of viral nucleic acids used by the immune system to recognize viral infection are CpG motifs found in viral DNA, which are detected via TLR9 (Lund J et al. 2003, J Exp Med 198: 513-520; Krug A et al. 2004, Blood 103: 1433-1437). CpG motifs are unmethylated CG dinucleotides with certain flanking bases. The frequency of CpG motifs is suppressed in vertebrates, allowing the vertebrate immune system to detect microbial DNA based on such CpG motifs (Krieg AM et al. 1995, Nature 374: 546-549; Bauer S et al. 2001, Proc Natl Acad Sci USA 98: 9237-9242; Wagner H et al. 2002, Curr Opin Microbiol 5: 62-69). Like TLR3, TLR9 is located in the endosomal compartment where it directly binds to CpG motifs (Latz E et al. 2004, Nat Immunol 5: 190-198).

In addition to long dsRNA and CpG DNA, two recent publications suggest a third mechanism by which viral nucleic acids are recognized. These studies demonstrate that single-stranded RNA (ssRNA) of ssRNA viruses is detected via TLR7 (mouse and human) and TLR8 (only human) (Diebold SS et al. 2004, Science 303: 1529-1531; Heil F et al. 2004, Science 303: 1526-1529). Guanine analogues have been identified earlier as specific ligands for TLR7 and TLR8 (Lee J et al. 2003, Proc Natl Acad Sci USA 100: 6646-6651; Heil F et al. 2003, Eur J Immunol 33: 2987-2997). Like TLR9 (receptor for CpG DNA) (Latz E et al. 2004, Nat Immunol 5: 190-198), TLR7 and TLR8 are located in the endosomal membrane (Heil F et al. 2003, Eur J Immunol 33: 2987-2997).

Detection of viral nucleic acids leads to the production of type I IFN (IFN-α and IFN-β). The major producer of type I IFN in humans is the plasmacytoid dendritic cell (PDC, also called interferon producing cell, IPC). The plasmacytoid dendritic cell (PDC) is a highly specialized subset of dendritic cells that is thought to function as a sentinel for viral infection and is responsible for the vast amount of type I IFN during viral infection (Asselin-Paturel C et al. 2001, Nat Immunol 2: 1144-1150). There is increasing evidence that PDC preferentially use nucleic acid-based molecular patterns to detect viral infection. TLR expression of human and mouse PDC is limited to TLR7 and TLR9 (Krug A et al. 2001, Eur J Immunol 31: 3026-3037; Hornung V et al. 2002, J Immunol 168: 4531-4537; Edwards AD et al. 2003, Eur J Immunol 33: 827-833).

IFN-α was the first type of interferon to be identified and commercialized; it is widely used clinically in the treatment of a variety of tumors (e.g., hairy cell leukemia, cutaneous T cell leukemia, chronic myeloid leukemia, non-Hodgkin's lymphoma, AIDS-related Kaposi's sarcoma, malignant melanoma, multiple myeloma, renal cell carcinoma, bladder cell carcinoma, colon carcinoma, cervical dysplasia) and viral diseases (e.g., chronic hepatitis B, chronic hepatitis C). IFN-α products that are currently in clinical use include the recombinant protein and the highly purified natural protein, both of which have high production costs. Therefore, there is a need for more economical ways of providing IFN-α to patients in need. Furthermore, IFN-α is currently administrated systematically and causes a broad spectrum of side effects (e.g. fatigue, flu-like symptoms, diarrhea). Most alarmingly, IFN-α causes a decrease in bone marrow function which leads to increased susceptibility to life-threatening infections, anemia and bleeding problems. Therefore, there is a need for ways of providing IFN-α in a more localized (i.e., target-specific) matter to reduce the occurrence of side effects.

In addition to inducing an anti-viral interferon response, dsRNA also induces post-transcription gene silencing, a highly conserved anti-viral mechanism known as RNA interference (RNAi). Briefly, the RNA III Dicer enzyme processes dsRNA into short interfering RNA (siRNA) of approximately 22 nucleotides. The antisense strand of the siRNA binds a target mRNA via base pairing and serves as a guide sequence to induce cleavage of the target mRNA by an RNA-induced silencing complex RISC. dsRNA has been an extremely powerful tool in studying gene functions in C. *elegence* and Drosophila via gene silencing. However, its use in mammalian cells has been limited because the interferon response it elicits is detrimental to most mammalian cells.

Subsequently, it was found that siRNA was also capable of inducing RNAi, causing degradation of the target mRNA in a sequence-specific manner and it was thought to be short enough to bypass dsRNA-induced nonspecific effects in mammalian cells (Elbashri SM et al. 2001, Nature 411:494-498). Since then, siRNA has been widely used as a gene silencing tool in deciphering mammalian gene functions in research and drug discovery, and there has been great interest in its potential in therapeutic applications.

siRNA can be used to reduce or even abolish the expression of disease/disorder-related genes for preventing or treating diseases caused by the expression or overexpression of the disease-related genes. Such diseases include, but are not limited to, infections, metabolic diseases, autoimmune diseases and cancer. However, concern has been raised recently about the potential for siRNA to activate immune responses which may be undesirable for certain indications and thus limit the use of siRNA as a gene silencing agent for therapeutic purposes (Sioud M et al. 2003, Biochem. Biophys. Res. Commun. 312:1220-1225). Therefore, there is a need for methods for predicting the potential of a given siRNA to induce an interferon response and for methods for designing and preparing siRNAs for gene silencing which are devoid of unwanted immunostimulatory activities.

On the other hand, for certain therapeutic applications, for example, the prevention or treatment of cancer and viral infections, immunostimulatory activity may be desirable as an additional functional activity of the siRNA.

In an effort to apply siRNA for the specific downregulation of TLR9 in PDC in our previous publication (Hornung V et al. 2005, Nat Med 11: 263-270), we made the surprising observation that, despite the inability of PDC to detect long dsRNA, certain siRNA sequences were potent *in vitro* inducers of IFN-α in PDC. We found that i) short interfering RNA (siRNA) induces IFN-α in human plasmacytoid dendritic cells when transfected with cationic lipids, ii) this activity of siRNA is sequence-dependent but independent of the G or U content of the siRNA, iii) the immunostimuatory activity of siRNA and the antisense activity are two independent functional activities of siRNA, iv) the immune recognition of siRNA occurs on the single strand level, v) siRNAs containing the 9mer sequence motif 5'-GUCCUUCAA-3' show potent immunostimulatory activity, and vi) such siRNAs induce systemic immune responses in mice, and vii) the induction of immune responses by siRNA requires the presence of TLR7 in mice. Our findings suggest that the 9mer sequence motif 5'-GUCCUUCAA-3' may be a ligand for TLR7.

The natural ligand for TLR7 has not been well defined to date. Guanine analogues have been identified earlier as specific ligands for TLR7 and TLR8 (Lee J et al. 2003, Proc Natl Acad Sci USA 100: 6646-6651; Heil F et al. 2003, Eur J Immunol 33: 2987-2997), whereas guanosine ribonucleoside or a derivative thereof has been identified as TLR7 ligand in WO03086280.

It is an object of the present invention to identify RNA oligonucleotide motifs for stimulating an immune response, in particular, IFN-α induction. It is also an object of the present invention to identify ligands for activating TLR7 and TLR8. It is another object of the present invention to develop a method for determining the immunostimulatory activity, in particular, the IFN-α-inducing activity, of a RNA oligonucleotide. It is yet another object of the present invention to develop a method for predicting the immunostimulatory activity, in particular, IFN-α-inducing activity, of a RNA oligonucleotide. It is a further object of the invention to develop a method for designing and preparing RNA oligonucleotide having or lacking immunostimulatory activity, in particular, IFN-α-inducing activity. It is also an object of the invention to provide RNA oligonucleotides having high immunostimulatory activity which can be used to induce an immune response, in particular, IFN-α production, in patients in need thereof. It is yet another object of the present invention to provide siRNA molecules that either have or lack immunostimulatory activity which can be used to treat disorders caused by the expression or overexpression of disorder-related genes.

### Summary of the Invention

The present invention provides a method for determining the immunostimulatory activity of a RNA oligonucleotide, a method for predicting the immunostimulatory activity of a RNA oligonucleotide, a method for preparing a RNA oligonucleotide with high or low immunostimulatory activity, and a method for preparing a RNA oligoncleotide with gene silencing activity and with high or low immunostimulatory activity.

The present application also provides an in vitro method for inducing IFN-α production from a mammalian cell, and an in vitro method for activating a dendritic cell.

The present invention further provides a RNA oligonucleotide with immunostimulatory activity, a RNA oligonucleotide with gene silencing activity and with high or low immunostimulatory activity, and the therapeutic uses thereof.

In addition, the present invention provides a pharmaceutical composition comprising one or more of the RNA oligonucleotides of the invention.

### Brief Description of the Figures

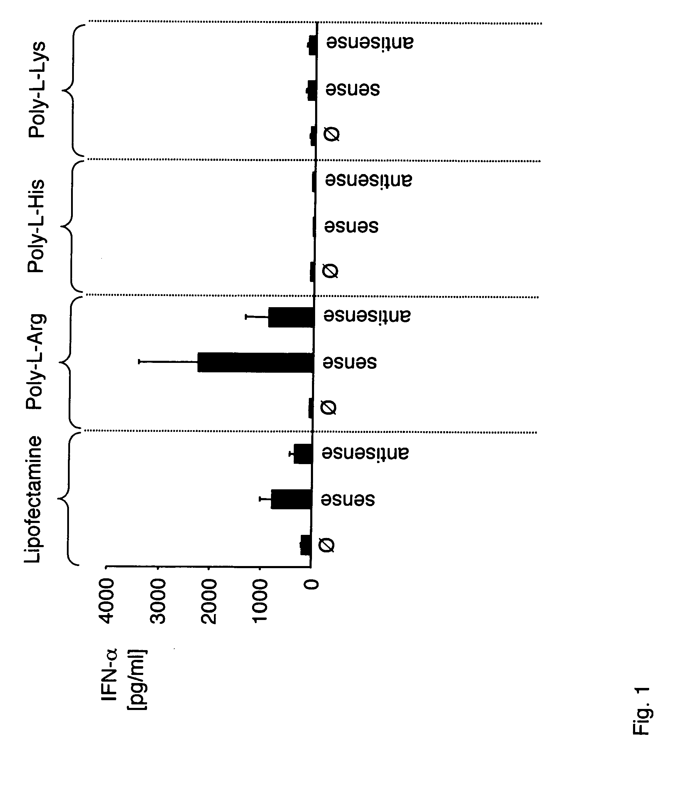

Figure 1: PBMC of three individual donors were isolated and stimulated with ssRNA oligonucleotides 9.2sense (5'-AGCUUAACCUGUCCUUCAA-3') and 9.2antisense (5'-UUGAAGGACAGGUUAAGCU-3') that were complexed with either Lipofectamine, poly-L-arginine, poly-L-histidine or poly-L-lysine in duplicates. 24 hours after stimulation supernatants were harvested and IFN-α was assessed by ELISA. Data are presented as mean values ± SEM.

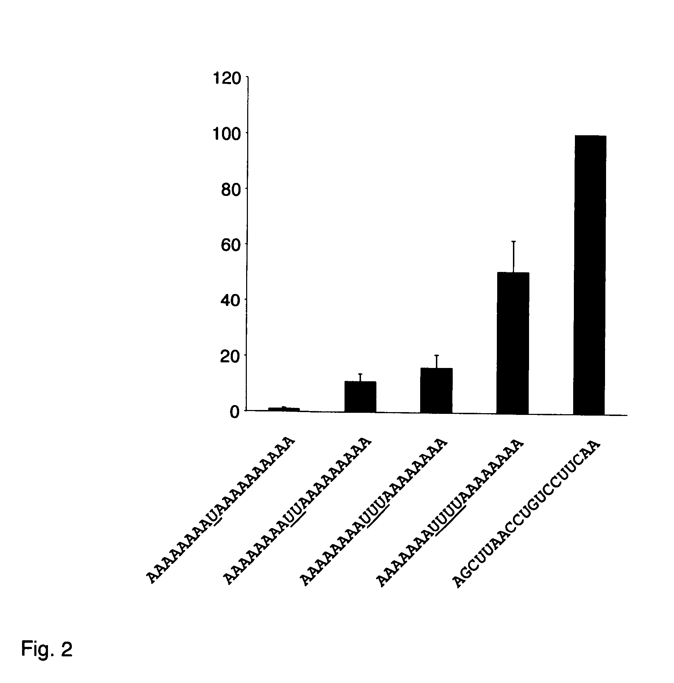

Figure 2: PBMC of three different healthy donors were isolated and stimulated with poly-L-arginine complexed ssRNA oligonucleotides in duplicates. 44 hours after stimulation IFN-a production was assessed in supernatant via ELISA. For all tested ssRNA oligonucleotides, the mean values of the measured duplicates were normalized to the positive control ssRNA oligonucleotide 9.2sense (5'-AGCUUAACCUGUCCUUCAA) by dividing the mean value of tested oligonucleotide by the mean value of 9.2sense (= 100%). Data from three different donors were summarized and are presented as mean values ± SEM.

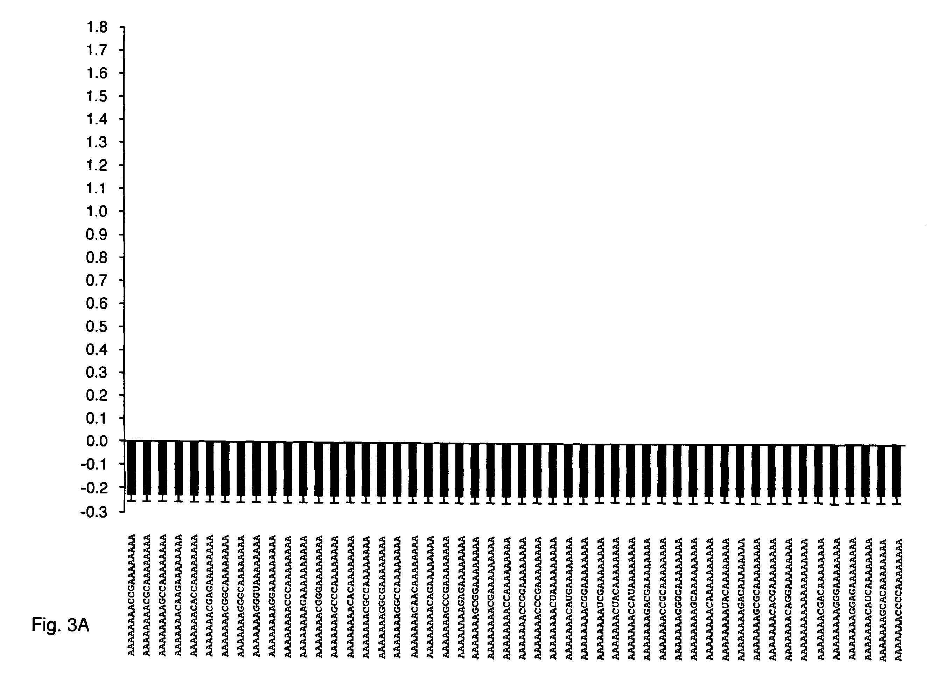

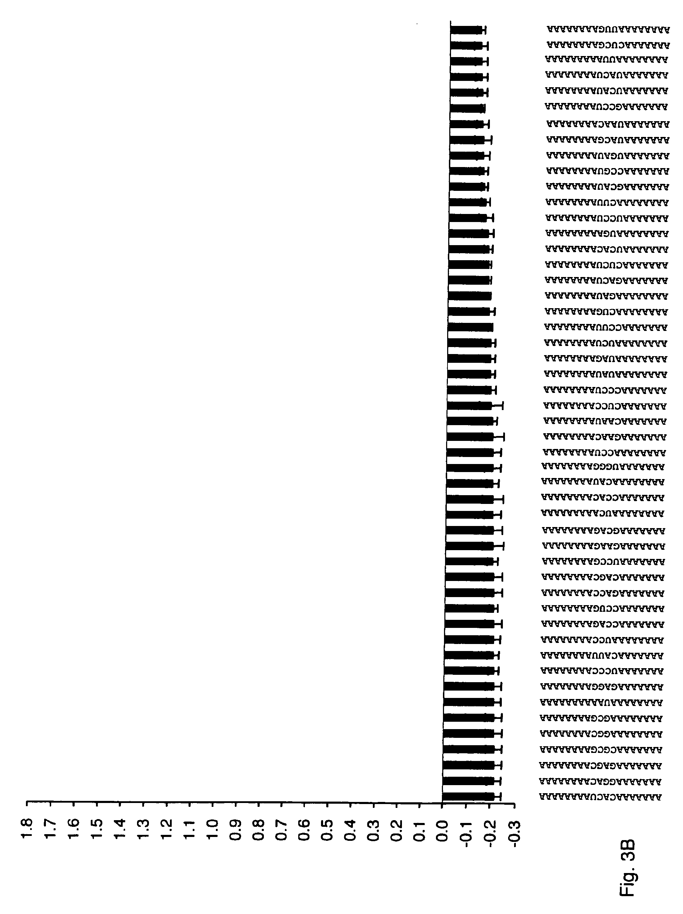

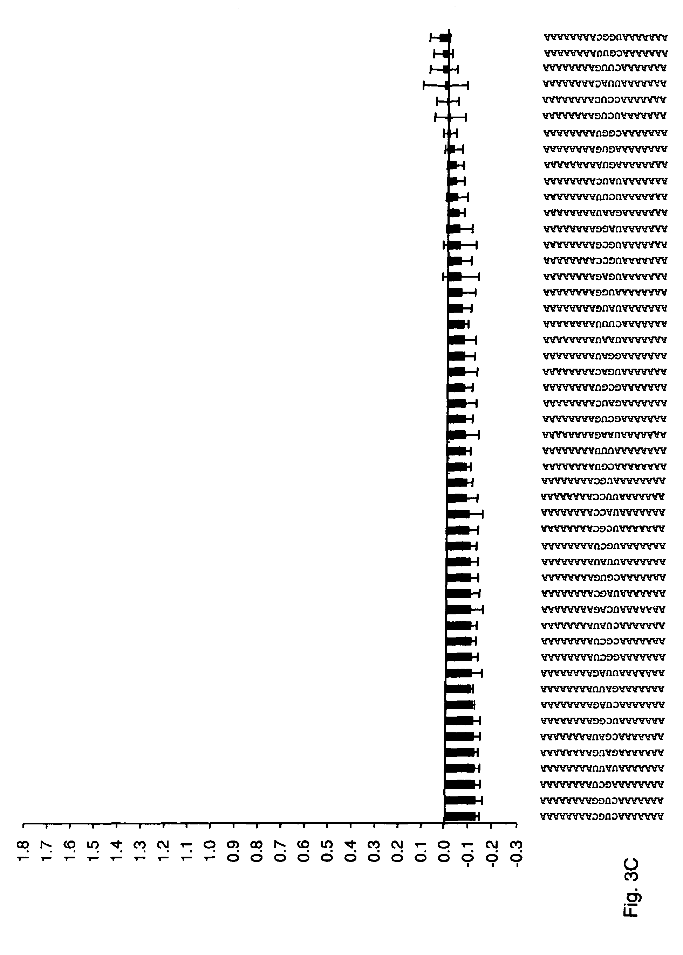

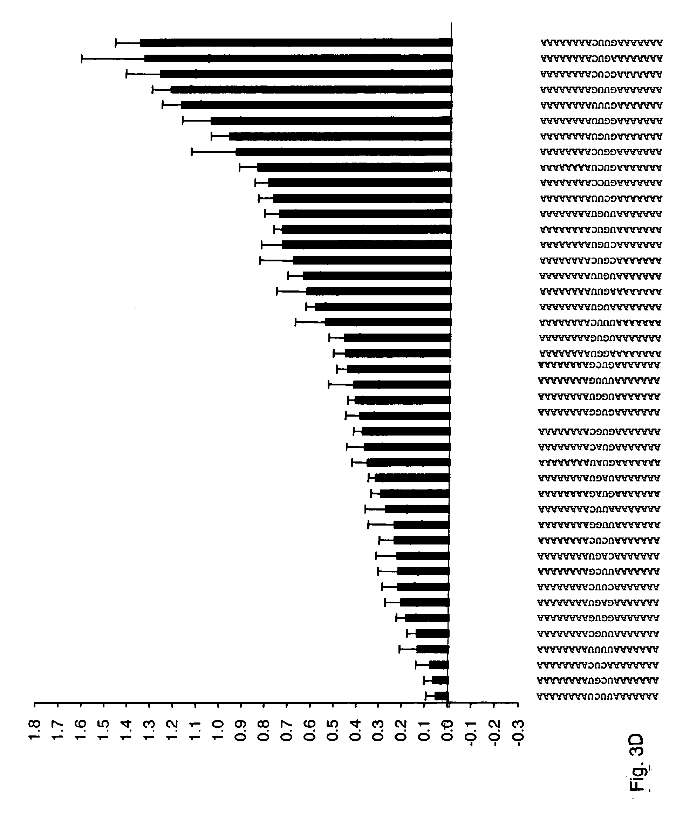

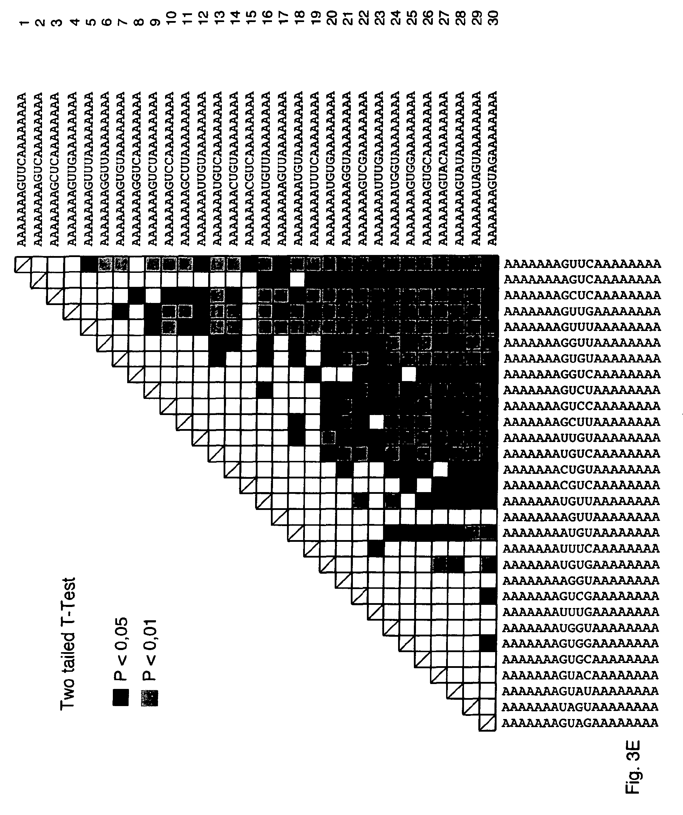

Figure 3: PBMC of six different healthy donors were isolated and stimulated with poly-L-arginine complexed ssRNA oligonucleotides in duplicates. 44 hours after stimulation IFN-a production was assessed in supernatant via ELISA. For all tested ssRNA oligonucleotides, the mean values of the measured duplicates were normalized to the positive control ssRNA oligonucleotide 9.2sense (5'-AGCUUAACCUGUCCUUCAA) by dividing the mean value of tested oligonucleotide by the mean value of 9.2sense (= IFN-a index of a given oligonucleotide). Next, all individual IFN-a indices were adjusted to the mean value of all IFN-a indices by subtracting the mean value of all IFN-a indices from the individual IFN-a index of a given oligonucleotide (= adjusted IFN-a index). Data from six individual donors were summarized and were assorted in ascending order displaying the corresponding SEM. In addition, a statistical analysis was performed to assess a putative significant difference for the adjusted IFN-α indices of all top thirty ssRNA oligonucleotides. A two-tailed Student's t-test was employed to calculate the p-value off all possible ssRNA oligonucleotide combinations. A p-value > 0,01 and < 0,05 is depicted by a black box, whereas a p-value < 0,01 is depicted as a grey box.

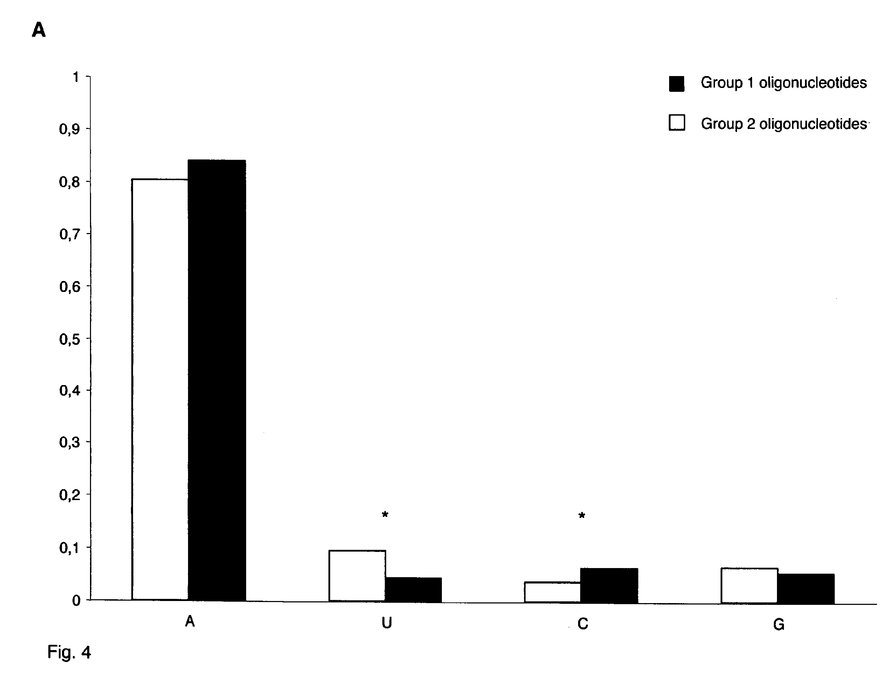

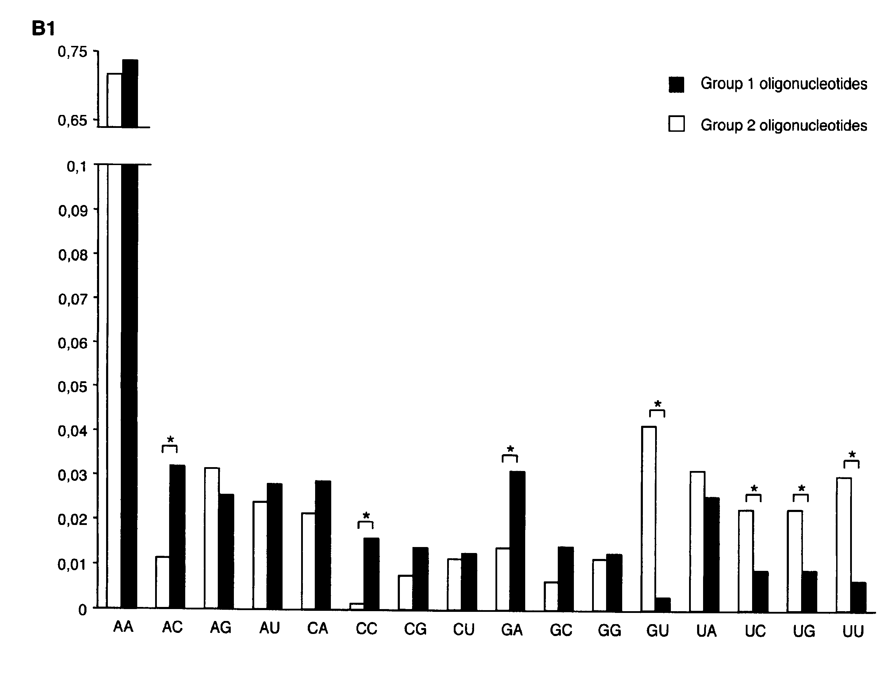

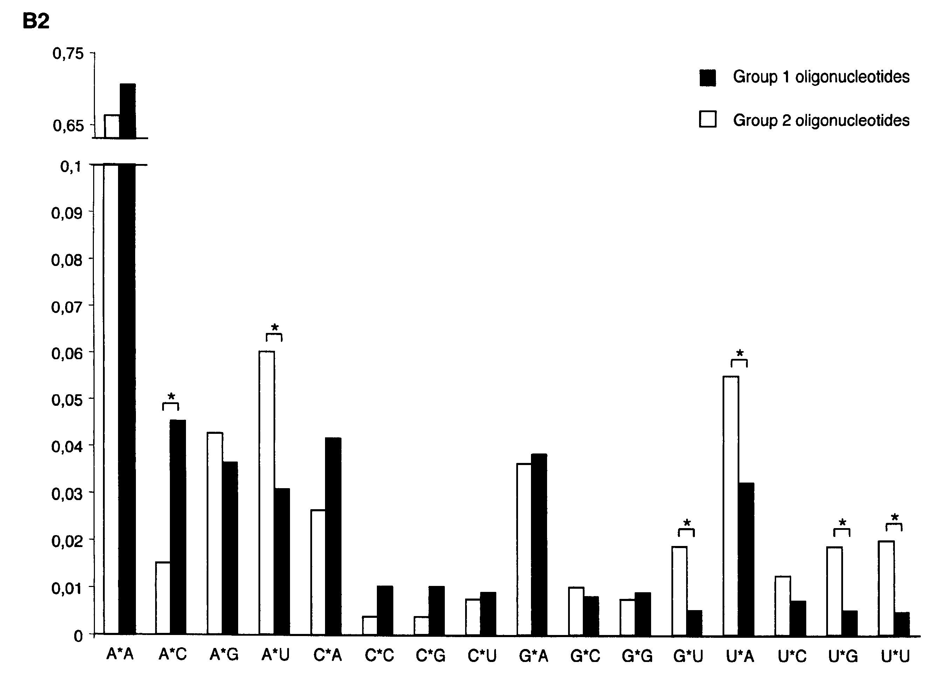

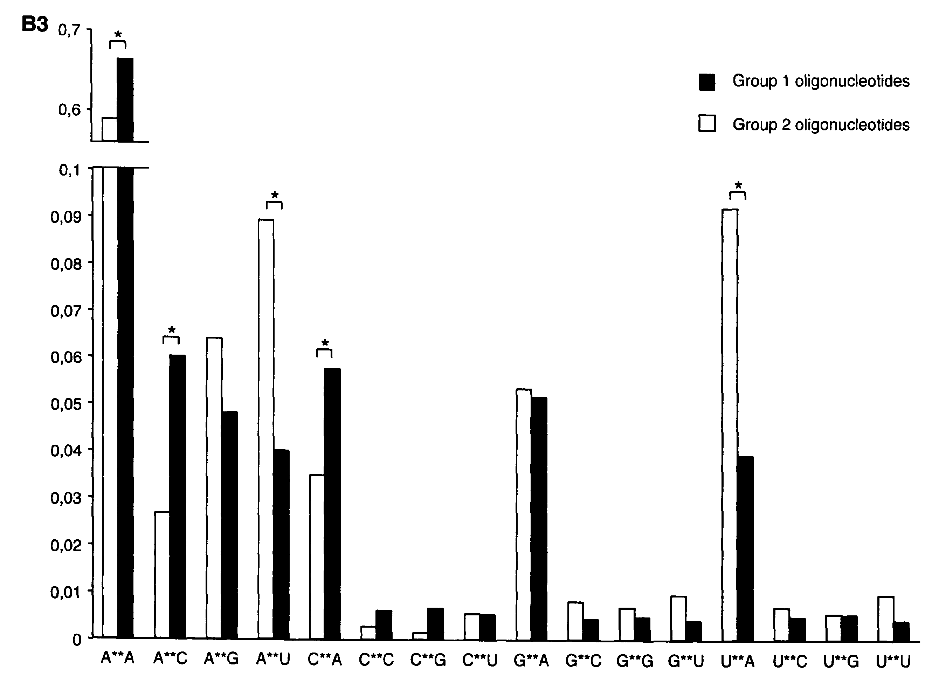

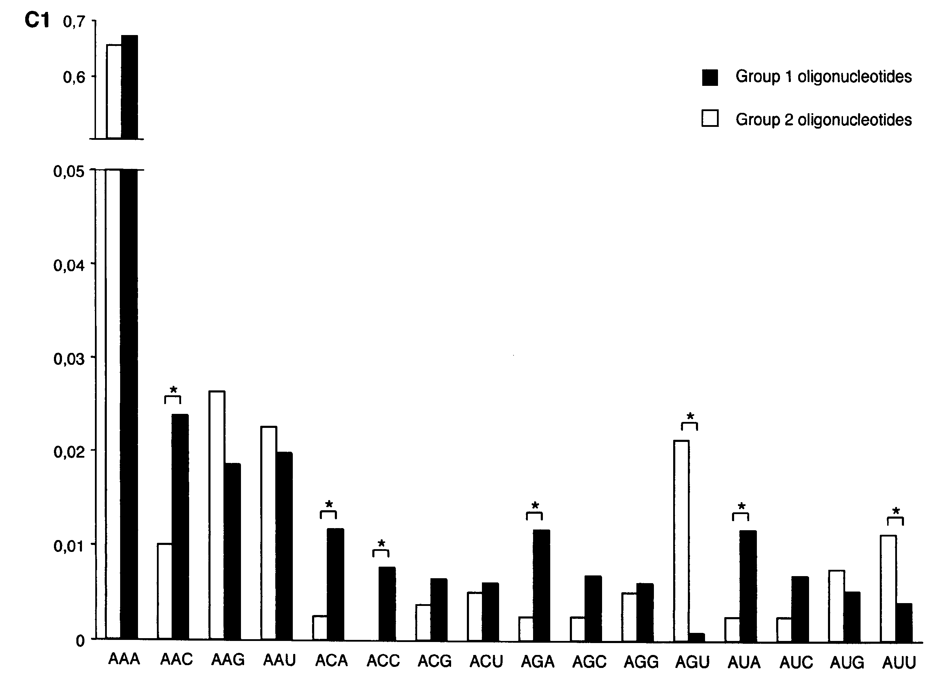

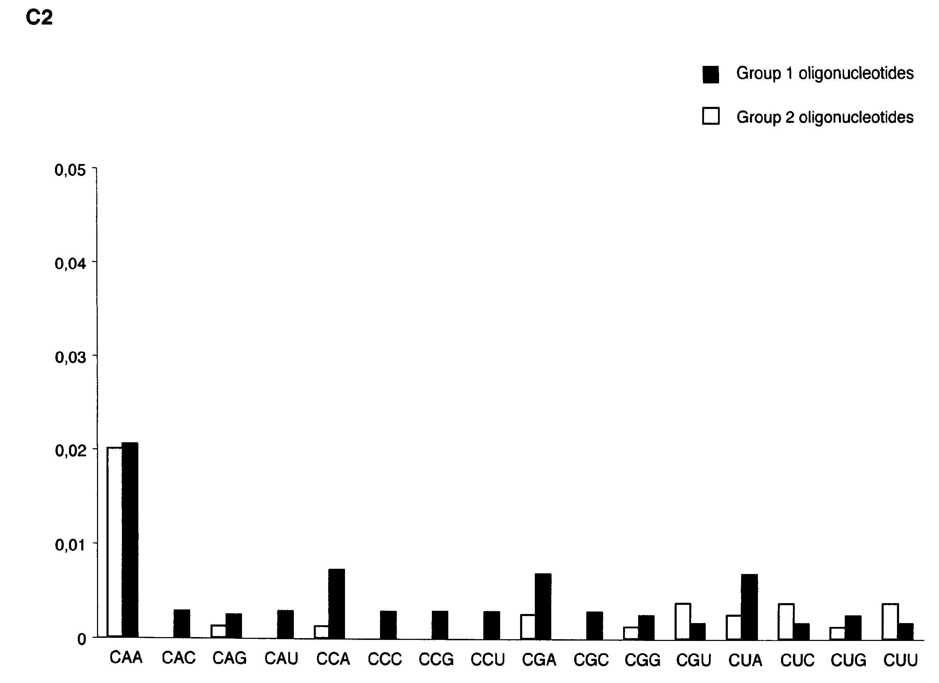

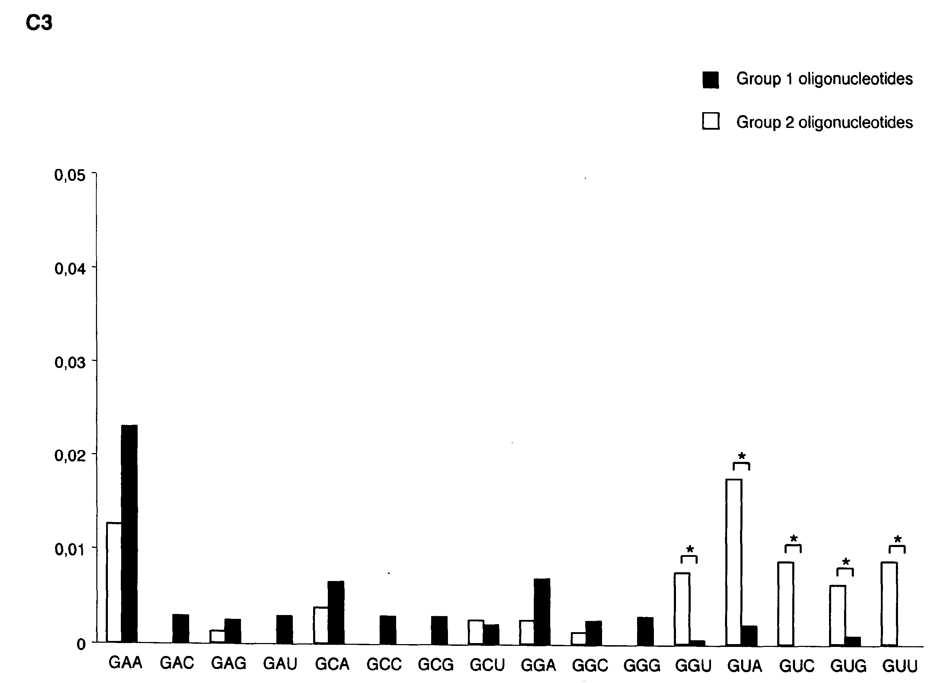

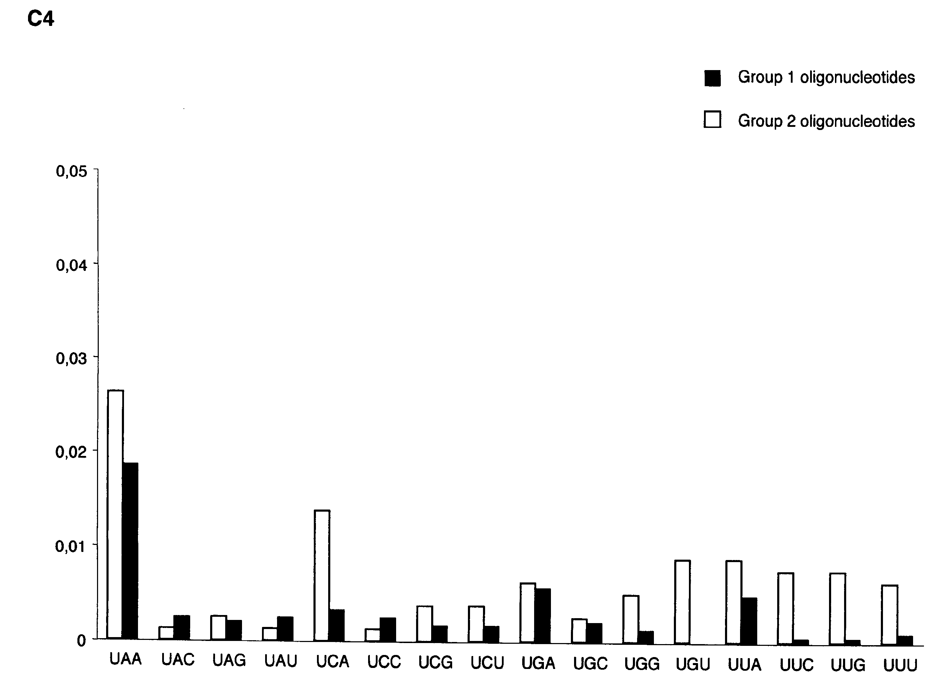

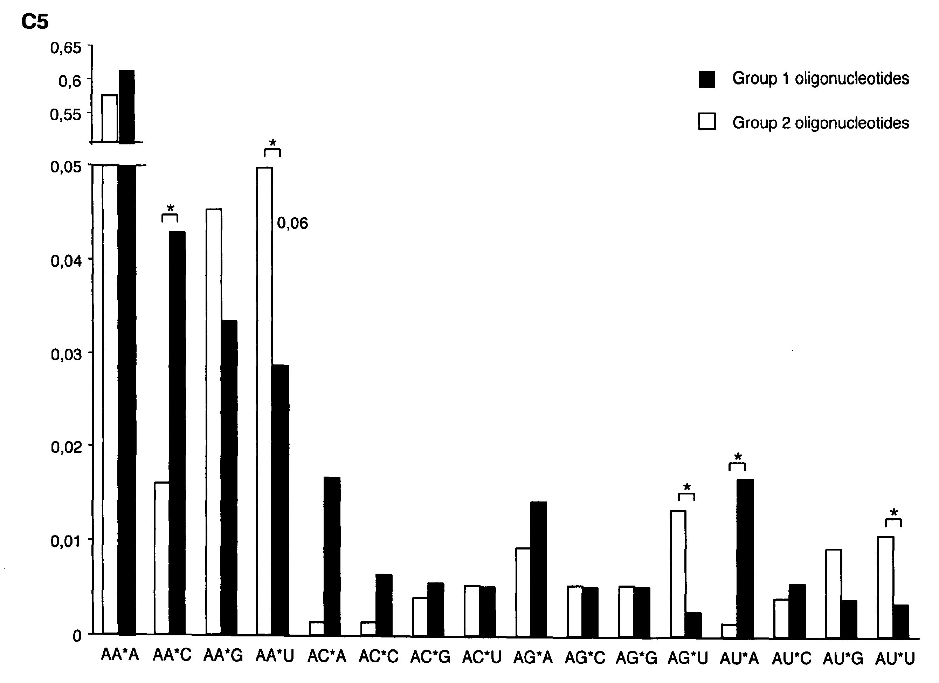

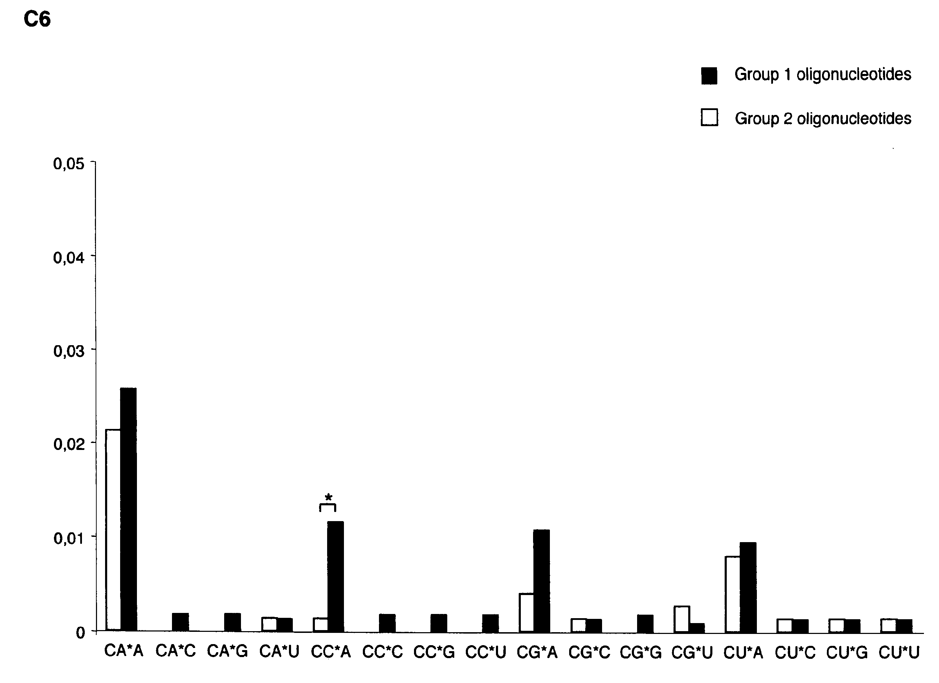

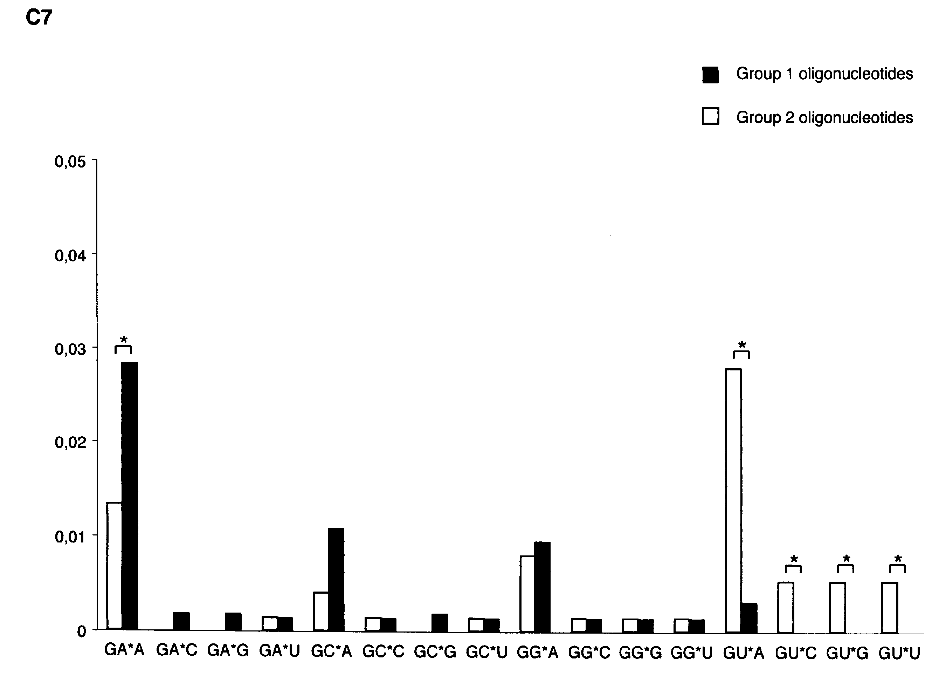

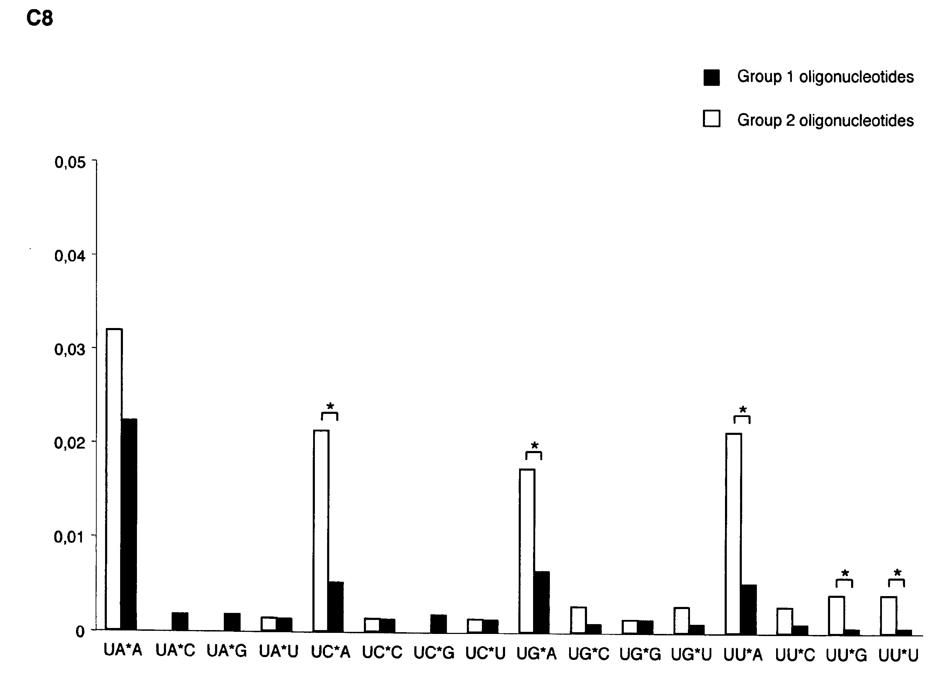

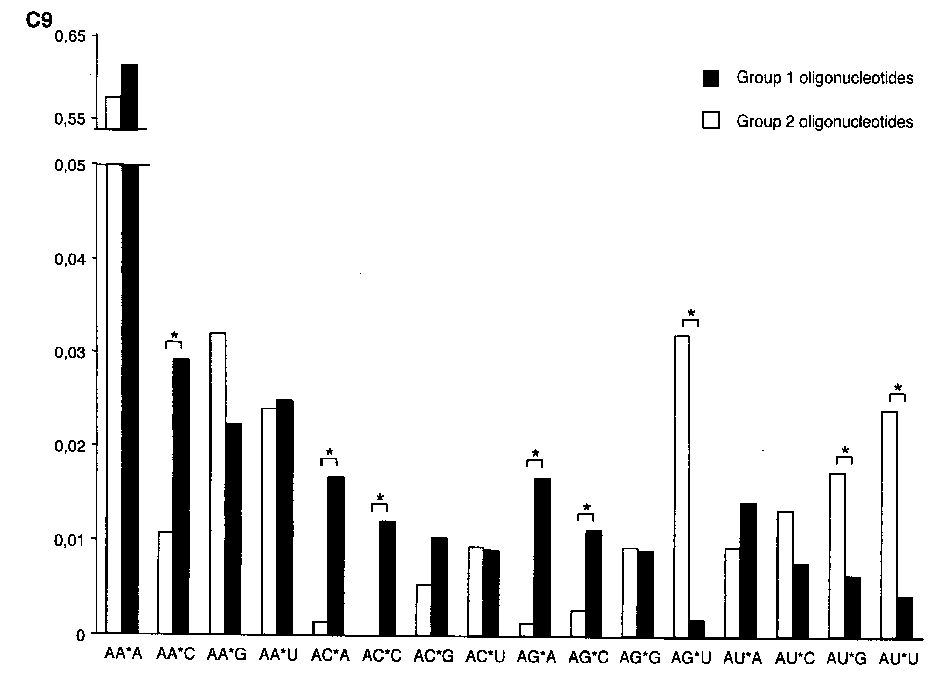

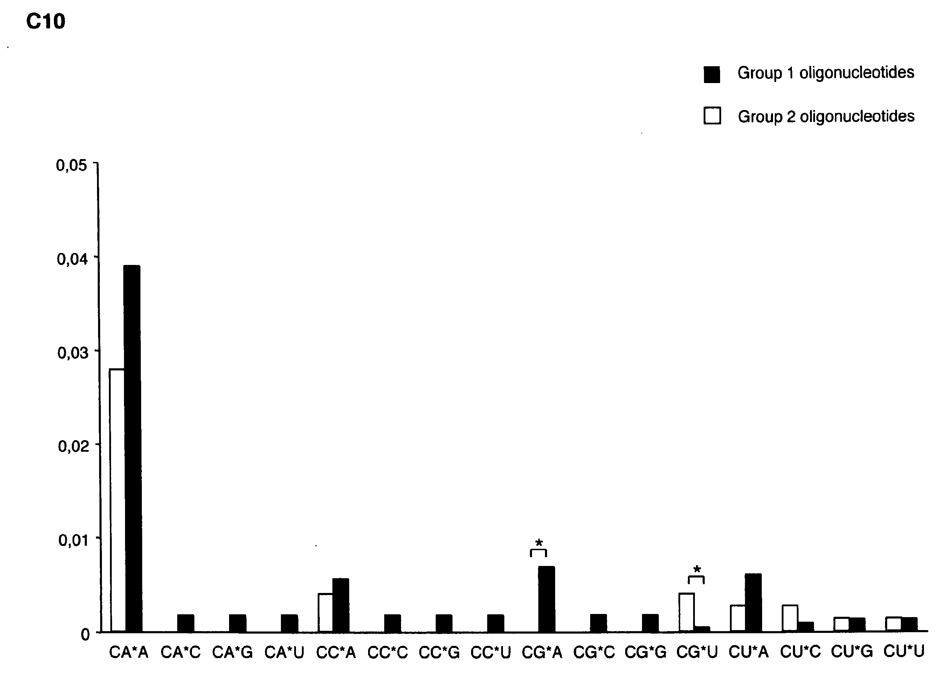

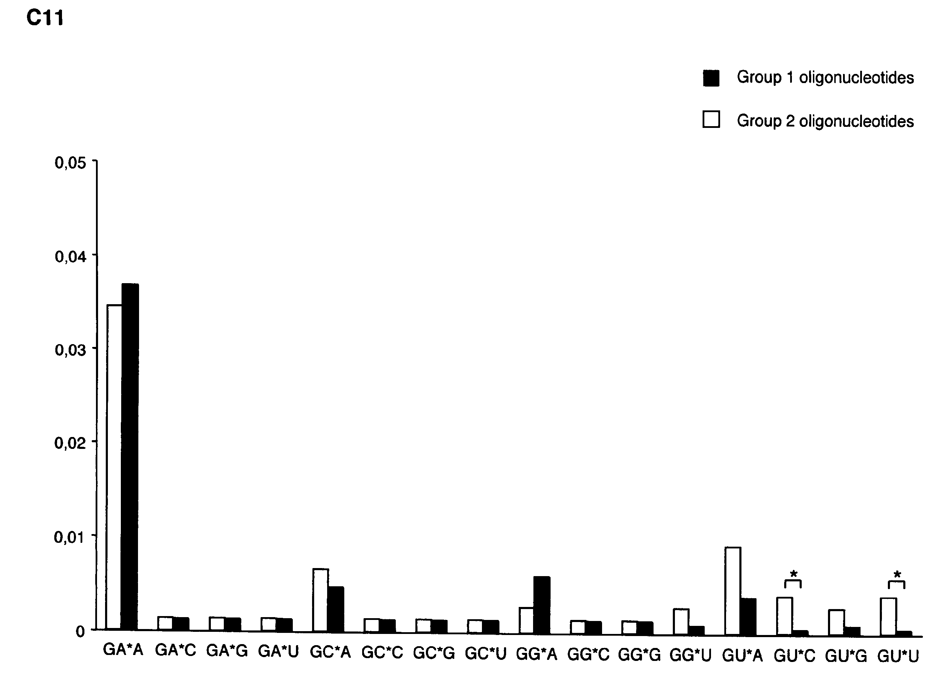

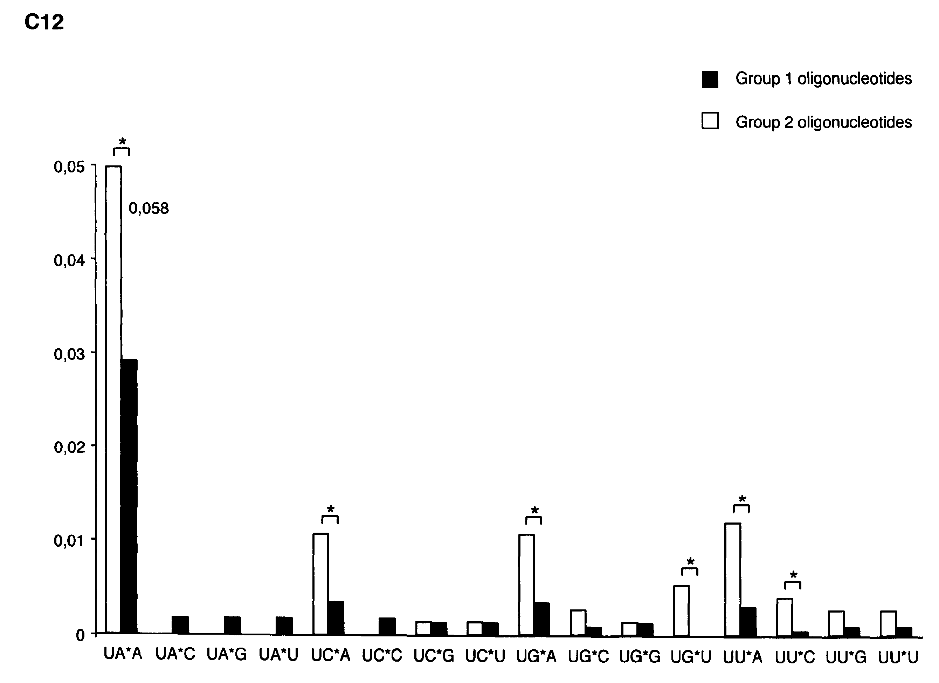

Figure 4: The occurrence of 1mer motifs (5'-X-3'), 2mer motifs (5'-XX-3', 5'-X*X-3', 5'-X**X-3') and 3mer motifs (5'-XXX-3', 5'-XX*X-3', 5'-X*XX-3') in ssRNA oligonucleotides with an IFN-a index below the mean IFN-a index (group 1) or above the mean IFN-α index (group 2) was analyzed. The relative occurrence of a given motif within a group of ssRNA oligonucleotides was calculated by dividing the absolute number of occurrences of a given motif within a group through the absolute number of occurrences of all possible motifs within this group. A significant overrepresentation or underrepresentation of a given motif was analyzed using a chi-square test. The null hypothesis of equal distribution within both groups was rejected when the calculated p-value was below 0.05 (significant differences in distribution are indicated by "*"). For all motifs analyzed, relative occurrences are depicted in Figure 4 for group 1 oligonucleotides (black bars) and for group 2 oligonucleotides (white bars): 1 mer motifs 5'-X-3' (Figure 4A); 2mer motifs 5'-XX-3' (Figure 4B1), 5'-X*X-3' Figure (Figure 4B2), 5'-X**X-3' (Figure 4B3) and 3mer motifs 5'-XXX-3' (Figure 4C1-4C4), 5'-XX*X-3' (Figure 4C5-4C8), 5'-X*XX-3' (Figure 4C9-4C12).

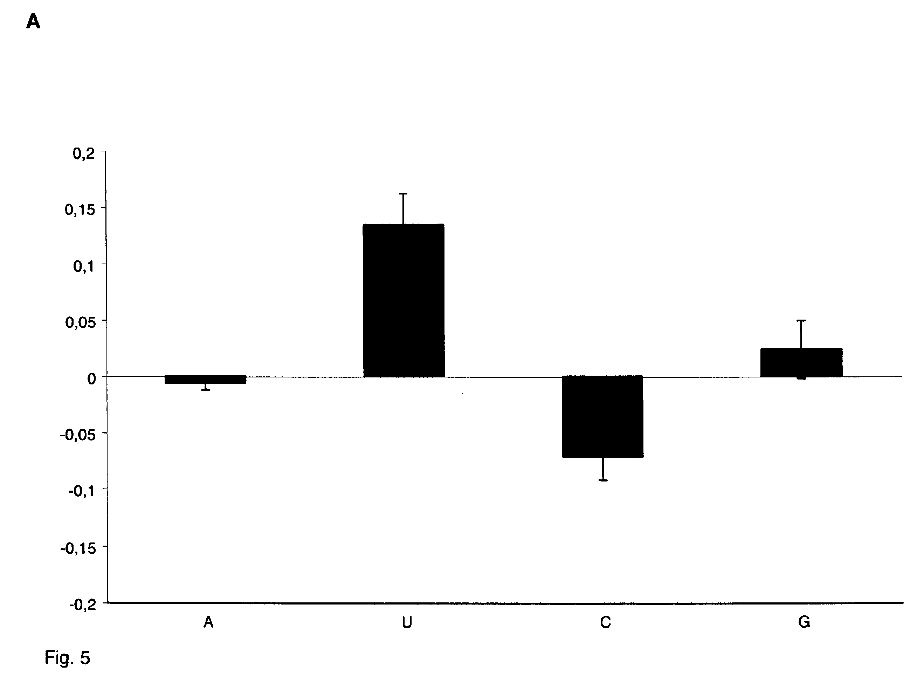

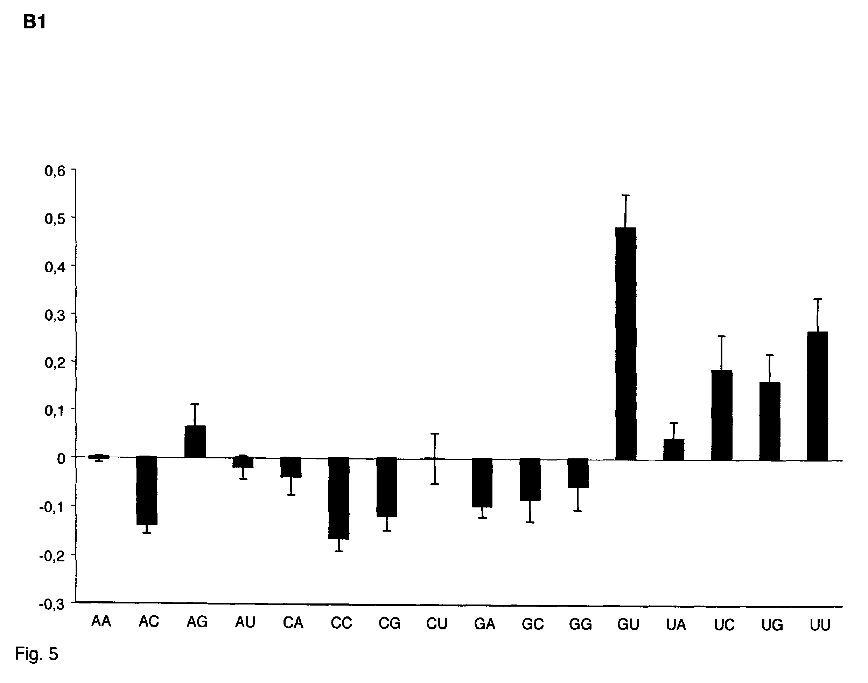

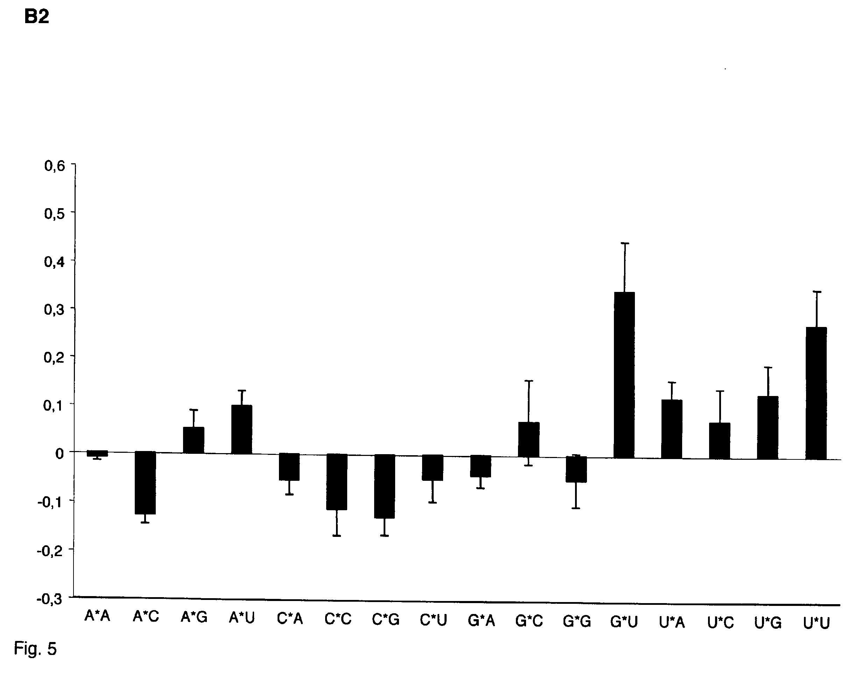

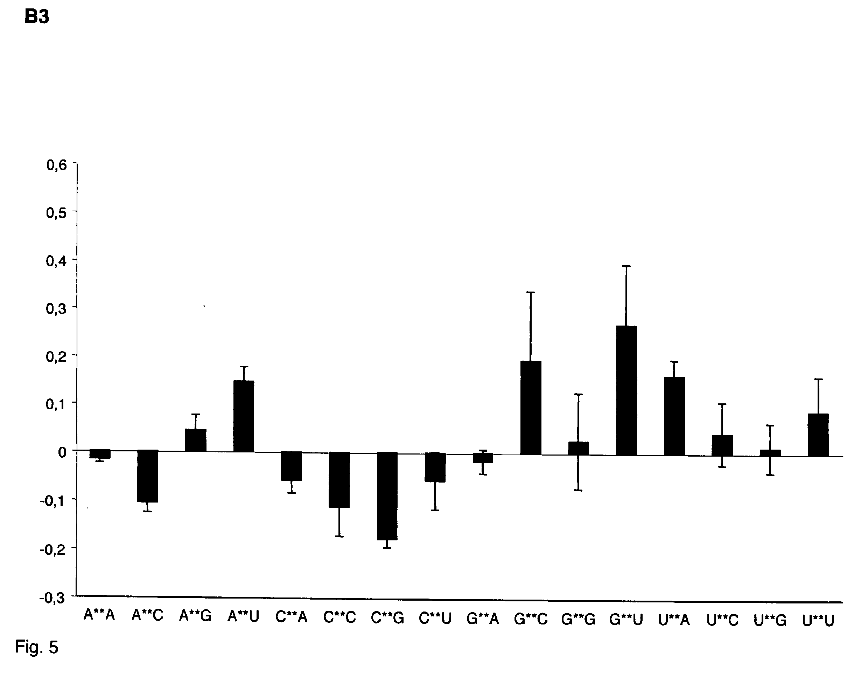

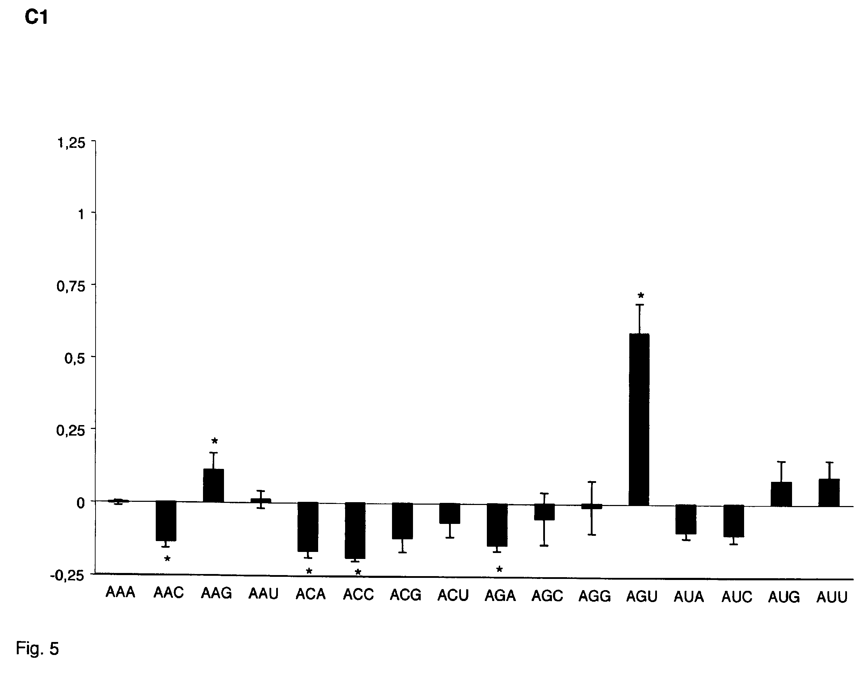

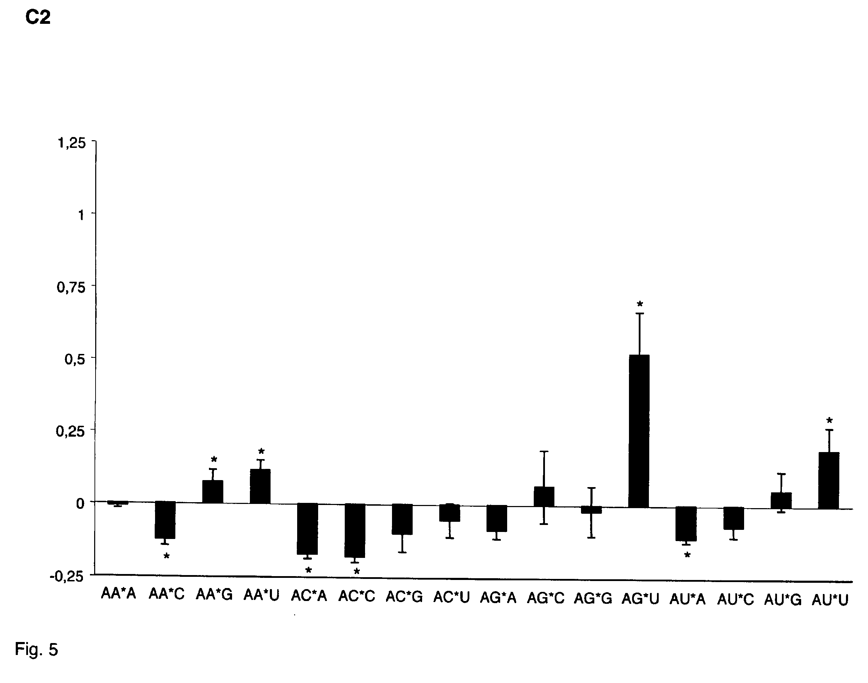

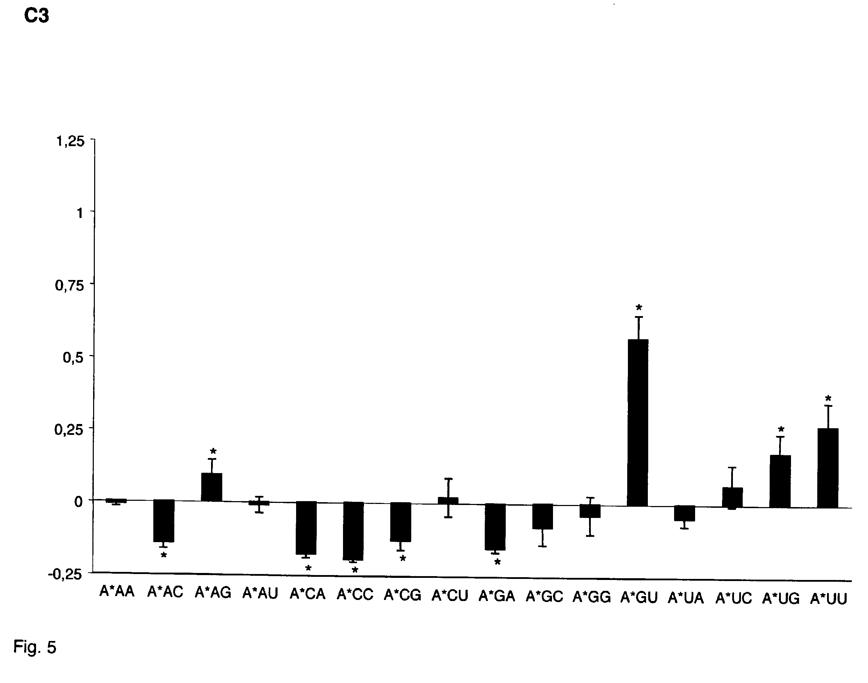

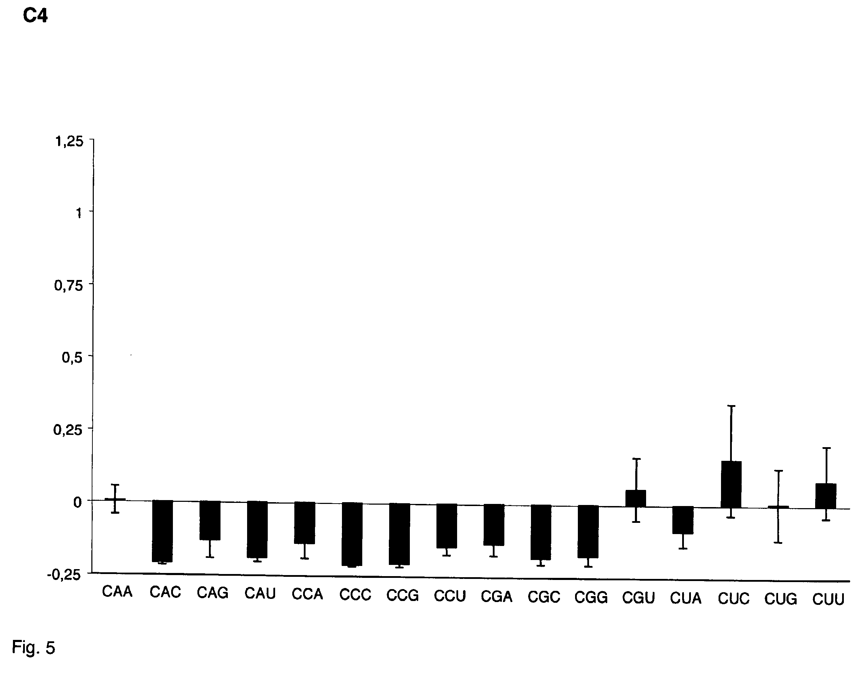

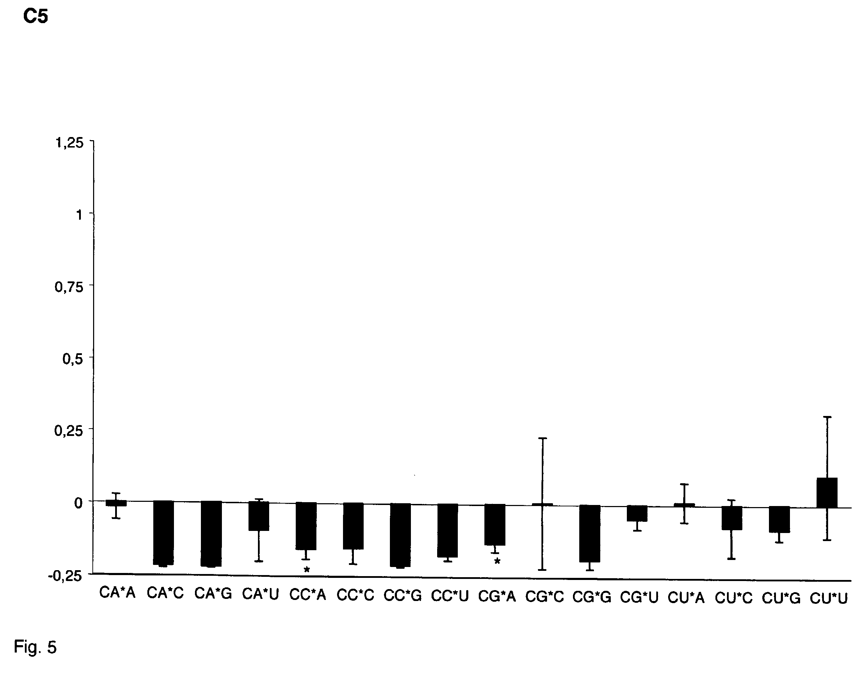

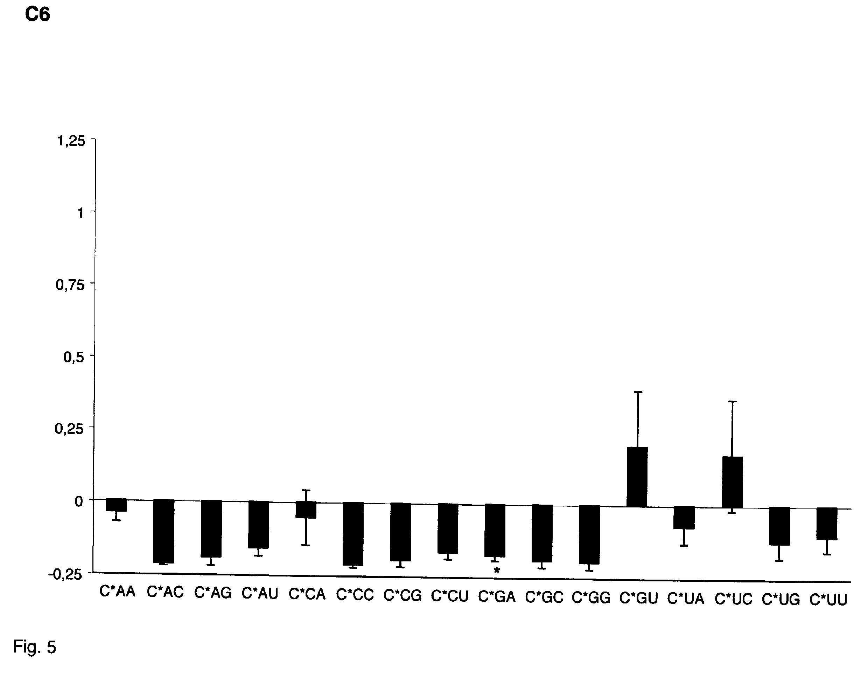

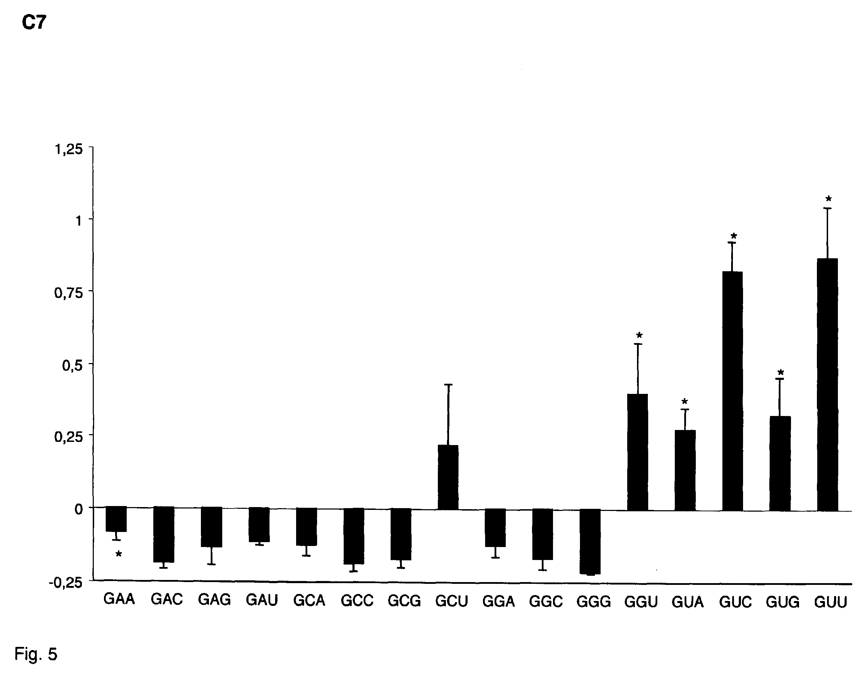

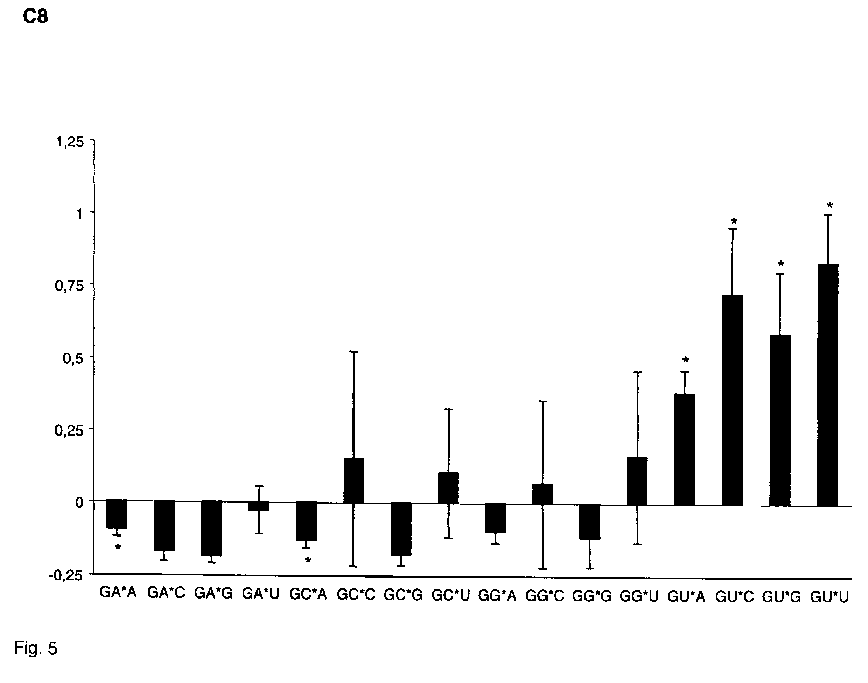

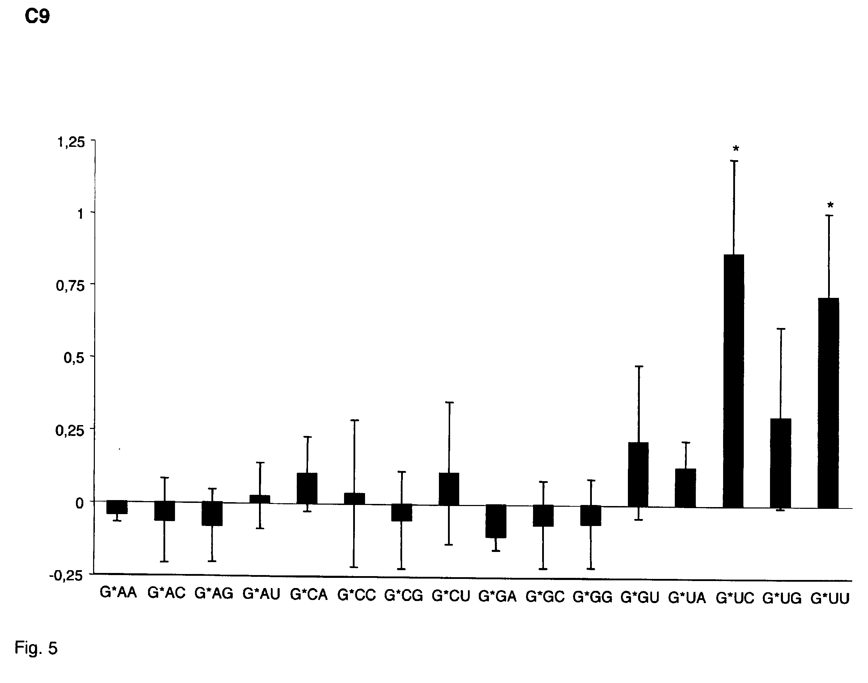

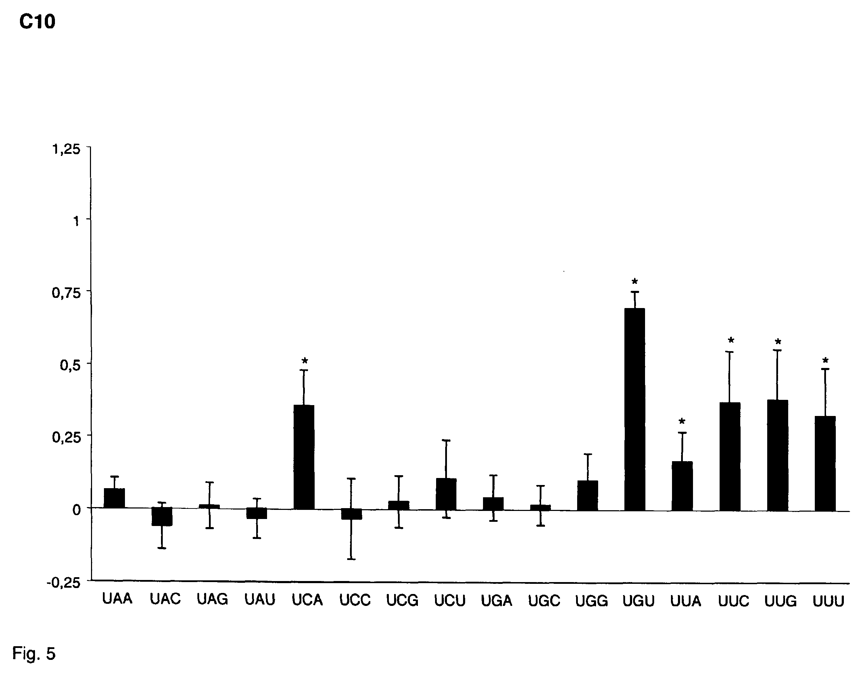

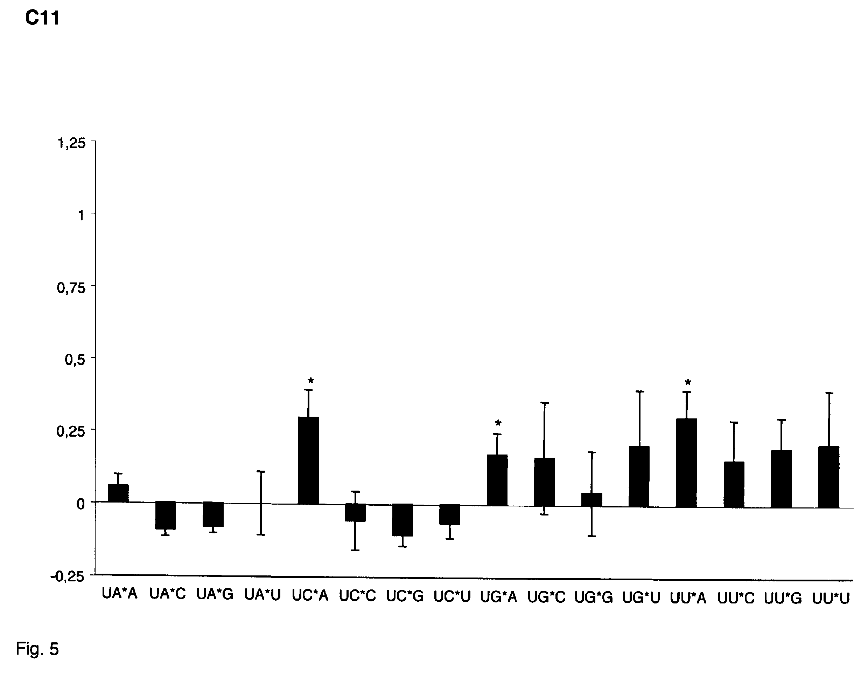

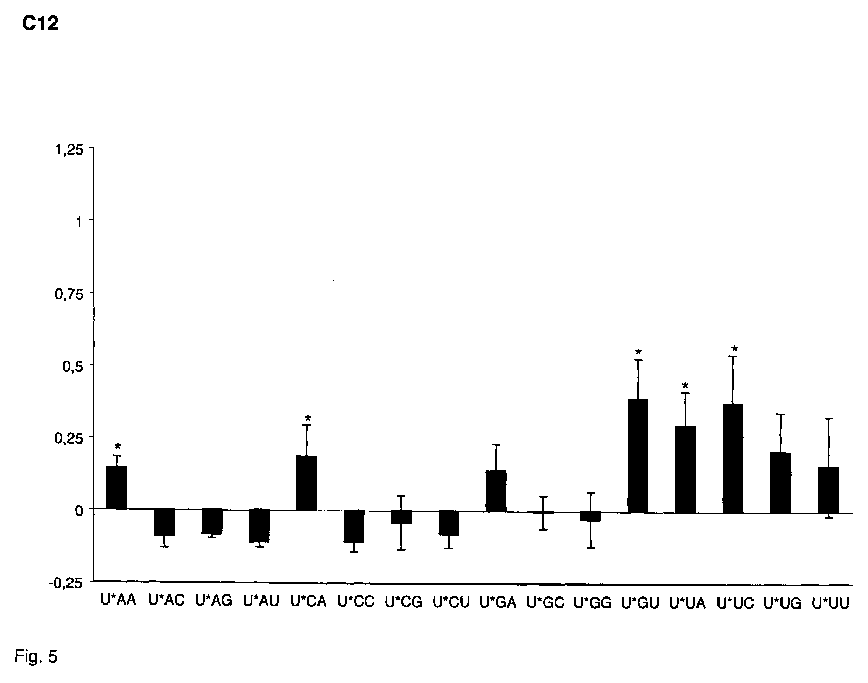

Figure 5: For all possible 1 mer motifs (5'-X-3'), 2mer motifs (5'-XX-3', 5'-X*X-3', 5'-X**X-3') or 3mer motifs (5'-XXX-3', 5'-XX*X-3', 5'-X*XX-3') a mean IFN-a index was assigned by calculating a mean IFN-α index of all ssRNA oligonucleotides containing the corresponding motifs (= IFN-a score of a given motif). The IFN-α score of all possible motifs is depicted in Figure 5 ± SEM: 1 mer motifs 5'-X-3' (Figure 5A); 2mer motifs 5'-XX-3' (Figure 5B1), 5'-X*X-3' Figure (Figure 5B2), 5'-X**X-3' (Figure 5B3) and 3mer motifs 5'-XXX-3' (Figure 5C1-5C4), 5'-XX*X-3' (Figure 5C5-5C8), 5'-X*XX-3' (Figure 5C9-5C12).

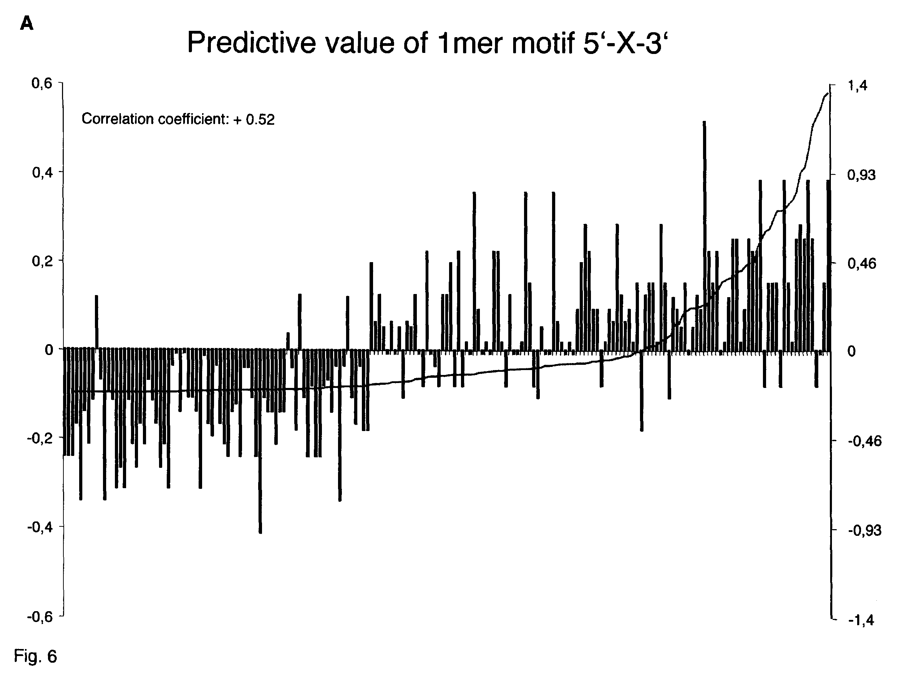

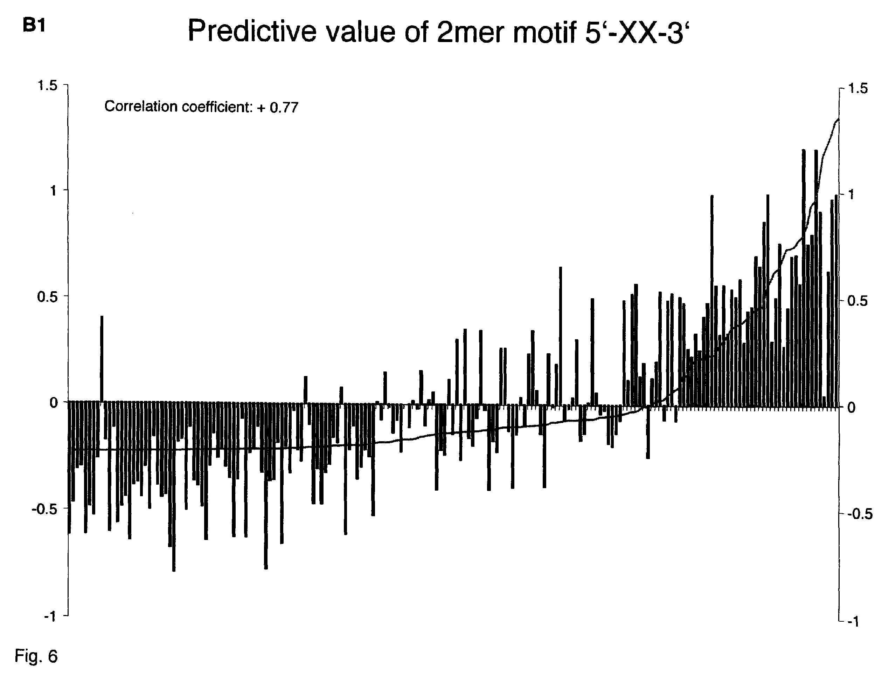

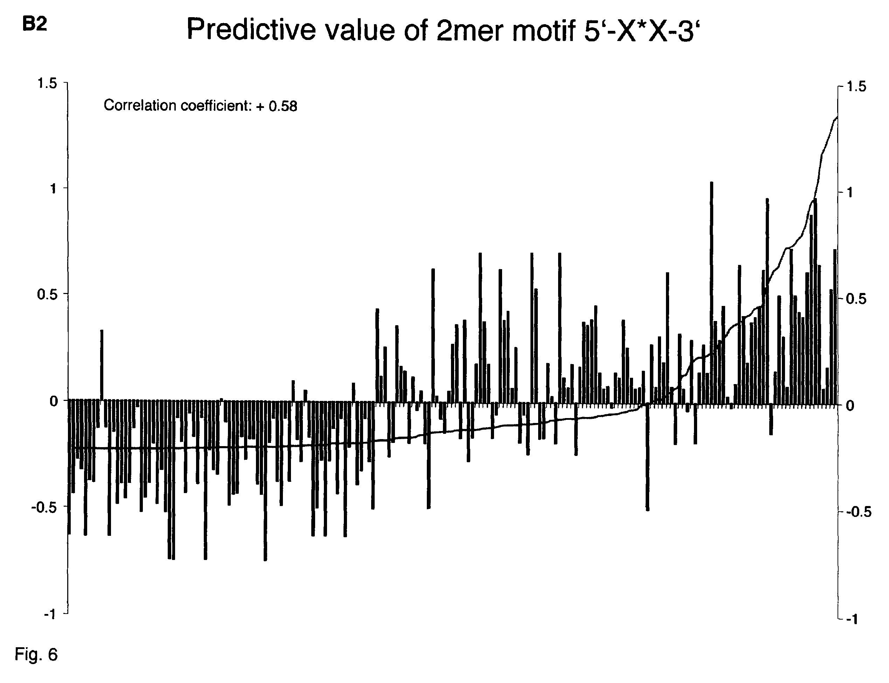

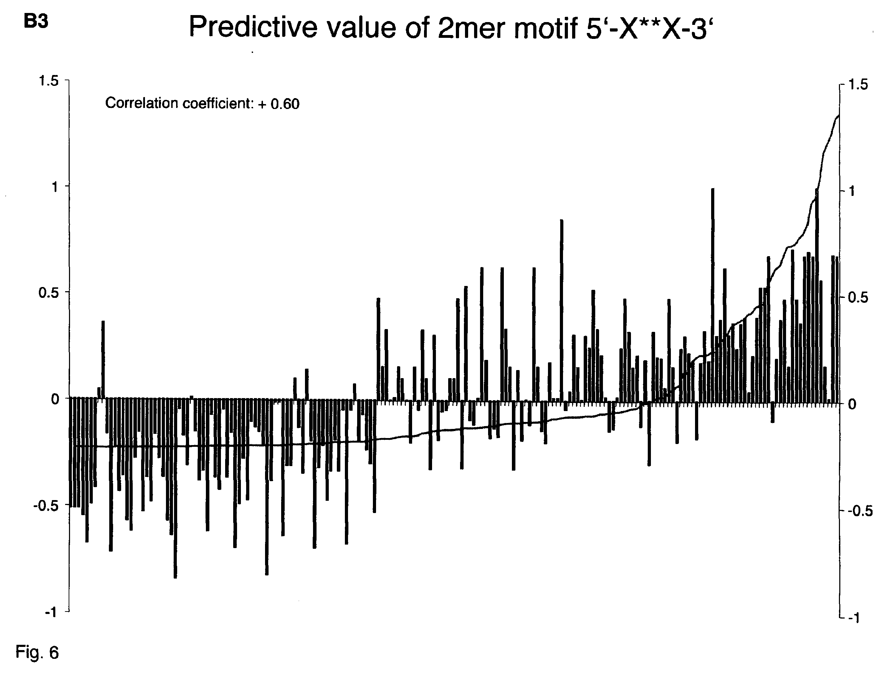

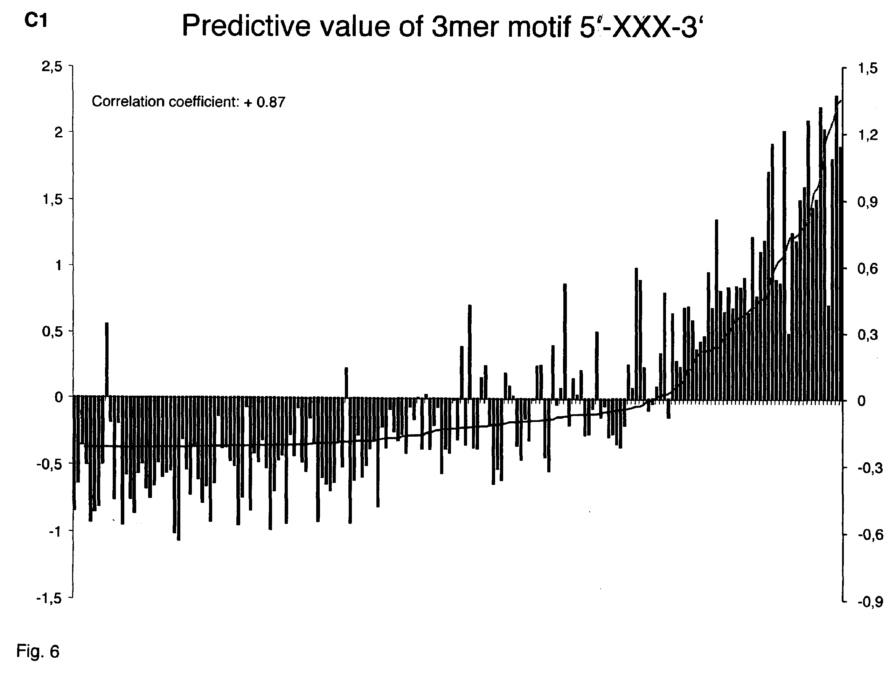

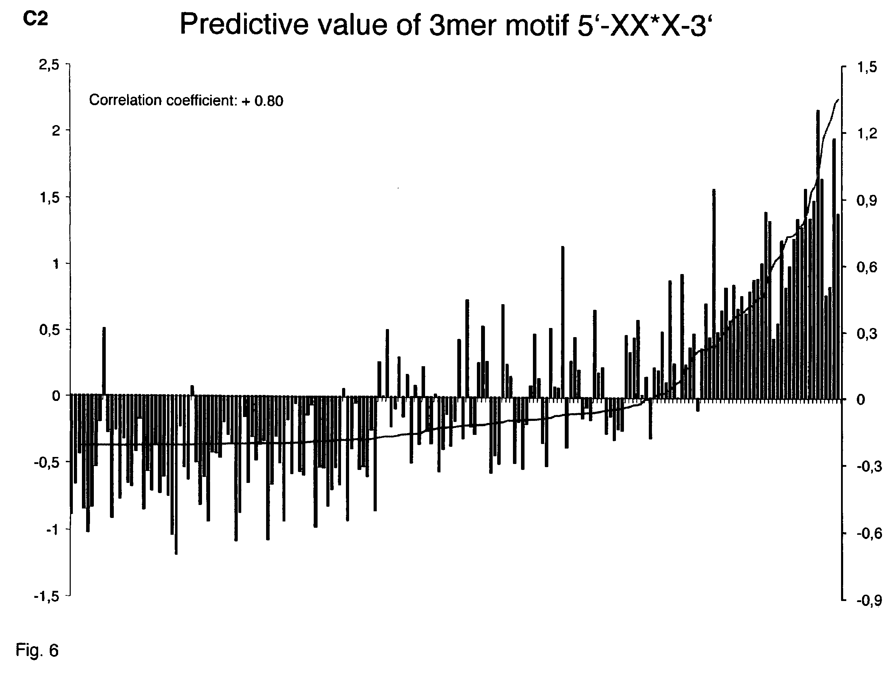

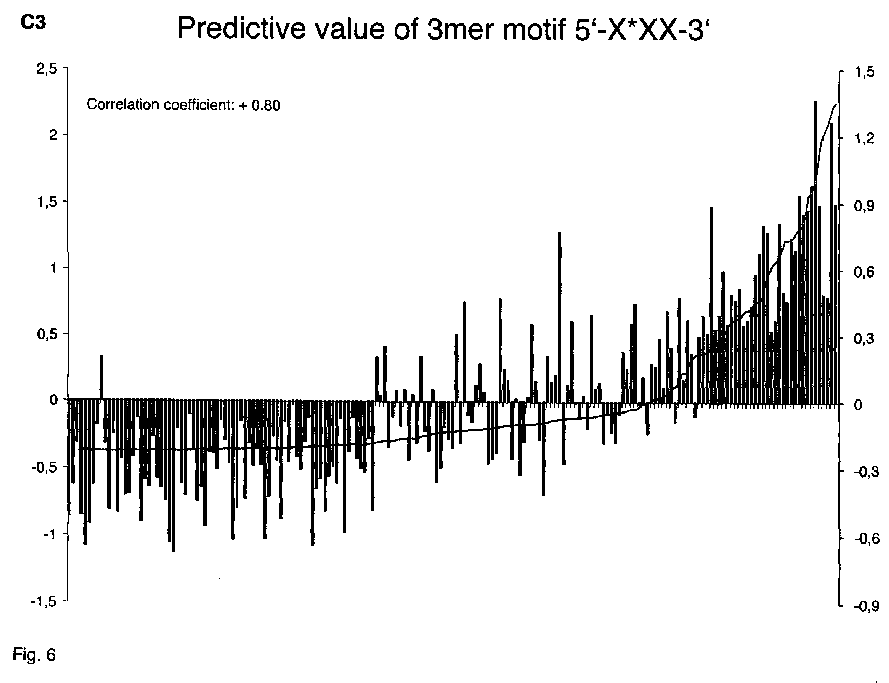

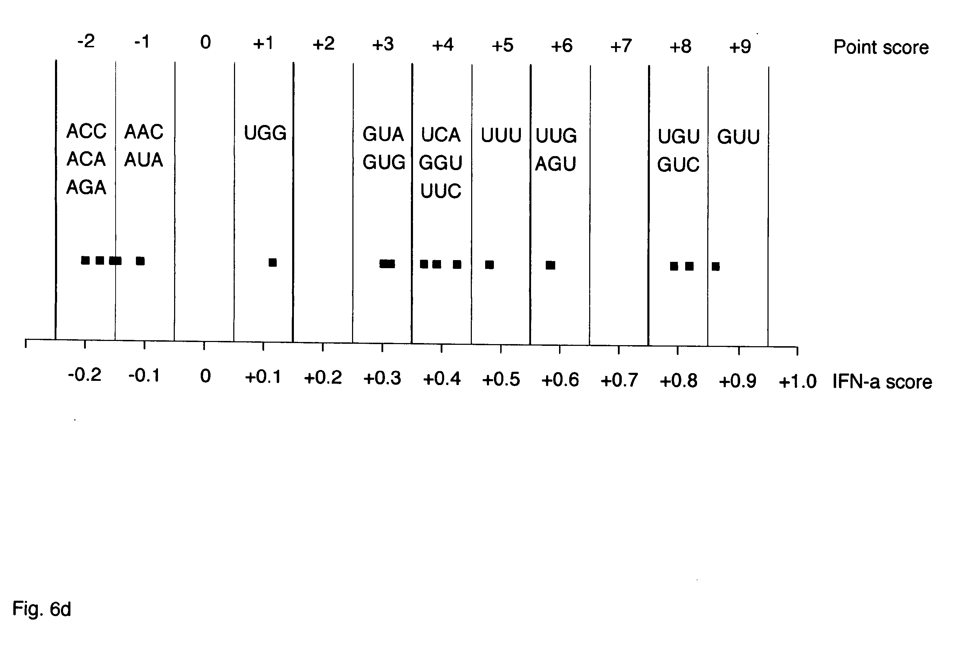

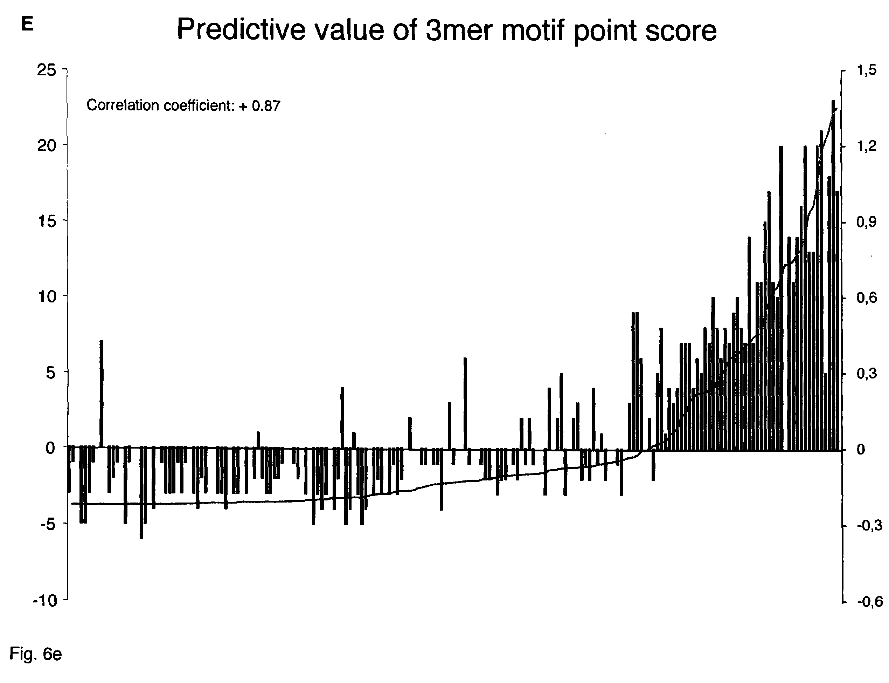

Figure 6: A calculated IFN-α index was assigned to each oligonucleotide by using the obtained motif-IFN-a scores. For each set of motifs [1mer motifs (5'-X-3'), 2mer motifs (5'-XX-3', 5'-X*X-3', 5'-X**X-3') or 3mer motifs (5'-XXX-3', 5'-XX*X-3', 5'-X*XX-3'] a predicted IFN-a index was calculated for each ssRNA oligonucleotide. Next, the obtained predicted IFN-a indices were compared to the actual adjusted IFN-α, indices. Data are depicted the following way: For all ssRNA oligonucleotides the predicted IFN-α indices are shown as a black bars, whereas data are sorted in ascending order according to the actual IFN-α score that is depicted as a red index line. The y-axis on the left side depicts the scale for the predicted IFN-α score, while the y-axis on the right side depicts the scale for the actual IFN-α score.

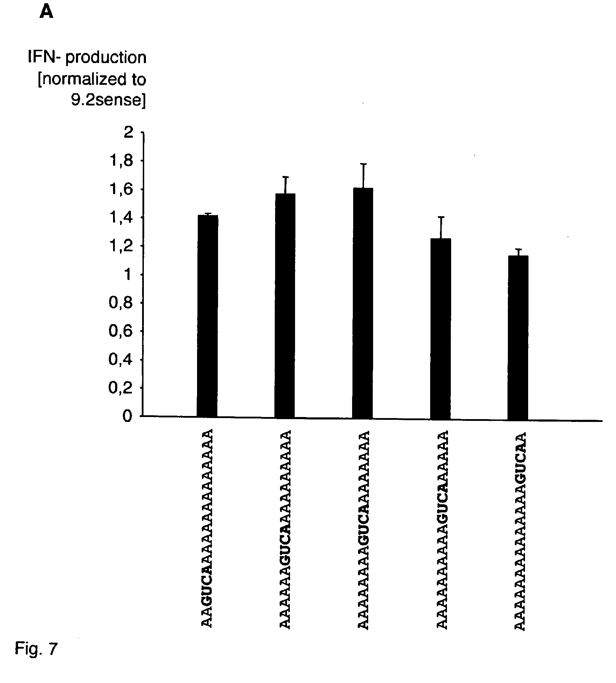

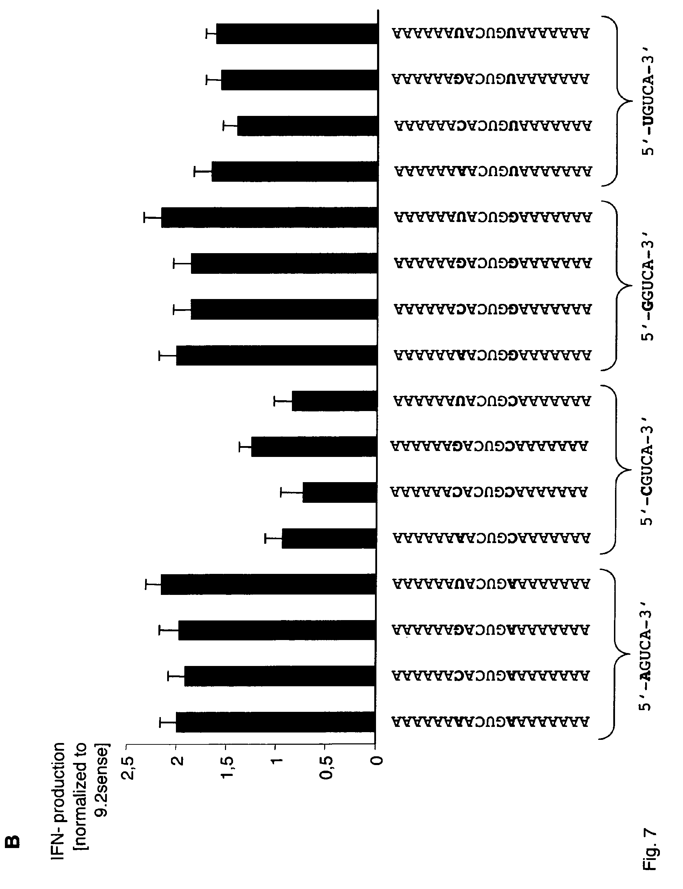

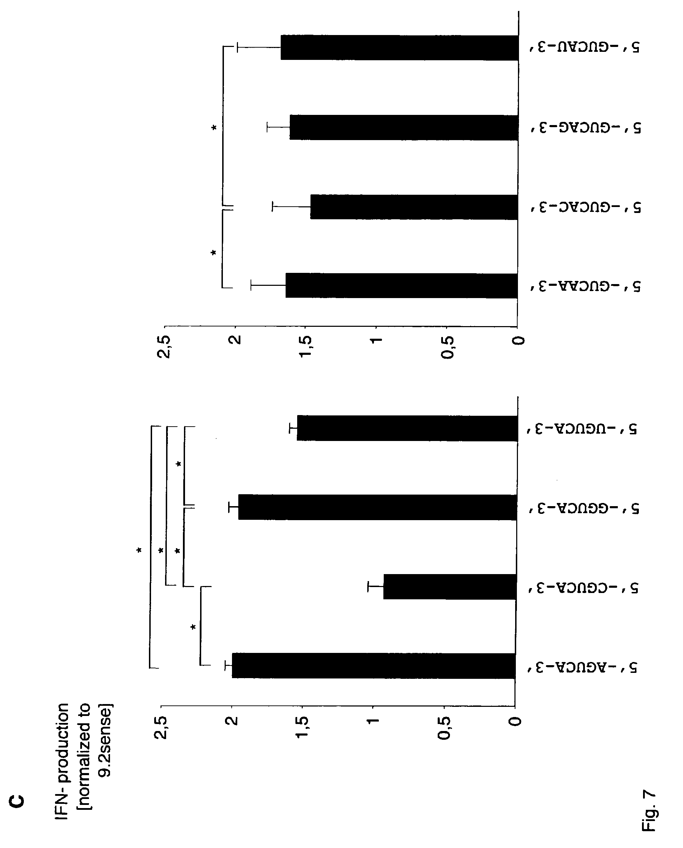

Figure 7: PBMC from healthy donors were isolated and stimulated with poly-L-arginine complexed ssRNA oligonucleotides in duplicates. 44 hours after stimulation IFN-a production was assessed in supernatant via ELISA. For all tested ssRNA oligonucleotides, the mean values of the measured duplicates were normalized to the positive control ssRNA oligonucleotide 9.2sense (5'-AGCUUAACCUGUCCUUCAA) by dividing the mean value of tested oligonucleotide by the mean value of 9.2sense (= 1). A: A panel of ssRNA oligonucleotides was tested with different positions of the 5'-GUCA-3'-motif within the 19mer ssRNA oligonucleotide (see table 4). The 5'-GUCA-3'-motif is indicated by bold letters. Data from two independent donors were summarized and are depicted as mean values ± SEM. B/C: 16 ssRNA oligonucleotides, which included all possible oligonucleotides with permutated bases at the flanking positions to the 5'- and the 3'-end of the central 5'-GUCA-3'-motif (table 5), were complexed with poly-L-arginine and used to stimulate PBMC. 44 hours after stimulation IFN-a production was assessed in supernatant via ELISA. Data for all 16 oligonucleotides from three independent donors were summarized as mean values ± SEM (B). In addition all 16 oligonucleotides were assorted into groups according to the base preceding or following the central 5'-GUCA-3'-motif (C). On the left side oligonucleotides with a common base preceding the central 5'-GUCA-3'-motif were grouped, whereas on the right side oligonucleotides with a common base following the central 5'-GUCA-3'-motif were grouped. Individual ssRNA oligonucleotide IFN-a data were summarized according to the respective group and are depicted as mean values ± SEM. A two-tailed Student's t-test was used to calculate a statistically significant difference between the various groups (p < 0,05 is indicated by a "*").

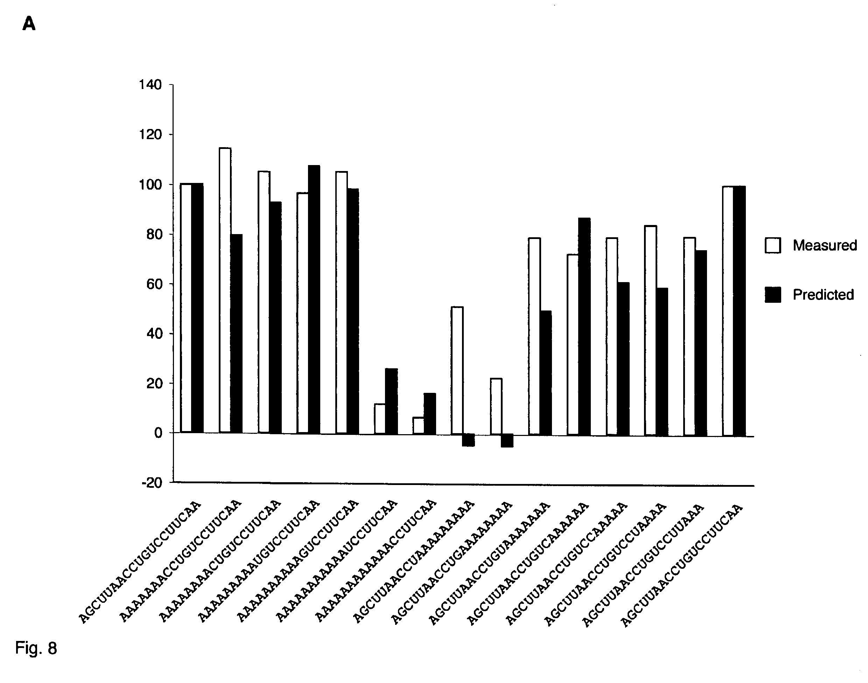

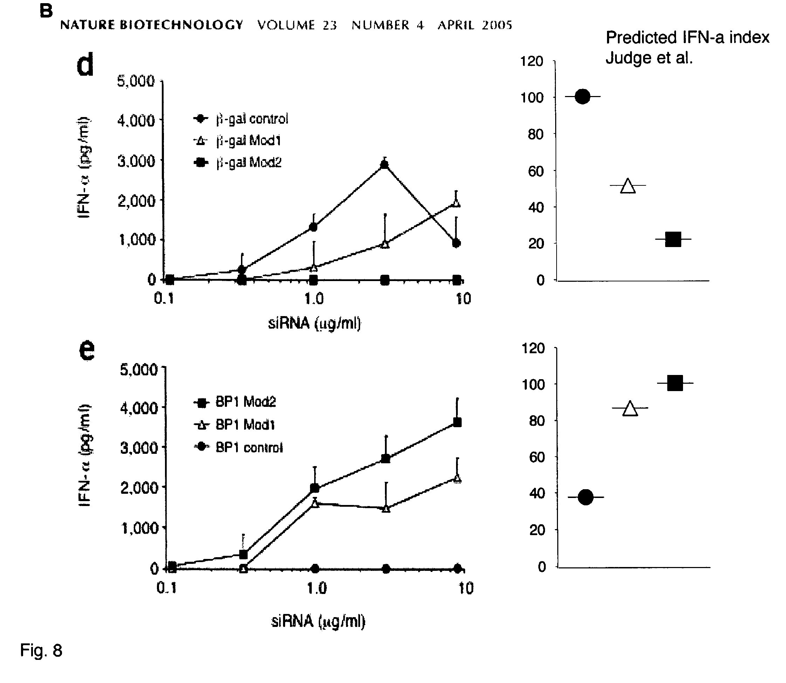

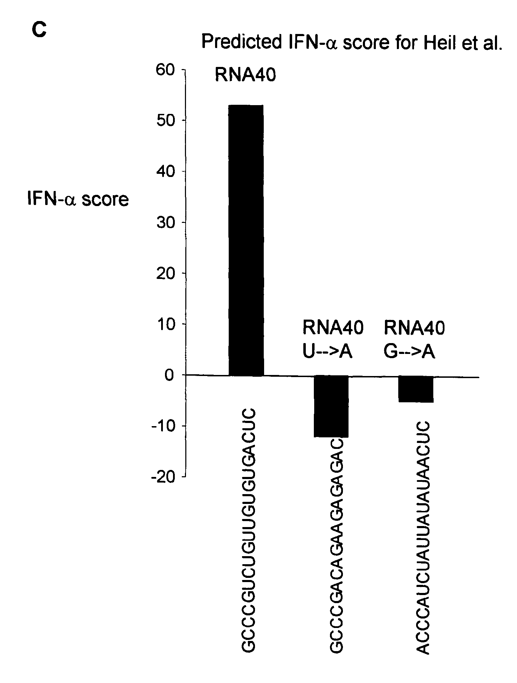

Figure 8: A: PBMC from two healthy donors were isolated and stimulated with poly-L-arginine complexed ssRNA oligonucleotides (Table 6) in duplicates. 44 hours after stimulation IFN-α production was assessed in supernatant via ELISA. IFN-α data were summarized as mean values and subsequently normalized to the positive control RNA9.2sense (5'-AGCUUAACCUGUCCUUCAA-3'). In addition, respective sequences were analyzed using the IFN-α point score matrix (Table 7) and subsequently normalized to RNA9.2sense. A correlation coefficient of 0.84 was calculated for these two sets of data. Measured IFN-α levels are depicted in white bars, whereas predicted IFN-α scores are shown in black bars (A). Next, IFN-α point score matrix was employed to analyze IFN-α-inducing RNA oligonucleotides that have been described in the literature. Given the fact that in the study performed by Judge et al. (2005, Nat Biotechnol 23:457-462) double-stranded RNA oligonucleotides were tested, a mean value for the individually analyzed single-stranded components was calculated. Data were normalized to the most potent RNA oligonucleotide (=100%) within the respective panel of oligonucleotides (B). For the prediction of single-stranded RNA oligonucleotides reported by Heil et al. (2004 Science 303:1526-1529), the predicted IFN-α point scores are depicted (C).

### Detailed Description of the Invention

### Definitions

As used herein, "a" and "an" refers to a group or species of entities, rather than one single individual.

### oligonucleotide

As used herein, the term "oligonucleotide" refers to a polynucleotide formed from a plurality of linked nucleoside units. Such oligonucleotides can be obtained from existing nucleic acid sources, including genomic or cDNA, but are preferably produced by synthetic methods including chemical synthesis, in vitro and in vivo transcription. In preferred embodiments each nucleoside unit includes a heterocyclic base and a pentofuranosyl, trehalose, arabinose, 2'-deoxy-2'-substituted arabinose, 2'-O-substituted arabinose or hexose sugar group. The nucleoside residues can be coupled to each other by any of the numerous known internucleoside linkages. Such internucleoside linkages include, without limitation, phosphodiester, phosphorothioate, phosphorodithioate, alkylphosphonate, alkylphosphonothioate, phosphotriester, phosphoramidate, siloxane, carbonate, carboalkoxy, acetamidate, carbamate, morpholino, borano, thioether, bridged phosphoramidate, bridged methylene phosphonate, bridged phosphorothioate, and sulfone internucleoside linkages. The term "oligonucleotide" also encompasses polynucleosides having one or more stereospecific internucleoside linkage (e.g., (Rp)- or (Sₚ)-phosphorothioate, alkylphosphonate, or phosphotriester linkages).

The oligonucleotides of the invention can include naturally occurring nucleosides, modified nucleosides, or mixtures thereof. As used herein, the term "modified nucleoside" is a nucleoside that includes a modified heterocyclic base, a modified sugar moiety, or a combination thereof. In some embodiments, the modified nucleoside is a non-natural pyrimidine or purine nucleoside, as herein described. In some embodiments, the modified nucleoside is a 2'-substituted ribonucleoside an arabinonucleoside or a 2'-deoxy-2'-substituted-arabinoside.

As used herein, the term "2'-substituted ribonucleoside" or "2'-substituted arabinoside" includes ribonucleosides or arabinonucleoside in which the hydroxyl group at the 2' position of the pentose moiety is substituted to produce a 2'-substituted or 2'-O-substituted ribonucleoside. Preferably, such substitution is with a lower alkyl group containing 1-6 saturated or unsaturated carbon atoms, or with an aryl group having 6-10 carbon atoms, wherein such alkyl, or aryl group may be unsubstituted or may be substituted, e.g., with halo, hydroxy, trifluoromethyl, cyano, nitro, acyl, acyloxy, alkoxy, carboxyl, carboalkoxy, or amino groups. Examples of 2'-O-substituted ribonucleosides or 2'-O-substituted-arabinosides include, without limitation 2'-O-methylribonucleosides or 2'-O-methylarabinosides and 2'-O-methoxyethylribonucleosides or 2'-O-methoxyethylarabinosides.

The term "2'-substituted ribonucleoside" or "2'-substituted arabinoside" also includes ribonucleosides or arabinonucleosides in which the 2'-hydroxyl group is replaced with a lower alkyl group containing 1-6 saturated or unsaturated carbon atoms, or with an amino or halo group. Examples of such 2'-substituted ribonucleosides or 2'-substituted arabinosides include, without limitation, 2'-amino, 2'-fluoro, 2'-allyl, and 2'-propargyl ribonucleosides or arabinosides.

The term "oligonucleotide" includes hybrid and chimeric oligonucleotides. A "chimeric oligonucleotide" is an oligonucleotide having more than one type of internucleoside linkage. One preferred example of such a chimeric oligonucleotide is a chimeric oligonucleotide comprising a phosphorothioate, phosphodiester or phosphorodithioate region and non-ionic linkages such as alkylphosphonate or alkylphosphonothioate linkages (see e.g., Pederson et al. U.S. Pat. Nos. 5,635,377 and 5,366,878).

A "hybrid oligonucleotide" is an oligonucleotide having more than one type of nucleoside. One preferred example of such a hybrid oligonucleotide comprises a ribonucleotide or 2'-substituted ribonucleotide region, and a deoxyribonucleotide region (see, e.g., Metelev and Agrawal, U.S. Pat. Nos. 5,652,355, 6,346,614 and 6,143,881).

RNA oligonucleotides discussed herein include otherwise unmodified RNA as well as RNA which have been modified (e.g., to improve efficacy), and polymers of nucleoside surrogates. Unmodified RNA refers to a molecule in which the components of the nucleic acid, namely sugars, bases, and phosphate moieties, are the same or essentially the same as that which occur in nature, preferably as occur naturally in the human body. The art has referred to rare or unusual, but naturally occurring, RNAs as modified RNAs, see, *e.g.,* Limbach et al. 1994, Nucleic Acids Res 22: 2183-2196. Such rare or unusual RNAs, often termed modified RNAs (apparently because these are typically the result of a post-transcriptional modification) are within the term unmodified RNA, as used herein. Modified RNA as used herein refers to a molecule in which one or more of the components of the nucleic acid, namely sugars, bases, and phosphate moieties, are different from that which occurs in nature, preferably different from that which occurs in the human body. While they are referred to as modified "RNAs," they will of course, because of the modification, include molecules which are not RNAs. Nucleoside surrogates are molecules in which the ribophosphate backbone is replaced with a non-ribophosphate construct that allows the bases to the presented in the correct spatial relationship such that hybridization is substantially similar to what is seen with a ribophosphate backbone, e.g., non-charged mimics of the ribophosphate backbone.

All nucleic acid sequences listed herein are in the 5' to 3' direction unless otherwise indicated.

The RNA oligonucleotide of the invention can be single-stranded, double stranded, or partially double-stranded.

A single-stranded RNA oligonucleotide may contain self-complementary sequences and forms a hairpin. For example, 5'-GACCTAGCCTAAAACTAGGTC-3'. The self-complementary sequence may be a palindromic sequence. For example, 5'AAAGATCCGGATCAAAA-3'.

A double stranded RNA oligonucleotide may have one- or two-nucleotide overhang at the 5' or 3' end of one or both strands.

A partially double-stranded RNA oligonucleotide may comprise two strands of the same or different length, wherein the at least one of the strands contains nucleotides outside the complementary sequence. For example,

### Example 1: 5'-AAAAGUUCAAAGCUCAAAA-3' 3'-CAAGUUUCGAG-5'

### Example 2: 5'-UCAAAGUCAAAAGCUCAAAGUUGAAAGUUUAAA-3' 3'-GACUUGAAAAUUUCAGUUUUCGAGUUUAAGUUGAAAACUCG-5'

### Example 3: 5'-UCAAAGUCAAAAGCUCAAAGUUGAAA-3' 3'- UUUCAGUUUUCGAGUUUAAGUUGAAAACUCG-5'

The length of a single-stranded RNA oligonucleotide is the number of nucleotides contained in the oligonucleotide.

In the case of a double-stranded or partially double-stranded oligonucleotide, the length of the oligonucleotide is the length of the individual strands. In other words, a partially double-stranded oligonucleotide can have two lengths.

### Enhanced Nuclease Resistance

For increased nuclease resistance and/or binding affinity to the target, an oligonucleotide can include, for example, 2'-modified ribose units and/or phosphorothioate linkages. For example, the 2' hydroxyl group (OH) can be modified or replaced with a number of different "oxy" or "deoxy" substituents.

Examples of "oxy"-2' hydroxyl group modifications include alkoxy or aryloxy (OR, e.g., R = H, alkyl, cycloalkyl, aryl, aralkyl, heteroaryl or sugar); polyethyleneglycols (PEG), O(CH₂CH₂O)ₙCH₂CH₂OR; "locked" nucleic acids (LNA) in which the 2' hydroxyl is connected, *e.g.,* by a methylene bridge, to the 4' carbon of the same ribose sugar; O-AMINE and aminoalkoxy, O(CH₂)ₙAMINE, (*e.g.*, AMINE = NH₂; alkylamino, dialkylamino, heterocyclyl amino, arylamino, diaryl amino, heteroaryl amino, or diheteroaryl amino, ethylene diamine, polyamino). It is noteworthy that oligonucleotides containing only the methoxyethyl group (MOE), (OCH₂CH₂OCH₃, a PEG derivative), exhibit nuclease stabilities comparable to those modified with the robust phosphorothioate modification.

"Deoxy" modifications include hydrogen (i.e. deoxyribose sugars, which are of particular relevance to the overhang portions of partially ds RNA); halo (e.g., fluoro); amino (*e.g.* NH₂; alkylamino, dialkylamino, heterocyclyl, arylamino, diaryl amino, heteroaryl amino, diheteroaryl amino, or amino acid); NH(CH₂CH₂NH)ₙCH₂CH₂-AMINE (AMINE = NH₂; alkylamino, dialkylamino, heterocyclyl amino, arylamino, diaryl amino, heteroaryl amino,or diheteroaryl amino), -NHC(O)R (R = alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with *e.g.,* an amino functionality.

Preferred substitutents are 2'-methoxyethyl, 2'-OCH3, 2'-O-allyl, 2'-C- allyl, and 2'-fluoro. To maximize nuclease resistance, the 2' modifications can be used in combination with one or more phosphate linker modifications (e.g., phosphorothioate). The so-called "chimeric" oligonucleotides are those that contain two or more different modifications. The inclusion of furanose sugars in the oligonucleotide backbone can also decrease endonucleolytic cleavage. An oligonucleotide agent can be further modified by including a 3' cationic group, or by inverting the nucleoside at the 3'-terminus with a 3'-3' linkage. In another alternative, the 3'-terminus can be blocked with an aminoalkyl group, e.g., a 3' C5-aminoalkyl dT. Other 3' conjugates can inhibit 3'-5' exonucleolytic cleavage. While not being bound by theory, a 3' conjugate, such as naproxen or ibuprofen, may inhibit exonucleolytic cleavage by sterically blocking the exonuclease from binding to the 3'-end of oligonucleotide. Even small alkyl chains, aryl groups, or heterocyclic conjugates or modified sugars (D-ribose, deoxyribose, glucose etc.) can block 3'-5'-exonucleases.

Similarly, 5' conjugates can inhibit 5'-3' exonucleolytic cleavage. While not being bound by theory, a 5' conjugate, such as naproxen or ibuprofen, may inhibit exonucleolytic cleavage by sterically blocking the exonuclease from binding to the 5'-end of oligonucleotide. Even small alkyl chains, aryl groups, or heterocyclic conjugates or modified sugars (D-ribose, deoxyribose, glucose etc.) can block 3'-5'-exonucleases.

Single-stranded RNA oligonucleotides which contain self-complementary sequences and form a hairpin structure have enhanced nuclease resistance compared to single-stranded oligonucleotides which do not.

### 5'-Phosphate Modifications

The oligonucleotides of the present invention can be 5' phosphorylated or can include a phosphoryl analog at the 5' prime terminus. 5'-phosphate modifications of the antisense strand include those which are compatible with RISC mediated gene silencing. Suitable modifications include: 5'-monophosphate ((HO)2(O)P-O-5'); 5'-diphosphate ((HO)2(O)P-O-P(HO)(O)-O-5'); 5'-triphosphate ((HO)2(O)P-O-(HO)(O)P-O-P(HO)(O)-O-5'); 5'-guanosine cap (7-methylated or non-methylated) (7m-G-O-5'-(HO)(O)P-O-(HO)(O)P-OP(HO)(0)-0-5'); 5'-adenosine cap (Appp), and any modified or unmodified nucleotide cap structure. Other suitable 5'-phosphate modifications will be known to the skilled person.

### Tethered Ligands

The RNA oligonucleotides of the present invention also include those with tethered ligands. The properties of a RNA oligonucleotide, including its pharmacological properties, can be influenced and tailored by the introduction of ligands, e.g. tethered ligands.

The ligands may be coupled, preferably covalently, either directly or indirectly via an intervening tether, to the RNA oligonucleotide. In preferred embodiments, the ligand is attached to the oligonucleotide *via* an intervening tether.

In preferred embodiments, a ligand alters the distribution, targeting or lifetime of a RNA oligonucleotide into which it is incorporated. In preferred embodiments a ligand provides an enhanced affinity for a selected target, e.g., molecule, cell or cell type, a cellular or organ compartment, tissue, organ or region of the body.

Preferred ligands can improve transport, hybridization, and specificity properties and may also improve nuclease resistance of the resultant natural or modified oligoribonucleotide, or a polymeric molecule comprising any combination of monomers described herein and/or natural or modified ribonucleotides.

A wide variety of ligands may be used. Ligands may include agents that allow for the specific targeting of the oligonucleotide; diagnostic compounds or reporter groups which allow for the monitoring of oligonucletotide distribution; cross-linking agents; nuclease-resistance conferring moieties; and natural or unusual nucleobases. General examples include lipophilic moleculeses, lipids, lectins, steroids (e.g.,uvaol, hecigenin, diosgenin), terpenes (e.g., triterpenes, e.g., sarsasapogenin, Friedelin, epifriedelanol derivatized lithocholic acid), vitamins, carbohydrates(e.g., a dextran, pullulan, chitin, chitosan, inulin, cyclodextrin or hyaluronic acid), proteins, protein binding agents, integrin targeting molecules,polycationics, peptides, polyamines, and peptide mimics.

The ligand may be a naturally occurring or recombinant or synthetic molecule, such as a synthetic polymer, e.g., a synthetic polyamino acid. Examples of polyamino acids include polyamino acid is a polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacrylic acid), N-isopropylacrylamide polymers, or polyphosphazine. Example of polyamines include: polyethylenimine, polylysine (PLL), spermine, spermidine, polyamine, pseudopeptide-polyamine, peptidomimetic polyamine, dendrimer polyamine, arginine, amidine, protamine, cationic moieties, e.g., cationic lipid, cationic porphyrin, quaternary salt of a polyamine, or an alpha helical peptide.

Ligands can also include targeting groups, e.g., a cell or tissue targeting agent, e.g., , a thyrotropin, melanotropin, surfactant protein A, Mucin carbohydrate, a glycosylated polyaminoacid, transferrin, bisphosphonate, polyglutamate, polyaspartate, or an RGD peptide or RGD peptide mimetic.

Ligands can be proteins, e.g., glycoproteins, lipoproteins, e.g. low density lipoprotein (LDL), or albumins, e.g. human serum albumin (HSA), or peptides, e.g., molecules having a specific affinity for a co-ligand, or antibodies e.g., an antibody, that binds to a specified cell type such as a cancer cell, endothelial cell, or bone cell. Ligands may also include hormones and hormone receptors. They can also include non-peptidic species, such as cofactors, multivalent lactose, multivalent galactose, N-acetyl-galactosamine, N-acetyl-glucosamine, multivalent mannose, or multivalent fucose. The ligand can be, for example, a lipopolysaccharide, an activator of p38 MAP kinase, or an activator of NF-κB. The ligand can be a substance, e.g., a drug, which can increase the uptake of the oligonucleotide agent into the cell, for example, by disrupting the cell's cytoskeleton, e.g., by disrupting the cell's microtubules, microfilaments, and/or intermediate filaments. The drug can be, for example, taxon, vincristine, vinblastine, cytochalasin, nocodazole, japlakinolide, latrunculin A, phalloidin, swinholide A, indanocine, or myoservin.

In one embodiment, the ligand is a lipid or lipid-based molecule. Such a lipid or lipid-based molecule preferably binds a serum protein, e.g., human serum albumin (HSA). An HSA binding ligand allows for distribution of the conjugate to a target tissue, e.g., liver tissue, including parenchymal cells of the liver. Other molecules that can bind HSA can also be used as ligands. For example, neproxin or aspirin can be used. A lipid or lipid-based ligand can (a) increase resistance to degradation of the conjugate, (b) increase targeting or transport into a target cell or cell membrane, and/or (c) can be used to adjust binding to a serum protein, e.g., HSA.

A lipid based ligand can be used to modulate, e.g., control the binding of the conjugate to a target tissue. For example, a lipid or lipid-based ligand that binds to HSA more strongly will be less likely to be targeted to the kidney and therefore less likely to be cleared from the body. A lipid or lipid-based ligand that binds to HSA less strongly can be used to target the conjugate to the kidney.

In another embodiment, the ligand is a moiety, e.g., a vitamin or nutrient, which is taken up by a target cell, e.g., a proliferating cell. These are particularly useful for treating disorders characterized by unwanted cell proliferation, e.g., of the malignant or non-malignant type, e.g., cancer cells. Exemplary vitamins include vitamin A, E, and K. Other exemplary vitamins include the B vitamins, e.g., folic acid, B12, riboflavin, biotin, pyridoxal or other vitamins or nutrients taken up by cancer cells.

In another embodiment, the ligand is a cell-permeation agent, preferably a helical cell-permeation agent. Preferably, the agent is amphipathic. An exemplary agent is a peptide such as tat or antennapedia. If the agent is a peptide, it can be modified, including a peptidylmimetic, invertomers, non-peptide or pseudo-peptide linkages, and use of D-amino acids. The helical agent is preferably an alpha-helical agent, which preferably has a lipophilic and a lipophobic phase.

In a preferred embodiment, the ligand is an antibody or a fragment thereof which is specific for a moiety present in a cell to be targeted. The moiety may be a protein, a carbohydrate structure, a polynucleotide, or a combination thereof. The moiety may be secreted, associated with the plasma membrane (e.g., on the extracellular or intracellular surface), cytosolic, associated with intracellular organelles (e.g., ER, Golgi complex, mitochondria, endosome, lysosome, secretory vesicle) or nuclear. The antibody may be monoclonal or polyclonal. The antibody may be chemeric or humanized. The antibody may be a single chain antibody. The antibody fragment may be a Fab fragment, a F(ab')₂ fragment, or any fragments that retain the antigen-binding specificity of the intact antibody.

### immunostimulatory activity

As used herein, "immunostimulatory activity" refers to the capability of a molecule or a composition to induce an immune response. In one aspect, the immunostimulatory activity refers to the type I-IFN-inducing activity, in particular, the IFN-α-inducing activity.

As used herein, "inducing an immune response" means initiating or causing an increase in one or more of B-cell activation, T-cell activation, natural killer cell activation, activation of antigen presenting cells (e.g., B cells, dendritic cells, monocytes and macrophages), cytokine production, chemokine production, specific cell surface marker expression, in particular, expression of co-stimulatory molecules. In one aspect, such an immune response involves the production of type I IFN, in particular, IFN-α, in cells such as PDC.

As used herein, "IFN-α-inducing activity" refers to the capability of a molecule or composition to induce IFN-α production from a cell capable of producing IFN-α. Cells capable of producing IFN-α include, but are not limited to, peripheral blood mononuclear cells (PBMC) (e.g., B cells, dendritic cells (myeloid dendritic cells and plasmacytoid dendritic cells), macrophages, monocytes, natural killer cells, granulocytes), endothelial cells, and cell lines (e.g., THP1; cells transfected with expression vectors for TLR-7 and/or TLR-8 such as CHO cells, COS cells, HEK293 cells). Cells capable of producing IFN-α include those that express TLR7, TLR8, or both TLR7 and TLR8.

### gene silencing activity

As used herein, "gene silencing" refers to the downregulation or the abolition of the expression of a target gene. Gene silencing as used herein, occurs at the post-transcriptional level. Gene silencing may be directly or indirectly mediated by siRNA, shRNA and antisense RNA.

Both the antisense-strand of the siRNA and the antisense RNA have complementary to the target mRNA and are the effector strand of the gene silencing activity. The term complementary is well understood by those skilled in the art. For example, A is complementary to T, G is complementary to C, 5'-AG-3' is complementary to 5'-CT-3'.

The degree of complementarity between two oligonucleotides is the percentage of complementary bases in the overlapping region of the two oligonucleotides. The degree of complementarily can be determined manually or automatically by various engines such as BLAST. For example, ATCG has 100% complementarity to CGAT and CGATGG, and 75% complementarity to CGTT and CGTTGG. Furthermore, the degree of complementarity between a RNA oligonucleotide and any sequences present in the public databases (e.g., EMBL, GeneBank) can be determined by the BLAST program.

The degree of complementarity between the antisense strand of the siRNA or the antisense RNA and the target mRNA is at least 80% 81%, 82%, 83%, preferably at least 84%, 85%, 86%, 87%, 88%, more preferably at least 89%, 90%, 91%, 92%, 93%, even more preferably at least 94%, 95%, 96%, 97%, 98%, 99%, and most preferably 100%.

The gene silencing activity of a RNA oligonucleotide can be determined experimentally by methods well known in the art. For Example, the RNA oligonucleotide may be introduced into a cell by a method known in the art such as transfection and transduction; the mRNA level of the target gene can be determined by routine methods such as Northern blot analysis, quantitative PCR, RNase protection assay, and branching DNA; and the protein expression level can be determined by routine methods such as Western blotting, ELISA, and biological activity assays specific to the target protein. Furthermore, the mRNA level of all known and hypothetical genes can be determined at the global level using the microarray technology. Technologies in the field of proteonomics allow for the protein levels of a large number of genes to be determined at the global level as well.
Naked RNA oligonceotide may be transfected into a cell via electroporation. RNA oligonucleotide may be complexed with a complexation agent which facilitates the uptake of the oligonucletide into a cell. Such complexation agents include, but are not limited to cationic lipids (e.g., Lipofectamine, Oligofectamine, DOTAP), cationic peptides, and calcium phosphate.

The gene silencing activity of a RNA oligonucleotide can be predicted by algorithms such as the one disclosed in Reynolds et al. 2004, Nat Biotechnol 22:326-330.

### siRNA

As used herein, "siRNA" stands for short interfering RNA, and has the same definition as that established in the art. siRNA is double-stranded and is usually between 19 and 27 nucleotide in length. In vivo, siRNA is the product of Dicer activity on long dsRNA. The antisense strand of siRNA is complementary to the target mRNA; it binds the target mRNA and induces RISC-mediated target mRNA degradation. siRNA can be chemically synthesized, produced in vitro by Dicer-mediated enzymatic degradation of long dsRNA, produced by in vitro transcription from linear (e.g. PCR products) or circular templates (e.g., viral or non-viral vectors), or produced by in vivo transcription from viral or non-viral vectors. Commercially available synthetic siRNA usually contain a core of 19 complemetary base pairs and a 2-nucleotide (UU or TT) 3' overhang on each strand.

siRNA may be chemically modified to have enhanced stability in vitro (especially in serum-containing media) and in vivo. siRNA may also be chemically modified to have enhanced uptake by cells in vitro and in vivo. Furthermore, siRNA may be linked to tethered ligands to have enhanced target specificity and improved pharmacological properties (such as half-life, clearance, distribution).

### shRNA

As used herein, "shRNA" stands for short hairpin RNA and has the same definition as that established in the art. shRNA is processed inside a cell into siRNA which mediates RNAi as described previously. The loop sequence in shRNA is not thought to be involved in RNAi, and it can be of various lengths and sequences. The preferred lengths and sequences of the loop are known to those skilled in the art.

Similar to siRNA, shRNA can be chemically synthesized, produced by in vitro transcription from linear (e.g. PCR products) or circular templates (e.g., viral or non-viral vectors), or produced by in vivo transcription from viral or non-viral vectors.

### antisense RNA

As used herein, "antisense RNA" has the same definition as that established in the art. Antisense RNA is complementary to target mRNA and it thought to interfere with the translation of the target mRNA. Antisense RNA molecules are usually 18-50 nucleotides in length. Antisense RNA may be modified to have enhanced stability, nuclease resistance, target specificity and improved pharmacological properties.

Similar to siRNA and shRNA, antisense RNA can be chemically synthesized, produced by in vitro transcription from linear (e.g. PCR products) or circular templates (e.g., viral or non-viral vectors), or produced by in vivo transcription from viral or non-viral vectors.

### disorder/disease-related gene and antigen

As used herein, "disorder/disease-related gene" refers to a gene that is expressed or overexpressed in a disease/disorder and that is not expressed or expressed in reduced amount under normal condition. For example, a mutant CF gene is expressed in cystic fibrosis patient but not in an individual without cystic fibrosis; ErbB2 (or Her2) is overexpressed in breast cancer cells compared to normal breast cells; a viral gene is expressed in infected cells but not in uninfected cells. The gene product of the disorder/disease-related gene is referred to herein as the "disorder/disease-related antigen".

### mammal

As used herein, the term "mammal" includes, without limitation, rats, mice, cats, dogs, horses, sheep, cattle, cows, pigs, rabbits, non-human primates, and humans.

### Technology Platform

The vertebrate immune system established different ways to detect invading pathogens based on certain characteristics of their microbial nucleic acids. Detection of microbial nucleic acids alerts the immune system leading to the appropriate type of immune responses that is required for the defence against the respective type of pathogen detected. Detection of viral nucleic acids leads to the production of type I IFN, the key cytokine for anti-viral defence. While it is well established that the recognition of microbial DNA is sequence-specific, involving the so-called CpG motifs, the optimal motif for the recognition of microbial RNA has not been defined yet. The present application provides a technology platform for identifying the optimal motif for the recognition of microbial RNA and the induction of type I IFN.

The technology platform of the present invention comprises three key features: i) the transfection of peripheral blood mononuclear cells (PBMC) from healthy donors with RNA oligonucleotides; ii) the generation of a RNA oligonucleotide library containing 4mer motifs on a poly adenosine (polyA) backbone; iii) the development of algorithms based on the experimental data generated for the RNA oligonucleotide library to predict the immunostimulatory activity of any given RNA oligonucleotide.

The first key feature of the technology platform is the method of introducing RNA oligonucleotides into PBMC. Naked RNA oligonucleotides are not taken up by the cells to any significant degree. RNA oligonucleotides normally need to form complexes with complexation agent (or transfection agent) in order to be introduced into cells. In the literature, cationic lipids such as lipofectamine or DOTAP are routinely used as complexation agent for the transfection of RNA oligonucleotides. However, RNA-cationic lipid complexes lead to rapid cell death of myeloid cells. Although myeloid cells are not the cellular source of IFN-α within PBMC (the source is PDC), death of myeloid cells in the cell culture negatively affects the reproducibility of IFN-α induction in PBMC. Therefore, the use of cationic lipids is limited to isolated PDC. However, isolated PDC are not suitable for large scale screening assays because PDC make up 0.2-0.6% of the PBMC in a normal individual; it is difficult to obtain enough cells for the assays.

To identify a complexation agent that is suitable for use with PBMC, we compared different types of cationic peptides, poly-His, poly-L-Lys, and poly-L-Arg. Poly-L-Arg was found to provide the most potent support for the immunostimulatory activity of RNA oligonucleotides when compared to other cationic peptides and cationic lipids (Figure 1). A protocol was then established that allows well-controlled and highly reproducible complex formation between the RNA oligonucleotide and the complexation agent and subsequent RNA transfection into cells. Complex formation could be controlled by salt concentration, phosphate content and incubation time. Complex formation was monitored by the size of complexes and the functional activity over a range of concentrations. The use of poly-L-Arg did not affect the viability of myeloid cells and thus could be applied to PBMC without restrictions.

The second key issue of the technology platform was the generation of the RNA oligonucleotide library. An earlier study showed that a minimal length of 19 bases was required for the optimal immunostimulatory activity of an RNA oligonucleotide; furthermore, it showed that poly adenosine (poly A) was completely inactive (Hornung V et al. 2005, Nat Med 11:263-270). Therefore, the motif search was performed with a 19mer oligonucleotide on a poly A sequence background. By adding increasing numbers of uridine (U) in the center of such a poly A oligonucleotide, we found that a 4-nucleotide (4mer) motif in the center was sufficient to confer marked immunostimulatory activity (Figure 2). Importantly, after identifying the optimal 4mer sequence motifs for inducing IFN-α production, we found that changing the bases flanking the 4mer motifs did not further enhance the immunostimulatroy activity of the 4mer motifs (Figure 7B). The library of 193 RNA oligonucleotides used covered all 256 possible 4mer motifs. The reduction from 258 to 193 was possible because of redundant motifs caused by the poly A flanking regions. In additional studies we found that the exact location of the 4mer motif within the poly A backbone is not critical for the immunostimulatory activity (Figure 7A).

The third key feature of the technology platform was the generation of a data matrix and its mathematical analysis. Algorithms were developed that allowed an excellent prediction of the immunostimulatory activity of RNA oligonucleotides. The frequency of a given 4mer motif at a certain position within an oligonucleotide is only 1:256. Even though the most active 4mer motifs can be used as the core for constructing potent immunostimulatory RNA oligonucleotides, the IFN-α indices of the 4mer motifs are not particularly useful for predicting the activity of a given RNA oligonucleotide, or for designing RNA oligonucleotides with minimal immunostimulatory activity which is desired for an siRNA. Therefore, algorithms were established which based on parts of the 4mer motifs, namely 1, 2 or 3 bases either in a row (XXX) or with spacing (X*XX; XX*X). The highest predictive value was obtained with the algorithm using 3 bases (i.e., 3mer motifs). This 3mer-based algorithm allowed an impressively accurate prediction (correlation coefficient (r)=0.87) of the immunostimulatory activity of the 19mer RNA oligonucleoties carrying 4mer motifs in our library (Figure 6E) and RNA oligonucleotides previously published in the literature by us and others (Figure 8A-C).

There are a number of applications for the information generated by our technology platform: a) the 4mer motif data matrix can be used to design oligonucleotides with optimal IFN-α-inducing activity; b) 4mer motifs with minimal IFN-α-inducing activity can be used as the repertoire for selecting potential inhibitory sequence motifs; c) the 3mer-based algorithm (e.g., the IFN-α point score matrix) can be used to predict the immunostimulatory activity of a given RNA oligonucleotide; d) the 3mer-based algorithm (e.g., the IFN-α point score matrix) can be used to design RNA oligonucleotides with maximal immunostimulatory activity and additional sequence requirements for other functionalities such as gene silencing in the case of an siRNA (in this case the use of 4mer motif matrix is not useful since 4mer motifs are not frequent enough); e) the 3mer-based algorithm (e.g., the IFN-α point score matrix) can also be used to design RNA nucleotides with minimal immunostimulatory activity and additional sequence requirements for other functionalities such as gene silencing in the case of an siRNA (in this case the use of 4mer motif matrix is not useful since 4mer motifs are not frequent enough).

In the case of an immunostimulatory RNA, an oligonucleotide containing only one of the most potent 4mer motifs is 80% more active than the most active complex oligonucleotide containing a 9mer motif in the literature (Table 1). In a 19mer oligonucleotide, there is room for several potent 4mer motifs. A 19mer RNA olignucleotide containing more than one potent immunostimulatory 4mer motifs is expected to have even higher activity.

Furthermore, inhibitory motifs may exist that inhibit the immunostimulatory activity of a RNA oligonucleotide as in the case of CpG oligonucleotides. Such inhibitory motifs, by definition, are among the motifs with weak IFN-α-inducing activity. In the field of RNA interference, type I IFN induction usually is unwanted. The 3mer-based algorithm (e.g., the IFN-α point score matrix) described above can be used to select siRNA sequences with minimal immunostimulatory activity. A sequence analysis of cyclophylin B mRNA, one of the best studied targets for siRNA, identifies a number of siRNA sequences for which our algorithm (e.g., the IFN-α point score matrix) predicts minimal type I IFN induction and which still are known to be potent in gene silencing (Reynolds A et al. 2004, Nat Biotechnol 22: 326-330). This confirms our previous finding that RNA interference and IFN-α induction are two independent functional activities of a siRNA molecule.

Of note, the motif search performed in the present study focuses on the activity of RNA oligonucleotides to induce IFN-α. From previous studies, it is known that the cellular source of IFN-α within the PBMC is PDC. By analysing the level of IFN-α induction in PBMC, other activities of the RNA oligonucleotides on other cellular subsets of the PBMC, such as myeloid cells, are not addressed. Myeloid cells express TLR8 in addition to TLR7 and thus may show different nucleotide sequence specificities and may be induced to exhibit additional activities than IFN-α production. It therefore needs to be born in mind that ssRNA oligonucleotides are capable of inducing both PDC-dependent (i.e. IFN-α production) and PDC-independent activities (e.g., activation of myeloid cells). In contrast, we found that dsRNA oligonucleotides, such as siRNA, are only recognized by PDC but not myeloid cells. As a result, it is valid to predict the immunological activity of siRNA oligonculeotides based on their ability to induce IFN-α production.

### Method for Determining the Immunostimulatory Activity of an RNA Oligonucleotide

The present invention provides a method for determining the immunostimulatory activity, in particular, the IFN-α-inducing activity, of a RNA oligonucleotide, comprising the steps of:
(a) complexing the RNA oligonucleotide with a complexation agent;
(b) contacting a cell with the complexed RNA oligonucleotide, wherein the cell expresses TLR7 or TLR8 or both TLR7 and TLR8; and
(c) determining the amount of IFN-α produced by the cell of step (b), an increase of IFN-α production indicating immunostimulatory activity of the RNA oligonucleotide.

In one embodiment of the invention, the complexation agent is a polycationic peptide, preferably poly-L-arginine (poly-L-Arg). In one embodiment, the polycationic peptide, in particular, poly-L-Arg, is at least 24 amino acids in length. The polycationic peptide, in particular, poly-L Arg, may be a heterogeneous mixture of peptides of different lengths.

The cells expressing TLR7 or TLR8 or both TLR7 or TLR8 include, but are not limited to, peripheral blood mononuclear cells (PBMC), plasmacytoid dendritric cells (PDC), myeloid dendritic cells (MDC), B cells, macrophages, monocytes, natural killer cells, granulocytes, endothelial cells, cell lines such as THP1, and cells containing exogenous DNA which directs the expression of TLR7 or TLR8 or both TLR7 or TLR8 such as transfected CHO, HEK293, and COS cells.

In one embodiment of the invention, the cell is a mammalian cell, preferably a human cell or a cell of human origin.

The RNA oligonucleotide can be single-stranded, double-stranded or partially double-stranded.

### Method for Predicting the Immunostimulatory Activity of a RNA Oligonucleotide

The present invention provides a method for predicting the immunostimulatory activity, in particular the IFN-α-inducing activity, of a RNA oligonucleotide, comprising the steps of:
(a) identifying all possible 3-nucleotide (3mer) motifs contained in the oligonucleotide;
(b) assigning an IFN-α point score for each individual 3mer motif;
(c) calculating the IFN-α score of the oligonucleotide by calculating the sum of the IFN-α point scores of individual 3mer motifs; and
(d) assigning to the oligonucleotide a high immunostimulatory activity if the IFN-α score is at least 23, an intermediate immunostimulatory activity if the IFN-α score is between -4 and 23, and a low immunostimulatory activity if the IFN-α score is at most -4, when n=6;
   assigning to the oligonucleotide a high immunostimulatory activity if the IFN-α score is at least 26, an intermediate immunostimulatory activity if the IFN-α score is between -4 and 26, and a low immunostimulatory activity if the IFN-α score is at most -4, when n=7;
   assigning to the oligonucleotide a high immunostimulatory activity if the IFN-α score is at least 28, an intermediate immunostimulatory activity if the IFN-α score is between -5 and 23, and a low immunostimulatory activity if the IFN-α score is at most -5, when n=8;
   assigning to the oligonucleotide a high immunostimulatory activity if the IFN-α score is at least 30, an intermediate immunostimulatory activity if the IFN-α score is between -5 and 30, and a low immunostimulatory activity if the IFN-α score is at most -9, when n=9;
   assigning to the oligonucleotide a high immunostimulatory activity if the IFN-α score is at least 1.4909 x n + 22.014, an intermediate immunostimulatory activity if the IFN-α score is between 0.005 x n² - 0.2671 x n - 3.5531 and 1.4909 x n + 22.014, and a low immunostimulatory activity if the IFN-α score is at most 0.005 x n² - 0.2671 x n - 3.5531, when n is greater than 9,
wherein n is the length of the oligonucleotide.

A single-stranded RNA oligonucleotide of the length n (n≥6) is broken up into all possible 3mer motifs starting a the 5' end. This will result in a total number of n-2 possible 3mer motifs. For example the 20mer ssRNA oligonucleotide 5'-CAGAGCGGGAUGCGUUGGUC -3' can be broken up into the following 18 3mer motifs (5' -->3'): CAG, AGA, GAG, AGC, GCG, CGG, GGG, GGA, GAU, AUG, UGC, GCG, CGU, GUU, UUG, UGG, GGU, GUC.

Subsequently, all of the 3mer motifs are checked against the IFN-α point score matrix (Table 7).

**Table 7: IFN-α point score matrix**

| 3mer motif (5'→3') | IFN-α point score |
|---|---|
| ACA | -2 |
| ACC | -2 |
| AGA | -2 |
| AAC | -1 |
| AUA | -1 |
| UGG | +1 |
| GUA | +3 |
| GUG | +3 |
| GGU | +4 |
| UCA | +4 |
| UUC | +4 |
| UUU | +5 |
| AGU | +6 |
| UUG | +6 |
| GUC | +8 |
| UGU | +8 |
| GUU | +9 |

Whenever a 3mer motif is present in the IFN-α point score matrix, the listed point score is added to the so-called predicted IFN-α score of the oligonucleotide analyzed. A 3mer motif which is absent from the IFN-α point score matrix has a point score of 0. Thus the predicted IFN-α score of an oligonucelotide is the sum of IFN-α scores of all 3mer motifs that are present in the IFN-α point score matrix.

For example, for the 20mer ssRNA oligonucleotide 5'- CAGAGCGGGAUGCGUUGGUC -3', a predicted IFN-α score can be calculated as follows:

| 3mer motifs in the 20mer ssRNA | Score in the IFN-α point score matrix | predicted IFN-α score |
|---|---|---|
| CAG | 0 | 0 |
| AGA | (-2) | -2 |
| GAG | 0 | 0 |
| AGC | 0 | 0 |
| GCG | 0 | 0 |
| CGG | 0 | 0 |
| GGG | 0 | 0 |
| GGA | 0 | 0 |
| GAU | 0 | 0 |
| AUG | 0 | 0 |
| UGC | 0 | 0 |
| GCG | 0 | 0 |
| CGU | 0 | 0 |
| GUU | (+9) | +9 |
| UUG | (+6) | +6 |
| UGG | (+1) | +1 |
| GGU | (+4) | +4 |
| GUC | (+8) | +8 |
| Overall | | +26 |

The IFN-α score of a double-stranded RNA oligonucleotide is the higher of the two IFN-α scores for the two strands.

In the case of a double-stranded or partially double-stranded RNA oligonucleotide, the oligonucleotide is assigned high immunostimulatory activity if at least one of the strands meets the threshold for having high immunostimulatory activity as defined above; the oligonucleotide is assigned low immunostimulatory activity if both strands meet the threshold for having low immunostimulatory activity as defined above. The rest RNA oligonucleotides are assigned intermediate immunostimulatory activity.

### Method for Designing and Preparing RNA Oligonucleotides

The present application provides a method for preparing a RNA oligonucleotide having immunostimulatory activity, in particular, high IFN-α-inducing activity, comprising the steps of:
(a) providing candidate oligonucleotide sequence(s);
(b) identifying oligonucleotide sequence(s) with high immunostimulatory activity predicted according to the method of prediction described in the previous section;
(c) preparing the RNA oligonucleotide(s) identified for high immunostimulatory activity in step (b); and
(d) optionally testing the immunostimulatory activity of the RNA oligonucleotide(s) prepared in step (c) according to the method of determination described previously; and
(e) further optionally modifying the oligonucleotide(s) to optimize the immunostimulatory activity.

The present application also provides a method for preparing a RNA oligonucleotide having low immunostimulatory activity, in particular, low IFN-α-inducing activity, comprising the steps of:
(a) providing candidate oligonucleotide sequence(s);
(b) identifying oligonucleotide sequence(s) with low immunostimulatory activity predicted according to the method of prediction described in the previous section;
(c) preparing the RNA oligonucleotide(s) identified for low immunostimulatory activity in step (b); and
(d) optionally testing the RNA oligonucleotide(s) prepared in step (c) for the lack of immunostimulatory activity according to the method of determination described previously; and
(e) further optionally modifying the oligonucleotide(s) to minimize the immunostimulatory activity.

The RNA oligonucleotide can be single-stranded, double-stranded or partially double-stranded.

The RNA oligonucleotide can have other functionalities such as the gene silencing activity.

The methods provided by the present application can be used to prepare immunostimulatory RNA oligonucleotides, siRNA, shRNA or antisense RNA with high or low immunostimulatory activity.

Some of the RNA oligonucleotides which have low immunostimulatory activity, i.e., the non-immunostimulatory oligonucleotides, may in fact have inhibitory activity against immune activation. Such an immunoinhibitory oligonucleotide may be able to prevent immune activation induced by an immunostimulatory oligonucleotide when used in combination.

RNA oligonucleotides can be prepared by methods including, but are not limited to, chemical synthesis, in vitro and in vivo transcription from linear templates (e.g., PCR product) and circular templates (e.g., viral or non-viral vectors).

### Method for Preparing siRNA Having High or Low Immunostimulatory Activity

The present invention provides a method for preparing an siRNA having gene silencing activity for a target gene and having immunostimulatory activity, in particular, IFN-α-inducing activity, comprising the steps of:
(a) identifying all potential siRNA antisense sequences for a target mRNA;
(b) identifying antisense sequences that have gene silencing activity;
(c) predicting the immunostimulatory activity for the antisense sequences identified in step (b) and their complementary (i.e., sense) sequences;
(d) identifying siRNA wherein at least one of the sense and antisense sequences has an IFN-α score of at least 1.4909 x n + 31,014, wherein n is the length of the sequence and n is between 19 and 25;
(e) decreasing the IFN-α score threshold by 1 if no siRNA is identified in step (d), until at least one siRNA is identified;
(f) preparing the siRNA identified in step (d) or (e);
(g) optionally testing the gene silencing and/or the immunostimulatory activity of the siRNA prepared in (f);
(h) further optionally modify the siRNA prepared in (f) to optimize the gene silencing and/or immunostimulatory activity.

The present invention also provides an alternative method for preparing siRNA with gene silencing activity and immunostimulatory activity, comprising the steps of:
(a) identifying all potential siRNA antisense sequences for a target mRNA;
(b) predicting the immunostimulatory activity for all of the antisense sequences identified in (a) and their complementary (i.e., sense) sequences;
(c) identifying 10 siRNA with the highest IFN-α scores, wherein the IFN-α score of the siRNA is the higher of the two scores for the sense and the antisense strand;
(d) identifying siRNA with gene silencing activity among the 10 siRNA identified in step (c);
(e) identifying10 siRNA with the next highest IFN-α scores if no siRNA can be identified in step (d); repeat steps (d) and (e) until at least one siRNA is identified;
(f) preparing the siRNA identified in step (d) or (e);
(g) optionally testing the gene silencing and/or the immunostimulatory activity of the siRNA prepared in (f);
(h) further optionally modify the siRNA prepared in (f) to optimize the gene silencing and/or immunostimulatory activity.

The present invention further provides a method for preparing an siRNA having gene silencing activity for a target gene and having low (or minimal) immunostimulatory activity, in particular, IFN-α-inducing activity, comprising the steps of:
(a) identifying all potential siRNA antisense sequences for a target mRNA;
(b) identifying antisense sequences that have gene silencing activity;
(c) predicting the immunostimulatory activity for the antisense sequences identified in step (b) and their complementary (i.e., sense) sequences;
(d) identifying siRNA wherein both the sense and the antisense sequences have an IFN-α score of at most 0.6075 x n - 9.9484, wherein n is the length of the sequence and n is between 19 and 25;
(e) increasing the IFN-α score threshold by 1 if no siRNA is identified in step (b), until at least one siRNA is identified;
(f) preparing the siRNA identified in step (d) or (e);
(g) optionally testing the gene silencing and/or the immunostimulatory activity of the siRNA prepared in (f);
(h) further optionally modify the siRNA prepared in (f) to optimize the gene silencing activity and/or to minimize the immunostimulatory activity.

The present invention also provide an alternative method for preparing siRNA with gene silencing activity and low (or minimal) immunostimulatory activity, comprising the steps of:
(a) identifying all potential siRNA antisense sequences for a target mRNA;
(b) predicting the immunostimulatory activity for all of the antisense sequences identified in (a) and their complementary (i.e., sense) sequences;
(c) identifying 10 siRNA with the lowest IFN-α scores, wherein the IFN-α score of the siRNA is the higher of the two scores for the sense and the antisense strand;
(d) identifying siRNA with gene silencing activity among the 10 siRNA identified in step (c);
(e) identifying 10 siRNA with the next lowest IFN-α scores if no siRNA can be identified in step (d); repeat steps (d) and (e) until at least one siRNA is identified;
(f) preparing the siRNA identified in step (d) or (e);
(g) optionally testing the gene silencing and/or the immunostimulatory activity of the siRNA prepared in (f);
(h) further optionally modify the siRNA prepared in (f) to optimize the gene silencing activity and/or to minimize the immunostimulatory activity.

Candidate siRNA with gene silencing activity for a given gene can be identified using methods known to those skilled in the art, including, but not limited to, commercial engines such as those available from Dharmacon, Qiagen, Invitrogen. Furthermore, the gene silencing activity of an siRNA may be predicted using the algorithm of Reynolds et al. (2004, Nat Biotechnol 22:326-330) and may be determined experimentally.

The gene silencing activity of an siRNA can be determined experimentally by methods well known in the art. For Example, the RNA oligonucleotide may be introduced into a cell by a method known in the art such as transfection and transduction; the mRNA level of the target gene can be determined by routine methods such as Northern blot analysis, quantitative PCR, RNase protection assay, and branching DNA; and the protein expression level can be determined by routine methods such as Western blotting, ELISA, and biological activity assays specific to the target protein. Furthermore, the mRNA level of all known and hypothetical genes can be determined at the global level using the microarray technology. Technologies in the field of proteonomics allow for the protein levels of a large number of genes to be determined at the global level as well.

The same methods may be used for preparing an shRNA which comprises the sense and the antisense sequences of an siRNA identified by the above methods and a loop sequence. The preferred loop sequences for shRNA are known to those skilled in the art.

The same methods may also be used for preparing antisense RNA wherein only one strand, the antisense strand, needs to be identified and prepared.

The siRNA, shRNA and antisense RNA can be prepared by methods including, but are not limited to, chemical synthesis, in vitro and in vivo transcription from PCR products and viral or non-viral vectors.

### Immunostimulatory RNA Oligonucleotides

The present invention provides an immunostimulatory RNA oligonucleotide having immunostimulatory activity, in particular, IFN-α-inducing activity, comprising at least one, preferably at least two, more preferably at least three, even more preferably at least four, even more preferably at least five, and most preferably at least six, of the 4-nucleotide (4mer) motifs selected from the group consisting of:
GUUC (No. 1), GUCA (No. 2), GCUC (No. 3), GUUG (No. 4), GUUU (No. 5),
GGUU (No. 6), GUGU (No. 7), GGUC (No. 8), GUCU (No. 9), GUCC (No. 10),
GCUU (No. 11), UUGU (No. 12), UGUC (No. 13), CUGU (No. 14), CGUC (No. 15),
UGUU (No. 16), GUUA (No. 17), UGUA (No. 18), UUUC (No. 19), UGUG (No. 20),
GGUA (No. 21), GUCG (No. 22), UUUG (No. 23), UGGU (No. 24), GUGG (No. 25),
GUGC (No. 26), GUAC (No. 27), GUAU (No. 28), UAGU (No. 29), GUAG (No. 30),
UUCA (No. 31), UUGG (No. 32), UCUC (No. 33), CAGU (No. 34), UUCG (No. 35),
CUUC (No. 36), GAGU (No. 37), GGUG (No. 38), UUGC (No. 39), UUUU (No. 40),
CUCA (No. 41), UCGU (No. 42), UUCU (No. 43), UGGC (No. 44), CGUU (No. 45),
CUUG (No. 46), UUAC (No. 47),
wherein the nucleotide sequences of the motifs are 5' → 3',
wherein the oligonucleotide is between 6 and 64, preferably between 12 and 50, more preferably between 14 and 40, even more preferably between 16 and 36, and most preferably between 18 and 25 nucleotides in length,
wherein at least one strand of the RNA oligonucleotide has an IFN-α score of at least 23 when n=6; at least 26 when n=7; at least 28 when n=8; at least 30 when n=9; at least 1.4909 x n + 22.014 when n is greater than 9,
wherein n is the length of the oligonucleotide,
and wherein the oligonucleotide is not 5'-UUGAUGUGUUUAGUCGCUA-3' (Judge et al. 2005, Nat Biotechnol 23:457-462), 5'-GCACCACUAGUUGGUUGUC-3' (Sioud 2005, J Mol Biol 348:1079-1090), 5'-GUUGUAGUUGUACUCCAGC-3' (Sioud), 5'-GCCCGUCUGUUGUGUGACUC-3' (Heil et al. 2004 Science 303:1526-1529), 5'-GUCUGUUGUGUG-3' (Heil, et al.), 5'-GUUGUGGUUGUGGUUGUG-3' (WO 03/086280).

In one embodiment, the 4mer motifs are selected from the group consisting of No. 1-19, preferably, No. 1-6 of the 4mer motifs.

The immunostimulatory RNA oligonucleotide of the invention may comprise one or more copies of the same 4mer motif, or one or more copies of different 4mer motifs.

The present invention also provide an immunostimulatory RNA oligonucleotide having immunostimulatory, in particular, IFN-α-inducing activity, comprising at least one, preferably at least two, more preferably at least three, even more preferably at least four, even more preferably at least five, most preferably at least six, of the 4mer motifs selected from the group consisting of No. 1-6 of the 4mer motifs, wherein the spacer nucleotides which are not part of any of the 4mer motif(s) are identical, and wherein the spacer nucleotide is selected from the group consisting of A, T, C, G and variants and derivatives thereof.

In one embodiment, the spacer nucleotide is A or a derivative thereof.

In one embodiment, the immunostimulatory RNA oligonucleotide of the invention can comprise one or more copies of one type of 4mer motif (e.g., GUUC) on a poly A backbone. Examples of such an oligonucleotide includes, but are not limited to:
AAAAAAAGUUCAAAAAAAA
AAAGUUCAAAAAAAAAAAA
AAAAAAAAAAAAGUUCAAA
AAAGUUCAAAAAGUUCAAA
GUUCAAAGUUCAAAGUUCA

In another embodiment, the immunostimulatory RNA oligonucleotide of the invention can comprise one or more copies of more than one type of 4mer motif (e.g., GUUC GUCA, GCUC, GUUG, GUUU, GGUU) on a poly A backbone. Examples of such an oligonucleotide includes, but are not limited to:
AGUUCAAAGUCAAAAGCUC
AGUUCAGUUCAAGUCAAAGCUC
AAAGUUCAAAGUCAAAAGCUCAAAGUUGAAAGUUUAAAGGUUAAA

The more than one 4mer motifs in an immunostimulatory RNA oligonucleotide may overlap. For example, AAAAGUUGCUCAAAAAA.

In one embodiment, immunostimulatory RNA oligonucleotide of the invention does not have gene silencing activity for any known mammalian gene.

The immunostimulatory RNA oligonucleotide of the invention may be single-stranded, single-stranded containing a self-complementary sequence and can form a hairpin structure, double-stranded, or partially double-stranded.

Furthermore, the immunostimulatory RNA oligonucleotide of the invention may be covalently linked to one or more lipophilic groups which enhance the stability and the activity and facilitate the delivery of the RNA oligonucleotides.

As used herein, the term "lipophilic" or "lipophilic group" broadly refers to any compound or chemical moiety having an affinity for lipids. Lipophilic groups encompass compounds of many different types, including those having aromatic, aliphatic or alicyclic characteristics, and combinations thereof.

In specific embodiments, the lipophilic group is an aliphatic, alicyclic, or polyalicyclic substance, such as a steroid (e.g., sterol) or a branched aliphatic hydrocarbon. The lipophilic group generally comprises a hydrocarbon chain, which may be cyclic or acyclic. The hydrocarbon chain may comprise various substituents and/or at least one heteroatom, such as an oxygen atom. Such lipophilic aliphatic moieties include, without limitation, saturated or unsatarated fatty acids, waxes (e.g., monohydric alcohol esters of fatty acids and fatty diamides), terpenes (e.g., the C₁₀ terpenes, C₁₅ sesquiterpenes, C₂₀ diterpenes, C₃₀ triterpenes, and C₄₀ tetraterpenes), and other polyalicyclic hydrocarbons.

The lipophilic group may be attached by any method known in the art, including via a functional grouping present in or introduced into the RNA oligonucleotide, such as a hydroxy group (e.g., -CO-CH₂ -OH). Conjugation of the RNA oligonucleotide and the lipophilic group may occur, for example, through formation of an ether or a carboxylic or carbamoyl ester linkage between the hydroxy and an alkyi group R-, an alkanoyl group RCO- or a substituted carbamoyl group KNHCO- The alkyl group R may be cyclic (e.g., cyclohexyl) or acyclic (e.g., straight-chained or branched; and saturated or unsaturated). Alkyl group R may be a butyl, pentyl, hexyl, heptyl, octyl, nonyl, decyl, undecyl, dodecyl, tridecyl, tetradecyl, pentadecyl, hexadecyl, heptadecyl or octadecyl group, or the like. Preferably, the lipophilic group is conjugated to the 5'-hydroxyl group of the terminal nucleotide. In a preferred embodiment, the liphophilic group is 12-hydroxydodeconoic acid bisdecylamide.

In another embodiment, the lipophilic group is a steroid, such as sterol. Steroids are polycyclic compounds containing a perhydro-1,2-cyclopentanophenanthrene ring system. Steroids include, without limitation, bile acids (e.g., cholic acid, deoxycholic acid and dehydrocholic acid), cortisone, digoxigenin, testosterone, cholesterol and cationic steroids, such as cortisone.

In a preferred embodiment, the lipophilic group is cholesterol or a derivative thereof. A "cholesterol derivative" refers to a compound derived from cholesterol, for example by substitution, addition or removal of substituents. The steroid may be attached to the RNA oligonucleotide by any method known in the art. In a preferred embodiment, the liphophilic group is cholesteryl (6-hydroxyhexyl) carbamate.

In another embodiment, the lipophilic group is an aromatic moiety. In this context, the term "aromatic" refers broadly to mono- and polyaromatic hydrocarbons. Aromatic groups include, without limitation, C₆-C₁₄ aryl moieties comprising one to three aromatic rings, which may be optionally substituted; "aralkyl" or "arylalkyl" groups comprising an aryl group covalently linked to an alkyl group, either of which may independently be optionally substituted or unsubstituted; and "heteroaryl" groups. As used herein, the term "heteroaryl" refers to groups having 5 to 14 ring atoms, preferably 5, 6, 9, or 10 ring atoms; having 6, 10, or 14π electrons shared in a cyclic array; and having, in addition to carbon atoms, between one and about three heteroatoms selected from the group consisting of nitrogen (N), oxygen (O), and sulfur (S).

As used herein, a "substituted" alkyl, cycloalkyl, aryl, heteroaryl, or heterocyclic group is one having between one and about four, preferably between one and about three, more preferably one or two, non-hydrogen substituents. Suitable substituents include, without limitation, halo, hydroxy, nitro, haloalkyl, alkyl, alkaryl, aryl, aralkyl, alkoxy, aryloxy, amino, acylamino, alkylcarbamoyl, arylcarbamoyl, aminoalkyi, alkoxycarbonyl, carboxy, hydroxyalkyl, alkanesulfonyl, arenesulfonyl, alkanesulfonamido, arenesulfonamido, aralkylsulfonamido, alkylcarbonyl, acyloxy, cyano, and ureido groups.

The lipophilic group can be covalently linked directly or indirectly via a linker to the RNA oligonucleotide. The covalent linkage may or may not comprise a phosphodiester group. And the linker may be of various lengths. The preferred lengths of the linker are known to those skilled in the art and may be determined experimentally.

In one embodiment, the lipophilic group is covalently linked to the 5' end of at least one strand of the RNA oligonucleotide.

In addition, the immunostimulatory oligonucleotide of the invention may be coupled to a solid support. By "coupled" it is meant that the oligonucleotide is covalently or non-covalently, directly or indirectly, linked to the solid support. Suitable solid supports include, but are not limited to, silicon wafers, synthetic polymer support such as polystyrene, polypropylene, polyglycidylmethacrylate, substituted polystyrene (e.g., aminated or carboxylated polystyrene, polyacrlamides, polyamides, polyvinylchlorides, etc.), glass, agarose, nitrocellulose, nylon and gelatin nanoparticles. Solid support may enhance the stability and the activity of the oligonucleotide, especially short oligonucleotides less than 16 nucleotides in length.

### Immunostimulatory RNA Oligonucleotide Conjugates

The present invention also provides immunomodulatory RNA oligonucleotide conjugates, comprising an immunomodulatory RNA oligonucleotide and an antigen conjugated to the oligonucleotide. In some embodiments, the antigen is conjugated to the oligonucleotide at a position other than its 3' end. In some embodiments, the antigen produces a vaccine effect.

The antigen is preferably selected from the group consisting of disease/disorder-related antigens. The disorder may be a cancer, a dermatological disorder, an immune disorder, a metabolic disorder, a neurological disorder, an ocular disease, an infection, or other hereditary and non-hereditary disorders. The antigen may be a protein, a polypeptide, a peptide, a carbohydrate, or a combination thereof.

The immunostimulatory RNA oligonucleotide may be covalently linked to the antigen, or it is otherwise operatively associated with the antigen. As used herein, the term "operatively associated with" refers to any association that maintains the activity of both the oligonucleotide and the antigen. Non-limiting examples of such operative associations include being part of the same liposome or other such delivery vehicle or reagent. In embodiments wherein the oligonucleotide agent is covalently linked to the antigen, such covalent linkage preferably is at any position on the oligonucleotide that does not interfere with the immunostimulatory activity of the oligonucleotide.

### RNA Oligonucleotide with Gene Silencing Activity

The present invention provides a RNA oligonucleotide with gene silencing activity.

In one embodiment, the RNA oligonucleotide has both gene silencing activity and immunostimulatory activity, wherein at least one strand of the oligonucleotide has an IFN-α score of at least 1.4909 x n + 31.014, wherein n is the length of the oligonucleotide.

In another embodiment, the RNA oligonucleotide has gene silencing activity and low /minimal immunostimulatory activity, wherein all strand(s) of the oligonucleotide has(have) an IFN-α score of at most 0.6075 x n - 9.9484, wherein n is the length of the oligonucleotide.

The RNA oligonucleotide may be an siRNA, an shRNA or an antisense RNA. The siRNA is between 14 and 25 nucleotides in length; the shRNA is between 30 and 70 nucleotides in length; and the antisese RNA is between 14 and 50 nucleotides in length.

In the case of an immunostimulatory siRNA, the gene silencing activity resides on the antisense strands which is complementary to the target mRNA, whereas the immunostimulatory activity may reside on either the sense or the antisesen strand. In a perferred embodiment, the immunostimulatory activity resides on the sense strand; i.e., the sense strand has an IFN-α score of at least 1.4909 x n + 31.014.

In the case of an shRNA, the molecule is processed inside a cell to yield a siRNA molecule and the loop (linker) sequence. Therefore, the IFN-α score needs to be calculated not only for the intact molecule (a single-stranded RNA), but also for both strands of the resulting siRNA molecule and the single-stranded loop sequence. The shRNA is considered to have high immunostimulatory activity if at least one of the above-mentioned entities has an IFN-α score above the threshold of 1.4909 x n + 31.014; the molecule is considered to have low immunostimulatory activity if all of the entities mentioned above have an IFN-α score below the threshold of 0.6075 x n - 9.9484. The gene silencing activity resides in the sequence that corresponds to the antisense strand of the siRNA; whereas the immunostimulatory activity may reside in any part of the molecule. In a preferred embodiment, the immunostimulatory activity resides in the portion of an shRNA that corresponds to the sense strand of the corresponding siRNA or in the loop sequence; i.e., said portion has an IFN-a score of at least 1.4909 x n + 31.014.

In the case of an immunostimulatory antisense RNA, the immunostimulatory activity and the gene silencing activity have to reside on the same strand.

The gene silencing RNA oligonucleotide of the invention may be covalently linked to one or more lipophilic groups which enhance the stability and the activity and facilitate the delivery of the RNA oligonucleotides.

### Pharmaceutical Compositions

The present invention provides pharmaceutical compositions comprising one or more of the RNA oligonucleotides of the invention and a pharmaceutically acceptable carrier. The more than one RNA oligonucleotides may have the same, similar, or different functionalities including, but are not limited to immunostimulatory activity and gene silencing activity.

For example, a RNA oligonucleotide having immunostimulatory activity but lacking gene silencing activity may be combined with a RNA oligonucleotide having gene silencing activity and low immunostimulatory activity in a pharmaceutical composition to achieve both immune activation and gene silencing. Such a combination composition may be useful for treating disorders such as cancers and viral infections. Such a combination composition may be necessary when the two activities cannot be optimized on a single RNA oligonucleotide.

In one embodiment, the pharmaceutical composition further comprises a RNA complexation agent. In a preferred embodiment, the complexation agent is a polycationic peptide, preferably poly-L-arginine (poly-L-Arg). In a preferred embodiment, the polycationic peptide, in particular, poly-L-Arg, is at least 24 amino acids in length. The polycationic peptide, in particular, poly-L Arg, may be a heterogeneous mixture of peptides of different lengths.

The pharmaceutical composition of the invention may further comprises another agent such as an agent that stabilizes the RNA oligonucleotide(s), e.g., a protein that complexes with the oligonucleotide agent to form an iRNP. Still other agents include chelators, e.g., EDTA (e.g., to remove divalent cations such as Mg²⁺), salts, RNAse inhibitors (e.g., a broad specificity RNAse inhibitor such as RNAsin) and so forth.

The pharmaceutical composition of the present invention can further comprise one or more additional pharmaceutically active (or therapeutic) agents which are selected from the group consisting of agents that are used for the treatment of cancer, dermatological disorders, immune disorders, metabolic disorders, neurological disorders, ocular diseases, infections, and other hereditary and non-hereditary disorders in a mammal.

In certain embodiments, the additional pharmaceutically active agent is selected from the group consisting of immunostimulatory RNA oligonucleotides, immunostimulatory DNA oligonucleotides, cytokines, chemokines, growth factors, antibiotics, anti-angiogenic factors, chemotherapeutic agents, anti-viral agents, anti-fungal agents, anti-parasitic agents, and antibodies. In one embodiment, the additional pharmaceutically active agent is natural or recombinant IFN-α polypeptide, or a CpG-containing or non-CpG-containing DNA oligonucleotide capable of inducing IFN-α (see e.g., WO 01/22990, WO 03/101375). In another embodiment, the additional pharmaceutically active agent is natural or recombinant IL-12, or an immunostimulatory RNA oligonucleotide capable inducing IL-12 (see e.g. our co-pending application; Sugiyama et al. 2005, J Immunol 174:2273-2279). In yet another embodiment, the additional pharmaceutically active agent is an anti-angiogenic factor such as vasostatin or an anti-VEGF antibody. In certain embodiments, the additional pharmaceutically active agent is a cancer-specific agent such as Herceptin, Rituxan, Gleevec, Iressa.

A formulated oligonucleotide composition can assume a variety of states. In some examples, the composition is at least partially crystalline, uniformly crystalline, and/or anhydrous (e.g., less than 80, 50, 30, 20, or 10% water). In another example, the oligonucleotide agent is in an aqueous phase, e.g., in a solution that includes water, this form being the preferred form for administration via inhalation.

The aqueous phase or the crystalline compositions can be incorporated into a delivery vehicle, e.g., a liposome (particularly for the aqueous phase), or a particle (e.g., a microparticle as can be appropriate for a crystalline composition). Generally, the oligonucleotide composition is formulated in a manner that is compatible with the intended method of administration.

The pharmaceutical compositions encompassed by the invention may be administered by any means known in the art including, but not limited to, oral or parenteral routes, including intravenous, intramuscular, intraperitoneal, subcutaneous, transdermal, airway (aerosol), ocular, rectal, vaginal, and topical (including buccal and sublingual) administration. In preferred embodiments, the pharmaceutical compositions are administered by intravenous or intraparenteral infusion or injection. The pharmaceutical compositions can also be administered intraparenchymally, intrathecally, and/or by stereotactic injection.

For oral administration, the oligonucleotide agent useful in the invention will generally be provided in the form of tablets or capsules, as a powder or granules, or as an aqueous solution or suspension.

Tablets for oral use may include the active ingredients mixed with pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredient is mixed with water or an oil such as peanut oil, liquid paraffin or olive oil.

For intramuscular, intraperitoneal, subcutaneous and intravenous use, the pharmaceutical compositions of the invention will generally be provided in sterile aqueous solutions or suspensions, buffered to an appropriate pH and isotonicity. Suitable aqueous vehicles include Ringer's solution and isotonic sodium chloride. In a preferred embodiment, the carrier consists exclusively of an aqueous buffer. In this context, "exclusively" means no auxiliary agents or encapsulating substances are present which might affect or mediate uptake of oligonucleotide agent in the cells that harbor the target gene or virus. Such substances include, for example, micellar structures, such as liposomes or capsids, as described below. Although microinjection, lipofection, viruses, viroids, capsids, capsoids, or other auxiliary agents are required to introduce oligonucleotide agent into cell cultures, surprisingly these methods and agents are not necessary for uptake of oligonucleotide agent in vivo. The oligonucleotide agent of the present invention are particularly advantageous in that they do not require the use of an auxiliary agent to mediate uptake of the oligonucleotide agent into the cell, many of which agents are toxic or associated with deleterious side effects. Aqueous suspensions according to the invention may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

The pharmaceutical compositions can also include encapsulated formulations to protect the oligonucleotide agent against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811; PCT publication WO 91/06309; and European patent publication EP-A-43075.

In general, a suitable dose of a RNA oligonucleotide will be in the range of 0.001 to 500 milligrams per kilogram body weight of the recipient per day (e.g., about 1 microgram per kilogram to about 500 milligrams per kilogram, about 100 micrograms per kilogram to about 100 milligrams per kilogram, about 1 milligrams per kilogram to about 75 milligrams per kilogram, about 10 micrograms per kilogram to about 50 milligrams per kilogram, or about 1 microgram per kilogram to about 50 micrograms per kilogram). The pharmaceutical composition may be administered once per day, or the oligonucleotide agent may be administered as two, three, four, five, six or more sub-doses at appropriate intervals throughout the day. In that case, the oligonucleotide agent contained in each sub-dose must be correspondingly smaller in order to achieve the total daily dosage. The dosage unit can also be compounded for delivery over several days, e.g., using a conventional sustained release formulation which provides sustained release of the oligonucleotide agent over a several day period. Sustained release formulations are well known in the art. In this embodiment, the dosage unit contains a corresponding multiple of the daily dose.

The skilled artisan will appreciate that certain factors may influence the dosage and timing required to effectively treat a subject, including but not limited to the severity of the infection or disease/disorder, previous treatments, the general health and/or age of the subject, and other diseases/disorders present. Moreover, treatment of a subject with a therapeutically effective amount of a composition can include a single treatment or a series of treatments. Estimates of effective dosages and in vivo half-lives for the individual RNA oligonucleotide agent encompassed by the invention can be made using conventional methodologies or on the basis of in vivo testing using an appropriate animal model, as described elsewhere herein.

Toxicity and therapeutic efficacy of the RNA oligonucleotide and the pharmacuetical composition of the invention can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. oligonucleotide agents that exhibit high therapeutic indices are preferred.

The data obtained from cell culture assays and animal studies can be used in formulating a range of dosage for use in humans. The dosages of compositions of the invention are preferably within a range of circulating concentrations that include the ED50 with little or no toxicity. The dosage may vary within this range depending upon the dosage form employed and the route of administration utilized. For any oligonucleotide agent used in the method of the invention, the therapeutically effective dose can be estimated initially from cell culture assays. A dose may be formulated in animal models to achieve a circulating plasma concentration range of the oligonucleotide agent or, when appropriate, of the polypeptide product of a target sequence (e.g., achieving a decreased concentration of the polypeptide) that includes the IC50 (i.e., the concentration of the test oligonucleotide agent which achieves a half-maximal inhibition of symptoms) as determined in cell culture. Such information can be used to more accurately determine useful doses in humans. Levels in plasma may be measured, for example, by high performance liquid chromatography.

The administering physician can adjust the amount and timing of the administration of the pharmaceutical composition of the invention on the basis of results observed using standard measures of efficacy known in the art or described herein.

### Use of the RNA Oligonucleotide for Inducing an Immune Response

The present application provides the use of the immunostimulatory RNA oligonucleotide of the invention for the preparation of a pharmaceutical composition for inducing an immune response in a mammal.

Inducing an immune response means initiating or causing an increase in one or more of B-cell activation, T-cell activation, natural killer cell activation, activation of antigen presenting cells (e.g., B cells, dendritic cells, monocytes and macrophages), cytokine production, chemokine production, specific cell surface marker expression, in particular, expression of co-stimulatory molecules. In one aspect, such an immune response involves the production of type I IFN, in particular, IFN-α, in cells such as PDC.

### Use of the RNA Oligonucleotide for Treating Diseases/Disorders

The present invention provides the use of the immunostimulatory RNA oligonucleotide of the invention for the preparation of a pharmaceutical composition for preventing and/or treating a disorder selected from immune disorders, infections, and cancers in a mammal, wherein the induction of an immune response is beneficial to the mammal.

The present invention also provides the use of the RNA oligonucleotide of the invention which has both immunostimulatory activity and gene silencing activity for the preparation of a pharmaceutical composition for preventing and/or treating a disorder selected from infections and cancers in a mammal, wherein the induction of an immune response together with the downregulation of a disorder-related gene are beneficial to the mammal.

The present invention further provides the use of the RNA oligonucleotide of the invention which has gene silencing activity and low/minimal immunostimulatory activity for the preparation of a pharmaceutical composition for preventing and/or treating a disorder in a mammal caused by the expression or overexpression of a disorder-related gene, wherein the induction of an immune disorder it to be avoided. The disorder may be selected from cancer, dermatological disorders, immune disorders, metabolic disorders, neurological disorders, ocular diseases, infections, and other hereditary and non-hereditary disorders.

The immune disorders include, but are not limited to, allergy, autoimmune disorders, inflammatory disorders, and immunodeficiency.

Allergies include, but are not limited to, food allergies and respiratory allergies.

Autoimmune diseases include, but are not limited to, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, automimmune thyroiditis, dermatitis (including atopic dermatitis and eczematous dermatitis), psoriasis, Sjogren's Syndrome, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing, loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis.

Inflammatory disorders include, without limitation, airway inflammation which includes, without limitation, asthma.

Immunodeficiencies include, but are not limited to, spontaneous immunodeficiency, acquired immunodeficiency (including AIDS), drug-induced immunodeficiency (such as that induced by immunosuppressants used in transplantation and chemotherapeutic agents used for treating cancer).

In one embodiment, the immune disorders include those caused by pathological Th2 responses.

The infections include, but are not limited to viral infections, bacterial infections, anthrax, parasitic infections, fungal infections and prion infection.

Viral infections include, but are not limited to, infection by hepatitis C, hepatitis B, herpes simplex virus (HSV), HIV-AIDS, poliovirus, and smallpox virus. Examples of (+) strand RNA viruses which can be targeted for inhibition include, without limitation, picornaviruses, caliciviruses, nodaviruses, coronaviruses, arteriviruses, flaviviruses, and togaviruses. Examples of picornaviruses include enterovirus (poliovirus 1), rhinovirus (human rhinovirus 1A), hepatovirus (hepatitis A virus), cardiovirus (encephalomyocarditis virus), aphthovirus (foot-and-mouth disease virus O), and parechovirus (human echovirus 22). Examples of caliciviruses include vesiculovirus (swine vesicular exanthema virus), lagovirus (rabbit hemorrhagic disease virus), "Norwalk-like viruses" (Norwalk virus), "Sapporo-like viruses" (Sapporo virus), and "hepatitis E-like viruses" (hepatitis E virus). Betanodavirus (striped jack nervous necrosis virus) is the representative nodavirus. Coronaviruses include coronavirus (avian infections bronchitis virus) and torovirus (Berne virus). Arterivirus (equine arteritis virus) is the representative arteriviridus. Togavirises include alphavirus (Sindbis virus) and rubivirus (Rubella virus). Finally, the flaviviruses include flavivirus (Yellow fever virus), pestivirus (bovine diarrhea virus), and hepacivirus (hepatitis C virus).

In certain embodiments, the viral infections are selected from chornic hepatitis B, chornic hepatitis C, HIV infection, RSV infection, HSV infection, VSV infection, CMV infection, and influenza infection.

Cancers include, but are not limited to biliary tract cancer, brain cancer, breast cancer, cervical cancer, choriocarcinoma, colon cancer, endometrial cancer, esophageal cancer, gastric cancer, intraepithelial neoplasm, leukemia, lymphoma, liver cancer, lung cancer, melanoma, myelomas, neuroblastoma, oral cancer, ovarian cancer, pancreatic cancer, prostate cancer, rectal cancer, sarcoma, skin cancer, testicular cancer, thyroid cancer and renal cancer.

In certain embodiments, cancers are selected from hairy cell leukemia, chronic myelogenous leukemia, cutaneous T-cell leukemia, chronic myeloid leukemia, non-Hodgkin's lymphoma, multiple myeloma, follicular lymphoma, malignant melanoma, squamous cell carcinoma, renal cell carcinoma, prostate carcinoma, bladder cell carcinoma, breast carcinoma, ovarian carcinoma, non-small cell lung cancer, small cell lung cancer, hepatocellular carcinoma, basaliom, colon carcinoma, cervical dysplasia, and Kaposi's sarcoma (AIDS-related and non-AIDS related).

Dermatological disorders include, but are not limited to, psoriasis, acne, rosacea, eczema, molluscum contagious, seborrheic keratosis, actinic keratosis, verruca vulgaris.

Metabolic disorders include, but are not limited to, diabetes and obesity.

Ocular diseases include, but are not limited to, age-related macular degeneration.

Neurological disorders include, but are not limited to, Alzeimer' disease, Huntington's disease, Parkinson's disease, and spinal cord injury.

Hereditary diseases include, but are not limited to, cystic fibrosis.

In one embodiment, the pharmaceutical composition is for administration selected from the group consisting of airway, oral, ocular, parenteral (including intraveneous, intradermal, intramuscular, intraperitoneal, and subcutaneous), rectal, vaginal and topical (including buccal and sublingual) administration.

In another embodiment, the pharmaceutical composition is for use in combination with one or more treatments of disorders selected from treatments for cancer, dermatological disorders, immune disorders, metabolic disorders, neurological disorders, ocular diseases, infections, and other hereditary and non-hereditary disorders in a mammal. Such treatments include, but are not limited to, surgery, chemotherapy, radiation therapy, and the administration of pharmaceutically active (or therapeutic) agents such as immunostimulatory RNA oligonucleotides, immunostimulatory DNA oligonucleotides, cytokines, chemokines, growth factors, antibiotics, anti-angiogenic factors, chemotherapeutic agents, anti-viral agents, anti-fungal agents, anti-parasitic agents, and antibodies.

In one embodiment, pharmaceutically active agent is natural or recombinant IFN-α polypeptide, or a CpG-containing or non-CpG-containing DNA oligonucleotide capable of inducing IFN-α (see e.g., WO 01/22990, WO 03/101375). In another embodiment, the pharmaceutically active agent is natural or recombinant IL-12, or an immunostimulatory RNA oligonucleotide capable inducing IL-12 (see e.g. our co-pending application; Sugiyama et al. 2005, J Immunol 174:2273-2279). In yet another embodiment, the pharmaceutically active agent is an anti-angiogenic factor such as vasostatin or an anti-VEGF antibody. In certain embodiments, the pharmaceutically active agent is a cancer-specific agent such as Herceptin, Rituxan, Gleevec, Iressa.

Mammals include, but are not limited to, rats, mice, cats, dogs, horses, sheep, cattle, cows, pigs, rabbits, non-human primates, and humans. In a preferred embodiment, the mammal is human.

### In Vitro Method for Inducing IFN-α Production

The present invention provides an in vitro method of inducing IFN-α production in a mammalian cell, comprising the steps of:
(a) complexing an immunostimulatory RNA oligonucleotide of the invention with a complexation agent; and
(b) contacting the cell with the complex prepared in step (a).
   The mammalian cell is capable of producing IFN-α. In one embodiment, the mammalian cell expresses TLR7, TLR8, or both TLR7 and TLR8. The mammalian cells include, but are not limited to, peripheral blood mononuclear cells (PBMC), plasmacytoid dendritric cells (PDC), myeloid dendritic cells (MDC), B cells, macrophages, monocytes, natural killer cells, granulocytes, endothelial cells, cell lines such as THP1, and cells containing exogenous DNA which directs the expression of TLR7 or TLR8 or both TLR7 or TLR8 such as transfected CHO, HEK293 or COS cells.

In one embodiment of the invention, the complexation agent is a polycationic peptide, preferably poly-L-arginine (poly-L-Arg). In one embodiment, the polycationic peptide, in particular, poly-L-Arg, is at least 24 amino acids in length. The polycationic peptide, in particular, poly-L Arg, may be a heterogeneous mixture of peptides of different lengths.

In a preferred embodiment, the mammal is human.

### In Vitro Method for Activating Mammalian Dendritic Cells

The present application provides an in vitro method of activating mammalian dendritic cells, comprising the steps of:
(a) complexing an immunostimulatory RNA oligonucleotide of the invention with a complexation agent;
(b) contacting dendritic cells isolated from a donor mammal with the complexed RNA oligonucleotide; and
(c) contacting the dendritic cells with an antigen.

In one embodiment of the invention, the complexation agent is a polycationic peptide, preferably poly-L-arginine (poly-L-Arg). In one embodiment, the polycationic peptide, in particular, poly-L-Arg, is at least 24 amino acids in length. The polycationic peptide, in particular, poly-L Arg, may be a heterogeneous mixture of peptides of different lengths.

In one embodiment, the antigen is a disease/disorder-related antigen. The disorder may be a cancer, a dermatological disorder, an immune disorder, a metabolic disorder, a neurological disorder, an ocular disease, an infection, or other hereditary and non-hereditary disorders. The antigen may be a protein, a polypeptide, a peptide, a carbohydrate, or a combination thereof.

The present invention further provides the use of the in vitro activated dendritic cells for the preparation of a medicament for inducing an immune response in a mammal, wherein the in vitro activated dendritic cells are for transfer into a recipient that is the same or different from the donor.

In a preferred embodiment, the mammal is human.

The present invention is illustrated by the following examples.

### Examples

### Example 1. Stimulation of PBMC using poly-L-arginine-complexed single-stranded RNA oligonucleotides

First we sought to develop an assay system for the comparison of the IFN-a inducing capacity of single-stranded RNA (ssRNA) oligonucleotides on a large scale. Peripheral blood mononuclear cells (PBMC) were selected as a biological system that is relevant for future clinical application. Within PBMC, the plasmacytoid dendritic cell (PDC) is responsible for IFN-α production upon stimulation with ssRNA oligonucleotides. In order to stimulate IFN-α, production in PDC, ssRNA oligonucleotides require transfection. While cationic lipids such as lipofectamine are well-established to support IFN-α production in isolated PDC, the induction of IFN-α in PBMC was not satisfactory. In order to improve transfection of RNA in PBMC we tested a number of cationic polypeptides including poly-L-Lys, poly-His and poly-L-Arg. Among these three, poly-L-Arg (14 kD) was the most potent polycationic peptide and was even more potent than the cationic lipid lipofectamine to support the stimulatory activity of an established immunostimulatory RNA oligonucleotide (RNA9.2sense) (Fig. 1). Within PBMC, the source of IFN-α production were found to be PDC, since PDC depletion abrogated IFN-α production (data not shown). Importantly, poly-L-Arg complexation maintained the same sequence dependency (sense versus antisense strand) of ssRNA oligonucleotide-mediated IFN- α induction that was previously seen for lipofectamine (Hornung et al. 2005, Nat Med 11:263-270). This experimental system was found to be robust, since adding different concentrations of RNA oligonucleotide from 80 % to 120 % had little effect on the amount of IFN-α induced. This is in marked contrast to the use of cationic lipids that require exact adjustment of the net charges to avoid cytotoxicity. Together these data indicated that poly-L-Arg complexation is an effective and reliable system to screen for ssRNA oligonucleotide-induced IFN-α production in PBMC.

The experimental procedures are described in more detail in the following:

### PBMC isolation

Human PBMC were prepared from whole blood donated by young healthy donors. PBMC were obtained from whole blood by Ficoll-Hypaque density gradient centrifugation (Biochrom, Berlin, Germany). PBMC were cultured in RPMI-Medium (Biochrom, Berlin, Germany) supplemented with human AB-Serum (2 vol%, Firma, Germany) at a density of 2x10⁶ PBMC/ ml. Subsequently PBMC were plated into 96-well flat bottom wells at 200µl/ well. Cells were kept on ice until stimulation.

### Resuspension and annealing of ssRNA oligonucleotides

Lyophilized ssRNA oligonucleotides (Eurogentec, Belgium) were resuspended in sterile, RNase free water at a concentration of 620µg/ml. Subsequently 40µl of the ssRNA oligonucleotides, 40µl sterile, RNase free water and 20µl 5x annealing buffer (50 mM Tris-HCl, pH 8.0, 100 mM NaCl, 5 mM EDTA) were combined. This resulted in a final volume of 100µl with a final concentration of the ssRNA oligonucleotide of 248µg/ml. Subsequently the solution was incubated at 95°C for 2 minutes and cooled down to 20°C with a linear decrease in temperature in 60 minutes (-1.25 °C/ 1 minute). The solution was then stored at -80°C prior to usage (ssRNA oligonucleotide stock solution).

### Stimulation

If not otherwise indicated, ssRNA oligonucleotides were complexed using the polycationic polymer Poly-L-arginine Hydrochloride with a molecular weight of 5.000 - 15.000 (Prod. Number: P4663, Sigma, Munich, Germany). Lyophilized Poly-L-arginine Hydrochloride was dissolved in sterile, RNase free water at a concentration of 2000 µg/ml (Poly-L-arginine stock solution) and subsequently aliquoted and stored at a temperature of -20°C. ssRNA oligonucleotides were complexed using the following protocol:
1. The poly-L-arginine stock solution and the ssRNA oligonucleotide stock solution are thawed to 4°C.
2. Poly-L-arginine is diluted in Phosphate buffered saline to a concentration of 24 µg/ml (1 : 83.3 dilution from 2000 µg/ml stock solution).
3. Immediately the ssRNA oligonucleotide is added to a final concentration of 14.8 µg/ml (1 : 16.6 dilution from 248 µg/ml stock solution).
4. Subsequently, the solution is mixed thoroughly by vortexing for 10 seconds.
5. Next, 15µl of this solution is added immediately to 200µl PBMC in the 96-well flat bottom plate. The final concentration of poly-L-arginine is 1.67 µg/ml, whereas the final concentration of the ssRNA oligonucleotide is 1.03 µg/ml.

Throughout all procedures the temperature was kept between 20 - 25°C. On all 96-well plates PBMC from three individual donors were plated, whereas eleven different ssRNA oligonucleotides were tested. In addition, on each plate the ssRNA oligonucleotide 9.2 sense (5'-AGCUUAACCUGUCCUUCAA-3') was included as a positive control. All tested ssRNA oligonucleotides were run in duplicates. After stimulation cells were cultured at 37°C / 5% CO2 for 44 hours.

### ELISA

After 44 hours of cell culture supernatants were collected and stored in three individual aliquots at -80 °C. Prior to ELISA-procedures frozen supernatants were thawed at 20 - 25°C for two hours. To measure IFN-a the IFN-a module set Bender MedSystems (Prod. Number: BMS216MST, Graz, Austria) was used. This ELISA detects most of IFN-α isoforms at a detection range of 8-500 pg/ml. All ELISA procedures were performed according to manufacturer's recommendations.

### Example 2. Rational design of a 4mer-motif library to screen for potent motifs within ssRNA

Previous experiments have shown that a minimal length of 19 bases is required for maximal IFN-α induction by ssRNA oligonucleotides. Since poly adenosine oligonucleotides proved to be inactive in terms of IFN-α induction, we decided to generate a ssRNA oligonucleotide library by placing putative motifs into the center of poly adenosine RNA oligonucleotides. In a first set of experiments we determined the minimal length of a motif for IFN-α induction in our system. We designed a panel of 19mer poly-adenosine ssRNA oligonucleotides with increasing numbers of uracil in the center: 5'-U-3', 5'-UU-3', 5'-UUU-3' and 5'-UUUU-3'. These oligonucleotides were compared to the previously published standard ssRNA oligonucleotide RNA9.2sense (5'-AGCUUAACCUGUCCUUCAA-3'): while the 1mer motif 5'-U-3'hardly induced IFN-α, (0.8 % of RNA9.2sense), considerable amounts of IFN-a were observed for the 2mer motif 5'-UU-3'(10.6 % of RNA9.2sense), the 3mer motif 5'-UUU-3' (15.7 % of RNA9.2sense) and the 4mer motif 5'-UUUU-3'(50.5 % of RNA9.2sense) (Fig. 2). Since the largest increment of IFN-a induction was seen between the 3mer and 4mer motif, we generated a library of ssRNA oligonucleotides comprising all possible 4mer motifs in the centre of a poly adenosine oligonucleotide. In view of the fact that the flanking adenosine residues can be part of a 4mer RNA sequence motif, only 193 ssRNA oligonucleotides (table 1) were needed to cover all 256 possible 4mer motifs.

### Example 3. Generation and processing of raw data

All 193 ssRNA oligonucleotides were tested on PBMC of six individual healthy donors using poly-L-Arg for complexation. At 44 hours after stimulation with RNA oligonucleotides, supernatants were collected and IFN-α production was measured by ELISA. Prior to statistical analysis the raw data were processed as follows: for each cell culture plate the mean IFN-□ value of the experimental duplicates for each tested ssRNA oligonucleotide were normalized to the ssRNA oligonucleotide RNA9.2sense (5'-AGCUUAACCUGUCCUUCAA-3'). This standard RNA oligonucleotide was included as a positive control on all cell culture plates. Normalization was performed by calculating the ratio of IFN-α induced by the tested oligonucleotide and IFN-α induced by the standard oligonucleotide RNA9.2sense. Thus, for each tested oligonucleotide in an individual donor a mean ratio of IFN-a induction was obtained. In the following, this mean of the ratios is referred to as IFN-a index (one value of IFN-a index per donor). For example testing ssRNA oligonucleotide ANP144 (5'-AAAAAAAGUUGAAAAAAAA-3') in donor 1 gave the mean of the two raw values of the duplicates (IFN-α in supernatant) of 2024 pg/ml, whereas the control oligonucleotide RNA9.2sense (5'-AGCUUAACCUGUCCUUCAA-3') resulted in 1256 pg/ml. The corresponding IFN-α index of oligonucleotide ANP144 for donor 1 was calculated to be 1.61 (= 2024 pg/ml divided by 1256 pg/ml).

Next, the means of all IFN-α indices for every individual donor were calculated. Then the adjusted IFN-α indices were calculated as IFN-α index minus the mean of all IFN-α indices of one individual donor. For example ssRNA oligonucleotide ANP144 of donor 1 (5'-AAAAAAAGUUGAAAAAAAA-3') had an IFN-α index of 1.61, whereas the mean of all IFN-a indices of donor 1 was 0.37. The adjusted IFN-a index of ANP144 was calculated: 1.61 minus 0.37 = 1.24 ( = substracting 0.37 from). The adjusted IFN-α indices from all six donors were summarized by calculating the means and the corresponding standard error of mean. The data are depicted in ascending order (Fig. 3A, B, C, D). The adjusted IFN-a indices of the top thirty ssRNA oligonucleotides of all six donors were compared using a two-tailed Student's t-test (Fig. 3E). For most combinations tested, a significant difference was observed when the interval between the analyzed pairs was at least six or seven places in the assortment.

The ssRNA oligonucleotides were split into two groups: group 1 (table 2) comprising all ssRNA oligonucleotides with an IFN-α index that lower than the mean IFN-α index off all ssRNA oligonucleotides (or in other words with a mean of the adjusted IFN-α index below 0) (table 1), and group 2 (table 3) comprising all ssRNA oligonucleotides with an IFN-a index higher than the mean IFN-α index off all tested ssRNA oligonucleotides (or in other words with a mean of the adjusted IFN-α index below 0) (table 1). Thus group 1 contained all ssRNA oligonucleotides with an adjusted IFN-α index below 0, whereas group 2 contained all ssRNA oligonucleotides with an adjusted IFN-a index above 0. Group 1 consisted of 148 ssRNA oligonucleotides (75 %), whereas group 2 consisted of 45 ssRNA oligonucleotides (25 %).

### Example 4. Analyzing the predictive value of 1 mer, 2mer and 3mer motifs within the 4mer motif matrix

Next the frequency of 1 mer motifs (5'-X-3'), 2mer motifs (5'-XX-3', 5'-X*X-3', 5'-X**X-3') and 3mer motifs (5'-XXX-3', 5'-XX*X-3', 5'-X*XX-3') in ssRNA oligonucleotides with an IFN-a index below the mean IFN-a index (group 1) and above the mean IFN-α index (group 2) was analyzed. Multiple occurrences of a motif within one single ssRNA oligonucleotide was accounted for. For example, the motif 5'-GU-3' is present twice in the ssRNA oligonucleotide ANP142 (5'-AAAAAAAGUGUAAAAAAAA-3', group 2). Consequently the ssRNA oligonucleotide ANP142 contributed two counts for motif 5'-GU-3' within group 2. In order to compare the distribution of a specific motif between the two groups, the relative occurrence of a specific motif was calculated (ratio of the absolute number of occurrence of a specific motif over the total number of occurrence of all motifs). For example the motif 5'-GU-3'was found 7 times in group 1 (total number of 5'-XX-3'-motifs in group 1: 2482) and 33 times in group 2 (total number of 5'-XX-3'-motifs in group 2: 799). Thus the calculated relative occurrence for motif 5'-GU-3' in group 1 was 0.0028, whereas the respective relative occurrence for group 2 was 0.0413. In figure 4 the relative occurence of 1 mer motifs (5'-X-3'), 2mer motifs (5'-XX-3', 5'-X*X-3', 5'-X**X-3') and 3mer motifs (5'-XXX-3', 5'-XX*X-3', 5'-X*XX-3') within the two groups is shown. A significant overrepresentation or underrepresentation of a given motif was analyzed using a chi-square test. The null hypothesis of equal distribution within both groups was rejected when the calculated p-value was below 0.05 (significant differences in distribution are indicated by "*" in Fig. 4).

### Example 5. Calculating an individual IFN-a score for 1 mer-, 2mer- and 3mer-motifs

A mean IFN-α index for all possible 1 mer motifs (5'-X-3'), 2mer motifs (5'-XX-3', 5'-X*X-3', 5'-X**X-3') or 3mer motifs (5'-XXX-3', 5'-XX*X-3', 5'-X*XX-3') was obtained by calculating a mean IFN-α index of all ssRNA oligonucleotides containing the corresponding motifs. This mean IFN-a index is referred to as the IFN-α score of a given motif. For example the 3mer motif 5'-GUC-3' was contained in ssRNA oligonucleotides ANP 35, 83, 131, 137, 138, 139 and 179 with respective adjusted IFN-α indices of 1.33, 0.68, 0.93, 0.79, 0.44, 0.84, 0.73. The IFN-α score of the 3mer motif 5'-GUC-3'was thus calculated to be 0.82 with a standard error of mean of 0.11. The calculation of the IFN-α score of a motif did not account for the position of the motif within the sequence of the corresponding ssRNA oligonucleotides. Multiple occurrences of one motif within the same ssRNA oligonucleotide was accounted for by adding the corresponding IFN-α index times the number of its occurrence within the oligonucleotide to the calculation of the corresponding IFN-α score of the motif. Consequently an IFN-α score could be assigned to all possible 1 mer motifs (5'-X-3'), 2mer motifs (5'-XX-3', 5'-X*X-3', 5'-X**X-3') or 3mer motifs (5'-XXX-3', 5'-XX*X-3', 5'-X*XX-3') (Fig. 5).

### Example 6. Predicting the IFN-α index of ssRNA oligonucleotides using the IFN-α score of 1 mer-, 2mer- and 3mer-motifs

Next we tested the predictive value of the calculated 1 mer-, 2mer- and 3mer-motif IFN-α scores to predict ssRNA oligonucleotides with a low or high IFN-α index. Thus for each ssRNA oligonucleotide the occurrence of a set of motifs was tested and the respective IFN-a scores were assigned to the ssRNA. For example for the panel of 3mer motifs with unspaced sequences (5'-XXX-3') the ssRNA oligonucleotide ANP 35 (5'-AAAAAAAAGUCAAAAAAAA-3') was analyzed the following way:

| 3mer motif (5'-XXX-3') | IFN-α score | Occurrences within the ssRNA oligonucleotide ANP 35 | Assigned IFN-α score |
|---|---|---|---|
| 5'-AAA-3' | - 0,0041 | 12 | - 0,0503 |
| 5'-AAG-3' | +0,0990 | 1 | +0,0990 |
| 5'-AGU-3' | +0,5796 | 1 | +0,5796 |
| 5'-GUC-3' | +0,8115 | 1 | +0,8115 |
| 5'-UCA-3' | +0,3668 | 1 | +0,3668 |
| 5'-CAA-3' | +0,0033 | 1 | +0,0033 |
| overall | | | +1,8099 |

All ssRNA oligonucleotides were assigned an individual IFN-a score for all possible motif-combinations (1 mer-, 2mer- and 3mer-motifs). Next, the prediction that was obtained by using the assigned IFN-α scores was compared to the actual adjusted IFN-α indices for all ssRNA oligonucleotides and for each motif combination. Data were sorted in ascending order according to the adjusted IFN-α indices. For all predictions, the correlation coefficient was calculated: Using the IFN-α scores of 1 mer motifs (5'-X-3') to predict the actual adjusted IFN-α indices off all ssRNA oligonucleotides a correlation coefficient of 0.53 was obtained (Figure 6A). When 2mer motifs were used to predict the adjusted IFN-α indices a correlation coefficient of 0.77 was obtained for 5'-XX-3'-motifs, a correlation coefficient of 0.58 for 5'-X*X-3'-motifs and a correlation coefficient of 0.60 for 5'-X**X-3'-motifs (Figure 6B). Using 3mer motifs to predict the adjusted IFN-α indices, a correlation coefficient of 0.87 was calculated for 5'-XXX-3'-motifs, of 0.80 for 5'-XX*X-3'-motifs and of 0.80 for 5'-X*XX-3'-motifs.

Next, the most accurate prediction algorithm (using the individual IFN-α scores of the 5'-XXX-3'-motifs) was translated into a point score system. The matrix of the 64 different 3mer motifs (5'-XXX-3') was reduced to encompass only the motifs that were significantly over- or underrepresented in either group 1 or group 2 ssRNA oligonucleotides. Even though only 17 3mer motifs (5'-XXX-3') were left in this matrix, a correlation coefficient of 0.87 could still be calculated when using the respective IFN-α scores to predict the IFN-α indices of the complete ssRNA oligonucleotide library (data not shown). In addition, the respective IFN-α scores of the reduced 3mer motif matrix was translated in into a point score system by assigning a point score to each 3mer motif (Figure 6D). Using this point score system, a prediction of the measured IFN-α indices of all 193 ssRNA oligonucleotides was calculated with a corresponding correlation coefficient of 0.87 (Figure 6E).

### Example 7. Influence of the positioning and the flanking bases on the IFN-α-inducing activity of a potent 4mer motif within a 19mer ssRNA oligonucleotide

Next we sought to address the impact of moving a potent 4mer motif to either the 5' or the 3' end within a 19mer ssRNA oligonucleotide. A panel of ssRNA oligonucleotides was designed that included the potent 4mer motif 5'-GUCA-3' within a 19mer poly-A ssRNA oligonucleotide (table 4). The positioning of the 4mer motif was chosen to be either 2, 6, 8, 10 or 14 bases from the 5'-end of the ssRNA oligonucleotide. PBMC were stimulated with the respective ssRNA oligonucleotides and IFN-α production was assessed 44 hours after stimulation. Positioning the 4mer motif two bases from the 5'-end of the ssRNA oligonucleotide slightly decreased IFN-α induction by 13%, when compared to the center positioning (8 bases from the 5'- end). A higher decrease in IFN-α production (29%) was seen, when the 4mer motif was positioned on the 3'-end of the ssRNA oligonucleotide (14 bases from the 5'-end). Nevertheless all ssRNA oligonucleotides tested were still more potent in terms of IFN-α induction than the positive control 9.2sense (Figure 7A).

In addition, we addressed the influence of flanking bases on the stimulatory activity of the potent 4mer motif 5'-GUCA-3'. A panel of 16 ssRNA oligonucleotides, which included all possible oligonucleotides with permutated bases at the flanking positions to the 5'- and the 3'-end of the central 5'-GUCA-3'-motif (table 5), was tested (Figure 7B). As soon as the 5'-GUCA-3'-motif was modified with a preceding 5'-C-3', a significant decrease to less than 50% of the original activity was obtained (Figure 7C). In addition, when a 5'-U-3' preceded the 5'-GUCA-3'-motif, a significant decrease to about 77% of the original activity was observed (Figure 7C). In contrast, changing the preceding base to a 5'-G-3' did not impact on the stimulatory activity of the 5'-GUCA-3'-motif. The modification of the flanking base to the 3'-end had little impact on the stimulatory activity of the 5'-GUCA-3'-motif. Nevertheless, a slight, yet significant decrease in activity was detected, as soon as the base to the 3'-end was changed into a 5'-C-3'.

### Example 8. Predicting the immunostimulatory activity of complex 19mer ssRNA oligonucleotides using the 3mer motif 5'-XXX-3' based IFN-α point score matrix.

Next, we employed the IFN-α point score matrix to predict the potency of complex (i.e., no longer on a poly A backbone) 19mer ssRNA oligonucleotides in terms of IFN-α induction. Modified versions of RNA9.2sense that have been previously described (Hornung et al. 2005, Nat Med 11:263-270) were used to stimulate PBMC; IFN-α production was measured 44 h after stimulation. In addition, respective sequences were analyzed using the above-described algorithm that is based on the IFN-α score of 3mer motifs 5'-XXX-3'. Both the measured and the predicted data were normalized to the ssRNA oligonucleotide RNA9.2sense (set to 100%). In Fig. 8A both measured data and predicted data are depicted. The correlation coefficient for this analysis was calculated to be 0.84.

### Example 9. Validation of the method of prediction

Our method of predicting the immunostimulatory activity of an RNA oligonucleotide is further validated by data disclosed in various publications. To date, four publications describe IFN-α induction by RNA oligonucleotides in the human system: Heil F et al. 2004, Science 303: 1526-1529; Sioud M et al. 2005, J Mol Biol 348: 1079-1090; Hornung V et al. 2005, Nat Med 11: 263-270; Judge AD et al. 2005, Nat Biotechnol 2005. 23: 457-462.

Heil and colleagues (Heil F et al. 2004, Science 303: 1526-1529) found that when added to human PBMC as a complex with cationic lipid DOTAP, RNA40 (5'-GCC CGU CUG UUG UGU GAC UC-3', HIV-1 U5 region nt 108-127) but not RNA41 (U-A replacement of RNA40) or RNA42 (G-A replacement of RNA40) induced IFN-α, and that the source of IFN-α was PDC. In contrast, both RNA40 and RNA42, but not RNA41 induced TNF-α, IL-6 and IL-12p40; TNF-α was produced by CD11 c+ cells. Similar results were found with isolated murine PDC and macrophages. RNA33 (5-GUAGUGUGUG-3') and RNA34 (5'-GUCUGUUGUGUG-3'), both containing one phosphorothioate linkage at the 3'end, induced the same cytokine profile as RNA40 in the mouse system. Heil et al. stated that a sequence motif responsible for the IFN-α inducing activity of the RNA olignucleotides tested could not be identified; subsequently, the activity was attributed to the high GU content of the sequence. Our analysis of the results of Heil et al. reveals that RNA40 contains a 4mer motif, GUUG, which is the fourth most potent motif in inducing IFN-α production in our matrix. Furthermore, our 3mer-based algorithm predicts high IFN-α-inducing activity for RNA40, RNA33 and RNA34, but not for RNA41 and RNA42, which is in agreement with the experimental data (Figure 8D).

Another publication on ssRNA and IFN-α in the human system is from our own group (Hornung V et al. 2005, Nat Med 11: 263-270). In this publication, we identified a 9mer sequence motif which was responsible for the immunostimulatory activity of the ssRNA oligonucleotide RNA9.2 sense (5'-AGC UUA ACC UGU CCU UCA A-3', 9mer motif underlined). No motif shorter than the 9mer motif was characterized. The analysis of our previously published results reveals that the previously identified 9mer motif contains GUCC which is the tenth most potent immunostimulatory 4mer in our matrix.

Furthermore, our 3mer-based IFN-α point score matrix predicts RNA9.2 sense to be a highly active IFN-α-inducing sequence. Moreover, our 3mer-based IFN-α point score matrix offers a prediction of the IFN-α-inducing activities of other sequences tested in Hornung et al.; our prediction correlates very well with the published experimental data.

Another study in the human system was published by Sioud and colleagues (Sioud M et al. 2005, J Mol Biol 348: 1079-1090). The authors examined a panel of 32 siRNAs for their ability to induce TNF-α and IL-6 in PBMC. The most active sequence was number 27 (sense: 5'-GUCCGGGCAGGUCUACUUUTT-3') either as siRNA (double-stranded) or as the sense strand. As negative control, number 32 (sense: 5'-GCUGGAGAUCCUGAAGAACTT-3') was used. Of note, the whole panel was not screened for IFN-α-inducing activity; only sequence 27 was assayed for IFN-α induction in PBMC. Both the number 27 siRNA and the corresponding sense strand were found to induce IFN-α. DOTAP was used for transfection. Our analysis of the panel of 32 siRNAs reveals that only oligonucleotide number 27, but none of the other oligonucleotides of the panel, contains the motif GUCC, which is ranked number 10 on our most potent immunostimulatory 4mer list and which is also contained in the 9mer motif of our earlier paper (Hornung V et al. 2005, Nat Med 11: 263-270) discussed above. Furthermore, our 3mer-based IFN-α point score matrix predicts potent IFN-α-inducing activity for siRNA number 27. However, since siRNA number 27 was the only sequence examined for IFN-α-inducing activity in Sioud et al, a comprehensive analysis of the whole panel of siRNAs could not be carried out.

Besides our own previous publication (Hornung V et al. 2005, Nat Med 11: 263-270), Judge and colleagues are the only ones who proposed a sequence motif (UGUGU) for the IFN-α-inducing activity of RNA oligonucleotides (Judge AD et al. 2005, Nat Biotechnol 2005. 23: 457-462). Although most of their work was done with siRNA (double-stranded), for one of their potent immunostimulatory sequences, β-Gal control, both the sense and the antisense strand were tested. The sense strand, but not the antisense strand, was found to be active to inducing IFN-α in human PBMC. The sense strand (5'-UUGAUGUGUUUAGUCGCUA-3') contained the proposed UGUGU motif, while the antisense strand (5'-UAGCGACUAAACACAUCAA-3') did not. The introduction of one (UGCGU) or two (UGCGC) mismatches in the sense strand sequence of the β-Gal control siRNA led to the loss of IFN-α-inducing activity. On the other hand, the creation of the UGUGU motif, starting from UGGCU, in a primarily non-stimulatory siRNA, BP1, led to an enhanced IFN-α-inducing activity. Furthermore, Judge and colleagues showed that they could select non-stimulatory siRNA sequences by avoiding U-rich sequences and GUGU motifs. Indeed, in our 4mer matrix, GUGU is the 7th most active motif, and UGUG is the 20th most active motif. Furthermore, the relative IFN-α-inducing activities of β-Gal control siRNA, BP1 siRNA and their derivatives predicated by our 3mer-based IFN-α point score matrix correlates extremely well with the experimental data of Judge et al. (Figure 8B).

Additional publication reports the induction of IFN-α by RNA oligonucleotides in the mouse system.

Barchet W et al. (2005, Eur J Immunol 35: 236-242) reports IFN-α induction by RNA oliognucleotides in murine PDC. In this study, the RNA sequences examined were derived from the 5' and 3' untranslated regions (UTR) of Influenza virus. The following sequences were used:
5' UTR: 5'-AGUAGAAACAAGGUAGUUU-3' (19mer)
3' UTR: 5'- UUAACUACCUGCUUUUGCU-3' (19mer)
5'3' UTR: 5'-AGUAGAAACAAGGUAGUUUUUUGUUAACUACCUGCUUUUGCU-3' (42mer),
5' UTR U-C replacement: 5'-AGCAGAAACAAGGCAGCCC-3' (19mer)
5' UTR G-C replacement: 5'-ACUACAAACAACCUACUUU-3' (19mer)
5'UTR, 3'UTR and 5'3'UTR oligonucledtides all induced IFN-α production from murine PDC. The activity of 5'UTR was significantly reduced when the Gs were replaced by Cs, and abolished when the Us were replaced by Cs. No motif responsible for the IFN-α-inducing activity was defined in this study. According to our 4mer motif matrix, 5'UTR contains the 5th active motif GUUU, and 3'UTR contains the motif UUUU which is above average. The activity levels of the oligonucleotides used in Barchet et al. predicted by our 3mer-based IFN-α point score matrix correlates with the experimental data of Barchet et al.

It should be noted that in some studies double stranded RNA oligonucleotides were used. In such cases, a mean value for the individually analyzed single strands was calculated. Nevertheless, using the prediction algorithm, a good estimate of the actual IFN-α data could be obtained.

All of the publications discussed above validate the use of our 4mer matrix and algorithms for predicting the immunostimulatory activity of RNA oligonucleotides. The teaching in the prior art with regard to the prediction of IFN-α-inducing activity of RNA oligonucleotides has been limited. The only criteria available so far are the content of G and U (Heil et al. 2004, Science 303: 1526-1529), and the presence of the GUGU motif (Judge et al. 2005, Nat Biotechnol 2005. 23: 457-462). With our 3mer-based IFN-α point score matrix, we now can predict the immunostimulatory activity of any RNA oligonucleotide reliably.

### Example 10. Determining the threshold for high and low immunostimulatory activity

The immunostimulatory activity of any given RNA oligonucleotide can be predicted using the 3mer-based IFN-α point score matrix as described previously. For research and drug discovery and development purposes, two groups of RNA oligonucleotide are of interest: Group A oligonucleotides which have high or maximal IFN-α-inducing activity, and Group B oligonucleotides which have low or minimal IFN-α-inducing activity. Among all possible ssRNA oligonucleotides of a certain length, 1% of the oligonucleotides with the highest IFN-α scores are assigned to Group A; where as 1% of the oligonucleotides with the lowest IFN-α scores are assigned to Group B. The cut-off IFN-α score for Group A oligonucleotide is the threshold for high or maximal immunostimulatory activity; the cut-off IFN-α score for Group B oligonucleotide is the threshold for low or minimal immunostimulatory activity.

The IFN-α score thresholds for high/maximal and low/minimal immunostimulatory activity for 19mer ssRNA oligonucleotides are determined as follows:

A pool of all possible sequences of 19mer RNA oligonucleotides consists of 4¹⁹=274,877,906,944 oligonucleotides. The IFN-α score for every single RNA oligonucleotide in the pool is calculated using the 3mer-based IFN-α point score matrix. All 4¹⁹ oligonucleotides are ranked based on their calculated predicted IFN-α scores. The threshold for group A is determined to be
1.4909 x n + 22.014
(n = length of the ssRNA oligonucleotide, and n >9).

All ssRNA oligonucleotides with a calculated IFN-α score above the threshold value are grouped into Group A. The Group A threshold for 19mer ssRNA oligonucleotides is 50.3411. Non-limiting examples of Group A 19mer ssRNA oligonucleotides include the following:

| Sequence (5' -->3') | Predicted IFN-α score |
|---|---|
| GUUUGUUGCUUUGAUUGCC | 60 |
| UUGUAGUUCGUUGCUAGUG | 60 |
| AGUUCAUGGUGGGUUGUAC | 62 |
| UGUUUAAGUUGUUCUACCC | 62 |
| AAGUUUUGAUUUUUCAGUA | 63 |
| AGGCGUUUGUGUUCGGGUU | 65 |
| AGAUGUUGUAGGGUGUUUU | 66 |
| UAGUGUGUGUCAGUGUGAC | 71 |
| GGUUGCGUGUGGAGUUGUU | 72 |
| UGUAGUUUUGUUAGAGUCA | 75 |
| GUGUGGUUGCUGUUGUCAA | 77 |

The threshold for Group B oligonucleotides is determined to be:
(0.005 x n²) - (0.2671 x n) - 3.5531
(n = length of the ssRNA oligonucleotide, and X > 9)

All ssRNA oligonucleotides with a calculated IFN-α score below the threshold value are grouped into Group B. The Group B threshold for 19mer ssRNA oligonucleotide is - 6.823. Non-limiting examples of Group B 19mer oligonucleotide include the following:

| Sequence (5' -->3') | Predicted IFN-α score |
|---|---|
| GGGACCGAAAGACCAGACC | -10 |
| UAAGACUAGAAGAGACAGA | -10 |
| AGAUCCGAACCACCGACCA | -9 |
| GAACCAGAAAAUAGAGCAG | -8 |
| CAUAUAAGAAGACCAGCCA | -8 |
| UAAGAACCAACUGCUAGAA | -8 |
| CCCCUACAGACAGAAUACC | -7 |
| CUGGCAGAUAGAUAGAAGC | -7 |
| CUAGACCAGAACAAUCUCG | -7 |
| UUAGAGACAUAACAACAUU | -7 |
| GGACCAAACCUCUCGACAU | -7 |

For ssRNA oligonucleotides between 3 and 9 nucleotides in length, the Group A and Group B threshold values are given below in Table 8:

**Table 8. Threshold IFN-α scores for Group A and Group B oligonucleotides 3-9 nucleotides in length.**

| ssRNA oligonucleotide length | The predicted IFN-α score (using the IFN-α point score matrix) | |
|---|---|---|
| | Threshold for GROUP B ssRNA oligonucelotides | Threshold for GROUP A ssRNA oligonucelotides |
| 3 | -2 | 9 |
| 4 | -2 | 15 |
| 5 | -3 | 20 |
| 6 | -4 | 23 |
| 7 | -4 | 26 |
| 8 | -5 | 28 |
| 9 | -5 | 30 |

### Example 11. Desigining siRNA with high or low immunostimulatory activity

The threshold for Group A siRNA is
1.4909xn+31.014
(n = length of the ssRNA oligonucleotide and n >9).

An siRNA is considered a Group A siRNA, i.e., an siRNA with high or maximal immunostimulatory activity, if at least one of the strands, preferably the sense strand, has an IFN-α score above the threshold.

In order to maximize the chance of identifying at least one siRNA with optimized gene silencing as well as immunostimulatory activity, at least ten siRNA molecules need to be identified whose sense strands have an IFN-α score above the threshold for Group A siRNA. When fewer than ten siRNA can be identified that fit the above criteria, the threshold for Group A siRNA is decrease by 1 in a stepwise manner until ten siRNA can be identified.

Most commonly used siRNA are at least 19 nucleotides in length. The threshold for Group A siRNA for a 19mer is 59.3411.

The following example demonstrates the identification of Group A siRNA for mRNA of human cyclophilin B (hCyPB) (Accession No. M6087).

For hCyPB, 833 putative or potential siRNA duplexes (19mer) can be identified. The IFN-α score is calculated for both the sense and the antisense strand of all possible 833 19mer siRNA using the IFN-α point score matrix. All siRNA duplexes which contain at least one strand with an IFN-α score higher than 59.3411 are put into Group A. 11 siRNA duplexes are assigend to Group A because the IFN-α scores of their sense strands are above the threshold; 24 siRNA duplexes are assigned to Group A because the IFN-α scores of their antisense strands are above the threshold. The Group A hCyPB siRNA are listed in Table 9:

| Sense strand | IFN-α | Antisene strand | IFN-α |
|---|---|---|---|
| (5'→ 3') | score | (5'→ 3') | score |
| UAACAAACUCCUACCAACA | -9 | UGUUGGUAGGAGUUUGUUA | 74 |
| AACAAACUCCUACCAACAC | -9 | GUGUUGGUAGGAGUUUGUU | 77 |
| UACCAACACUGACCAAUAA | -8 | UUAUUGGUCAGUGUUGGUA | 63 |
| CUACCAACACUGACCAAUA | -8 | UAUUGGUCAGUGUUGGUAG | 63 |
| ACCAACACUGACCAAUAAA | -8 | UUUAUUGGUCAGUGUUGGU | 65 |
| ACAAACUCCUACCAACACU | -8 | AGUGUUGGUAGGAGUUUGU | 74 |
| ACUCCUACCAACACUGACC | -7 | GGUCAGUGUUGGUAGGAGU | 62 |
| CCUACCAACACUGACCAAU | -7 | AUUGGUCAGUGUUGGUAGG | 63 |
| UCCUACCAACACUGACCAA | -7 | UUGGUCAGUGUUGGUAGGA | 63 |
| AAACUCCUACCAACACUGA | -6 | UCAGUGUUGGUAGGAGUUU | 64 |
| CCAACACUGACCAAUAAAA | -6 | UUUUAUUGGUCAGUGUUGG | 66 |
| CAAACUCCUACCAACACUG | -6 | CAGUGUUGGUAGGAGUUUG | 66 |
| AACUCCUACCAACACUGAC | -6 | GUCAGUGUUGGUAGGAGUU | 67 |
| AACACUGACCAAUAAAAAA | -6 | UUUUUUAUUGGUCAGUGUU | 69 |
| CAACACUGACCAAUAAAAA | -6 | UUUUUAUUGGUCAGUGUUG | 70 |
| GCUACAAAAACAGCAAAUU | -5 | AAUUUGCUGUUUUUGUAGC | 60 |
| GGCUACAAAAACAGCAAAU | -5 | AUUUGCUGUUUUUGUAGCC | 60 |
| ACACUGACCAAUAAAAAAA | -5 | UUUUUUUAUUGGUCAGUGU | 65 |
| UGGCUACAAAAACAGCAAA | -4 | UUUGCUGUUUUUGUAGCCA | 60 |
| GUAACAAACUCCUACCAAC | -4 | GUUGGUAGGAGUUUGUUAC | 66 |
| CACUGACCAAUAAAAAAAA | -3 | UUUUUUUUAUUGGUCAGUG | 62 |
| ACUGACCAAUAAAAAAAAA | -3 | UUUUUUUUUAUUGGUCAGU | 64 |
| UACAAAAACAGCAAAUUCC | -1 | GGAAUUUGCUGUUUUUGUA | 60 |
| CUACAAAAACAGCAAAUUC | -1 | GAAUUUGCUGUUUUUGUAG | 60 |
| AAAAUGUGGGUUUUUUUUU | 60 | AAAAAAAAACCCACAUUUU | 5 |
| UGUGGUGUUUGGCAAAGUU | 63 | AACUUUGCCAAACACCACA | 3 |
| AAAUGUGGGUUUUUUUUUU | 65 | AAAAAAAAAACCCACAUUU | 0 |
| GUUUUUUUUUUUUUUAAUA | 68 | UAUUAAAAAAAAAAAAAAC | -1 |
| AAUGUGGGUUUUUUUUUUU | 70 | AAAAAAAAAAACCCACAUU | -5 |
| GGUUUUUUUUUUUUUUAAU | 73 | AUUAAAAAAAAAAAAAACC | -3 |
| GGGUUUUUUUUUUUUUUAA | 73 | UUAAAAAAAAAAAAAACCC | -3 |
| UGGGUUUUUUUUUUUUUUA | 74 | UAAAAAAAAAAAAAACCCA | -3 |
| AUGUGGGUUUUUUUUUUUU | 75 | AAAAAAAAAAAACCCACAU | -5 |
| GUGGGUUUUUUUUUUUUUU | 77 | AAAAAAAAAAAAAACCCAC | -3 |
| UGUGGGUUUUUUUUUUUUU | 80 | AAAAAAAAAAAAACCCACA | -5 |

The IFN-α score threshold for a Group B siRNA is
0.6075 x n - 9.9484
(n = length of the ssRNA oligonucleotide and n >13).

Both the sense and the antisense strands of an siRNA have to have an IFN-α score below the threshold for the siRNA to be assigned to Group B. In order to maximize the chance of identifying at least one siRNA with optimal gene silencing activity but minimal immunostimulatory activity, at least ten Group B siRNA duplexes should be identified for a given target mRNA. Should this condition not be met, the threshold IFN-α score is increased by 1 in a stepwise manner until ten Group B siRNA are identified.

The IFN-α score threshold for Group B 19mer siRNA duplexes is 1.5491.

None of the 833 potential hCyPB siRNA have an IFN-α score of lower than 1.5491 for both strands. When the threshold was increased to 2.5491, one siRNA is identified. In order to identify at least ten siRNA duplexes, the threshold IFN-α score has to be increased by 3 to 4.5491. The Group B siRNA thus identified are listed in Table 9:

| Sense strand | IFN-α | Antisense strand | IFN-α |
|---|---|---|---|
| (5'→ 3') | score | (5'→ 3') | score |
| AAGAUCGAGGUGGAGAAGC | 4 | GCUUCUCCACCUCGAUCUU | 2 |
| AGAUCGAGGUGGAGAAGCC | 4 | GGCUUCUCCACCUCGAUCU | 2 |
| CCGCCGCCCUCAUCGCGGG | 4 | CCCGCGAUGAGGGCGGCGG | 0 |
| CCUUCUGCGGCCGAUGAGA | 2 | UCUCAUCGGCCGCAGAAGG | 2 |
| CGCCGCCCUCAUCGCGGGG | 4 | CCCCGCGAUGAGGGCGGCG | 0 |
| CUUCCUGCUGCUGCCGGGA | 4 | UCCCGGCAGCAGCAGGAAG | 0 |
| GAGCGCUUCCCCGAUGAGA | 2 | UCUCAUCGGGGAAGCGCUC | 4 |
| GCCGCCGCCCUCAUCGCGG | 4 | CCGCGAUGAGGGCGGCGGC | 0 |
| GGCAAGAUCGAGGUGGAGA | 4 | UCUCCACCUCGAUCUUGCC | 4 |
| UCUUCCUGCUGCUGCCGGG | 4 | CCCGGCAGCAGCAGGAAGA | -2 |
| UGCCGCCGCCCUCAUCGCG | 4 | CGCGAUGAGGGCGGCGGCA | 0 |

**Table1: ssRNA oligonucleotides containing all possible 4mer motifs on the poly a backbone.**

| Name | Sequence |
|---|---|
| ANP-Oligo 001 | AAAAAAAAAAAAAAAAAAA |
| ANP-Oligo 002 | AAAAAAAACAAAAAAAAAA |
| ANP-Oligo 003 | AAAAAAAAGAAAAAAAAAA |
| ANP-Oligo 004 | AAAAAAAAUAAAAAAAAAA |
| ANP-Oligo 005 | AAAAAAAACCAAAAAAAAA |
| ANP-Oligo 006 | AAAAAAAACGAAAAAAAAA |
| ANP-Oligo 007 | AAAAAAAACUAAAAAAAAA |
| ANP-Oligo 008 | AAAAAAAAGCAAAAAAAAA |
| ANP-Oligo 009 | AAAAAAAAGGAAAAAAAAA |
| ANP-Oligo 010 | AAAAAAAAGUAAAAAAAAA |
| ANP-Oligo 011 | AAAAAAAAUCAAAAAAAAA |
| ANP-Oligo 012 | AAAAAAAAUGAAAAAAAAA |
| ANP-Oligo 013 | AAAAAAAAUUAAAAAAAAA |
| ANP-Oligo 014 | AAAAAAAACACAAAAAAAA |
| ANP-Oligo 015 | AAAAAAAACAGAAAAAAAA |
| ANP-Oligo 016 | AAAAAAAACAUAAAAAAAA |
| ANP-Oligo 017 | AAAAAAAACCCAAAAAAAA |
| ANP-Oligo 018 | AAAAAAAACCGAAAAAAAA |
| ANP-Oligo 019 | AAAAAAAACCUAAAAAAAA |
| ANP-Oligo 020 | AAAAAAAACGCAAAAAAAA |
| ANP-Oligo 021 | AAAAAAAACGGAAAAAAAA |
| ANP-Oligo 022 | AAAAAAAACGUAAAAAAAA |
| ANP-Oligo 023 | AAAAAAAACUCAAAAAAAA |
| ANP-Oligo 024 | AAAAAAAACUGAAAAAAAA |
| ANP-Oligo 025 | AAAAAAAACUUAAAAAAAA |
| ANP-Oligo 026 | AAAAAAAAGACAAAAAAAA |
| ANP-Oligo 027 | AAAAAAAAGAGAAAAAAAA |
| ANP-Oligo 028 | AAAAAAAAGAUAAAAAAAA |
| ANP-Oligo 029 | AAAAAAAAGCCAAAAAAAA |
| ANP-Oligo 030 | AAAAAAAAGCGAAAAAAAA |
| ANP-Oligo 031 | AAAAAAAAGCUAAAAAAAA |
| ANP-Oligo 032 | AAAAAAAAGGCAAAAAAAA |
| ANP-Oligo 033 | AAAAAAAAGGGAAAAAAAA |
| ANP-Oligo 034 | AAAAAAAAGGUAAAAAAAA |
| ANP-Oligo 035 | AAAAAAAAGUCAAAAAAAA |
| ANP-Oligo 036 | AAAAAAAAGUGAAAAAAAA |
| ANP-Oligo 037 | AAAAAAAAGUUAAAAAAAA |
| AN P-Oligo 038 | AAAAAAAAUACAAAAAAAA |
| ANP-Oligo 039 | AAAAAAAAUAGAAAAAAAA |
| ANP-Oligo 040 | AAAAAAAAUAUAAAAAAAA |
| ANP-Oligo 041 | AAAAAAAAUCCAAAAAAAA |
| ANP-Oligo 042 | AAAAAAAAUCGAAAAAAAA |
| ANP-Oligo 043 | AAAAAAAAUCUAAAAAAAA |
| ANP-Oligo 044 | AAAAAAAAUGCAAAAAAAA |
| ANP-Oligo 045 | AAAAAAAAUGGAAAAAAAA |
| ANP-Oligo 046 | AAAAAAAAUGUAAAAAAAA |
| ANP-Oligo 047 | AAAAAAAAUUCAAAAAAAA |
| ANP-Oligo 048 | AAAAAAAAUUGAAAAAAAA |
| ANP-Oligo 049 | AAAAAAAAUUUAAAAAAAA |
| ANP-Oligo 050 | AAAAAAACAACAAAAAAAA |
| ANP-Oligo 051 | AAAAAAACAAGAAAAAAAA |
| ANP-Oligo 052 | AAAAAAACAAUAAAAAAAA |
| ANP-Oligo 053 | AAAAAAACACCAAAAAAAA |
| ANP-Oligo 054 | AAAAAAACACGAAAAAAAA |
| ANP-Oligo 055 | AAAAAAACACUAAAAAAAA |
| ANP-Oligo 056 | AAAAAAACAGCAAAAAAAA |
| ANP-Oligo 057 | AAAAAAACAGGAAAAAAAA |
| ANP-Oligo 058 | AAAAAAACAGUAAAAAAAA |
| ANP-Oligo 059 | AAAAAAACAUCAAAAAAAA |
| ANP-Oligo 060 | AAAAAAACAUGAAAAAAAA |
| ANP-Oligo 061 | AAAAAAACAUUAAAAAAAA |
| ANP-Oligo 062 | AAAAAAACCACAAAAAAAA |
| ANP-Oligo 063 | AAAAAAACCAGAAAAAAAA |
| ANP-Oligo 064 | AAAAAAACCAUAAAAAAAA |
| ANP-Oligo 065 | AAAAAAACCCCAAAAAAAA |
| ANP-Oligo 066 | AAAAAAACCCGAAAAAAAA |
| ANP-Oligo 067 | AAAAAAACCCUAAAAAAAA |
| ANP-Oligo 068 | AAAAAAACCGCAAAAAAAA |
| ANP-Oligo 069 | AAAAAAACCGGAAAAAAAA |
| ANP-Oligo 070 | AAAAAAACCGUAAAAAAAA |
| ANP-Oligo 071 | AAAAAAACCUCAAAAAAAA |
| ANP-Oligo 072 | AAAAAAACCUGAAAAAAAA |
| ANP-Oligo 073 | AAAAAAACCUUAAAAAAAA |
| ANP-Oligo 074 | AAAAAAACGACAAAAAAAA |
| ANP-Oligo 075 | AAAAAAACGAGAAAAAAAA |
| ANP-Oligo 076 | AAAAAAACGAUAAAAAAAA |
| ANP-Oligo 077 | AAAAAAACGCCAAAAAAAA |
| ANP-Oligo 078 | AAAAAAACGCGAAAAAAAA |
| ANP-Oligo 079 | AAAAAAACGCUAAAAAAAA |
| ANP-Oligo 080 | AAAAAAACGGCAAAAAAAA |
| ANP-Oligo 081 | AAAAAAACGGGAAAAAAAA |
| ANP-Oligo 082 | AAAAAAACGGUAAAAAAAA |
| ANP-Oligo 083 | AAAAAAACGUCAAAAAAAA |
| ANP-Oligo 084 | AAAAAAACGUGAAAAAAAA |
| ANP-Oligo 085 | AAAAAAACGUUAAAAAAAA |
| ANP-Oligo 086 | AAAAAAACUACAAAAAAAA |
| ANP-Oligo 087 | AAAAAAACUAGAAAAAAAA |
| ANP-Oligo 088 | AAAAAAACUAUAAAAAAAA |
| ANP-Oligo 089 | AAAAAAACUCCAAAAAAAA |
| ANP-Oligo 090 | AAAAAAACUCGAAAAAAAA |
| ANP-Oligo 091 | AAAAAAACUCUAAAAAAAA |
| ANP-Oligo 092 | AAAAAAACUGCAAAAAAAA |
| ANP-Oligo 093 | AAAAAAACUGGAAAAAAAA |
| ANP-Oligo 094 | AAAAAAACUGUAAAAAAAA |
| ANP-Oligo 095 | AAAAAAACUUCAAAAAAAA |
| ANP-Oligo 096 | AAAAAAACUUGAAAAAAAA |
| ANP-Oligo 097 | AAAAAAACUUUAAAAAAAA |
| ANP-Oligo 098 | AAAAAAAGAACAAAAAAAA |
| ANP-Oligo 099 | AAAAAAAGAAGAAAAAAAA |
| ANP-Oligo 100 | AAAAAAAGAAUAAAAAAAA |
| ANP-Oligo 101 | AAAAAAAGACCAAAAAAAA |
| ANP-Oligo 102 | AAAAAAAGACGAAAAAAAA |
| ANP-Oligo 103 | AAAAAAAGACUAAAAAAAA |
| ANP-Oligo 104 | AAAAAAAGAGCAAAAAAAA |
| ANP-Oligo 105 | AAAAAAAGAGGAAAAAAAA |
| ANP-Oligo 106 | AAAAAAAGAGUAAAAAAAA |
| ANP-Oligo 107 | AAAAAAAGAUCAAAAAAAA |
| ANP-Oligo 108 | AAAAAAAGAUGAAAAAAAA |
| ANP-Oligo 109 | AAAAAAAGAUUAAAAAAAA |
| ANP-Oligo 110 | AAAAAAAGCACAAAAAAAA |
| AN P-Oligo 111 | AAAAAAAGCAGAAAAAAAA |
| ANP-Oligo 112 | AAAAAAAGCAUAAAAAAAA |
| ANP-Oligo 113 | AAAAAAAGCCCAAAAAAAA |
| ANP-Oligo 114 | AAAAAAAGCCGAAAAAAAA |
| ANP-Oligo 115 | AAAAAAAGCCUAAAAAAAA |
| ANP-Oligo 116 | AAAAAAAGCGCAAAAAAAA |
| ANP-Oligo 117 | AAAAAAAGCGGAAAAAAAA |
| ANP-Oligo 118 | AAAAAAAGCGUAAAAAAAA |
| ANP-Oligo 119 | AAAAAAAGCUCAAAAAAAA |
| ANP-Oligo 120 | AAAAAAAGCUGAAAAAAAA |
| ANP-Oligo 121 | AAAAAAAGCUUAAAAAAAA |
| ANP-Oligo 122 | AAAAAAAGGACAAAAAAAA |
| ANP-Oligo 123 | AAAAAAAGGAGAAAAAAAA |
| ANP-Oligo 124 | AAAAAAAGGAUAAAAAAAA |
| ANP-Oligo 125 | AAAAAAAGGCCAAAAAAAA |
| ANP-Oligo 126 | AAAAAAAGGCGAAAAAAAA |
| ANP-Oligo 127 | AAAAAAAGGCUAAAAAAAA |
| ANP-Oligo 128 | AAAAAAAGGGCAAAAAAAA |
| ANP-Oligo 129 | AAAAAAAGGGGAAAAAAAA |
| ANP-Oligo 130 | AAAAAAAGGGUAAAAAAAA |
| ANP-Oligo 131 | AAAAAAAGGUCAAAAAAAA |
| ANP-Oligo 132 | AAAAAAAGGUGAAAAAAAA |
| ANP-Oligo 133 | AAAAAAAGGUUAAAAAAAA |
| ANP-Oligo 134 | AAAAAAAGUACAAAAAAAA |
| ANP-Oligo 135 | AAAAAAAGUAGAAAAAAAA |
| ANP-Oligo 136 | AAAAAAAGUAUAAAAAAAA |
| ANP-Oligo 137 | AAAAAAAGUCCAAAAAAAA |
| ANP-Oligo 138 | AAAAAAAGUCGAAAAAAAA |
| ANP-Oligo 139 | AAAAAAAGUCUAAAAAAAA |
| ANP-Oligo 140 | AAAAAAAGUGCAAAAAAAA |
| ANP-Oligo 141 | AAAAAAAGUGGAAAAAAAA |
| ANP-Oligo 142 | AAAAAAAGUGUAAAAAAAA |
| ANP-Oligo 143 | AAAAAAAGUUCAAAAAAAA |
| ANP-Oligo 144 | AAAAAAAGUUGAAAAAAAA |
| ANP-Oligo 145 | AAAAAAAGUUUAAAAAAAA |
| ANP-Oligo 146 | AAAAAAAUAACAAAAAAAA |
| ANP-Oligo 147 | AAAAAAAUAAGAAAAAAAA |
| ANP-Oligo 148 | AAAAAAAUAAUAAAAAAAA |
| ANP-Oligo 149 | AAAAAAAUACCAAAAAAAA |
| ANP-Oligo 150 | AAAAAAAUACGAAAAAAAA |
| ANP-Oligo 151 | AAAAAAAUACUAAAAAAAA |
| ANP-Oligo 152 | AAAAAAAUAGCAAAAAAAA |
| ANP-Oligo 153 | AAAAAAAUAGGAAAAAAAA |
| ANP-Oligo 154 | AAAAAAAUAGUAAAAAAAA |
| ANP-Oligo 155 | AAAAAAAUAUCAAAAAAAA |
| ANP-Oligo 156 | AAAAAAAUAUGAAAAAAAA |
| ANP-Oligo 157 | AAAAAAAUAUUAAAAAAAA |
| ANP-Oligo 158 | AAAAAAAUCACAAAAAAAA |
| ANP-Oligo 159 | AAAAAAAUCAGAAAAAAAA |
| ANP-Oligo 160 | AAAAAAAUCAUAAAAAAAA |
| ANP-Oligo 161 | AAAAAAAUCCCAAAAAAAA |
| ANP-Oligo 162 | AAAAAAAUCCGAAAAAAAA |
| ANP-Oligo 163 | AAAAAAAUCCUAAAAAAAA |
| ANP-Oligo 164 | AAAAAAAUCGCAAAAAAAA |
| ANP-Oligo 165 | AAAAAAAUCGGAAAAAAAA |
| ANP-Oligo 166 | AAAAAAAUCGUAAAAAAAA |
| ANP-Oligo 167 | AAAAAAAUCUCAAAAAAAA |
| ANP-Oligo 168 | AAAAAAAUCUGAAAAAAAA |
| ANP-Oligo 169 | AAAAAAAUCUUAAAAAAAA |
| ANP-Oligo 170 | AAAAAAAUGACAAAAAAAA |
| ANP-Oligo 171 | AAAAAAAUGAGAAAAAAAA |
| ANP-Oligo 172 | AAAAAAAUGAUAAAAAAAA |
| ANP-Oligo 173 | AAAAAAAUGCCAAAAAAAA |
| ANP-Oligo 174 | AAAAAAAUGCGAAAAAAAA |
| ANP-Oligo 175 | AAAAAAAUGCUAAAAAAAA |
| ANP-Oligo 176 | AAAAAAAUGGCAAAAAAAA |
| ANP-Oligo 177 | AAAAAAAUGGGAAAAAAAA |
| ANP-Oligo 178 | AAAAAAAUGGUAAAAAAAA |
| ANP-Oligo 179 | AAAAAAAUGUCAAAAAAAA |
| ANP-Oligo 180 | AAAAAAAUGUGAAAAAAAA |
| ANP-Oligo 181 | AAAAAAAUGUUAAAAAAAA |
| ANP-Oligo 182 | AAAAAAAUUACAAAAAAAA |
| ANP-Oligo 183 | AAAAAAAUUAGAAAAAAAA |
| ANP-Oligo 184 | AAAAAAAUUAUAAAAAAAA |
| ANP-Oligo 185 | AAAAAAAUUCCAAAAAAAA |
| ANP-Oligo 186 | AAAAAAAUUCGAAAAAAAA |
| ANP-Oligo 187 | AAAAAAAUUCUAAAAAAAA |
| ANP-Oligo 188 | AAAAAAAUUGCAAAAAAAA |
| ANP-Oligo 189 | AAAAAAAUUGGAAAAAAAA |
| ANP-Oligo 190 | AAAAAAAUUGUAAAAAAAA |
| ANP-Oligo 191 | AAAAAAAUUUCAAAAAAAA |
| ANP-Oligo 192 | AAAAAAAUUUGAAAAAAAA |
| ANP-Oligo 193 | AAAAAAAUUUUAAAAAAAA |

**Table 2: Group 1 ssRNA-Oligonucleotides**

| Name | Sequence 5' → 3' | adjusted | SEM |
|---|---|---|---|
| | | IFN-α index | |
| ANP-Oligo 018 | AAAAAAAACCGAAAAAAAA | -0,23 | 0,03 |
| ANP-Oligo 020 | AAAAAAAACGCAAAAAAAA | -0,23 | 0,03 |
| ANP-Oligo 029 | AAAAAAAAGCCAAAAAAAA | -0,23 | 0,03 |
| ANP-Oligo 051 | AAAAAAACAAGAAAAAAAA | -0,23 | 0,03 |
| ANP-Oligo 053 | AAAAAAACACCAAAAAAAA | -0,23 | 0,03 |
| ANP-Oligo 075 | AAAAAAACGAGAAAAAAAA | -0,23 | 0,03 |
| ANP-Oligo 080 | AAAAAAACGGCAAAAAAAA | -0,23 | 0,03 |
| ANP-Oligo 128 | AAAAAAAGGGCAAAAAAAA | -0,23 | 0,03 |
| ANP-Oligo 130 | AAAAAAAGGGUAAAAAAAA | -0,23 | 0,03 |
| ANP-Oligo 009 | AAAAAAAAGGAAAAAAAAA | -0,23 | 0,03 |
| ANP-Oligo 017 | AAAAAAAACCCAAAAAAAA | -0,23 | 0,03 |
| ANP-Oligo 003 | AAAAAAAAGAAAAAAAAAA | -0,23 | 0,03 |
| ANP-Oligo 081 | AAAAAAACGGGAAAAAAAA | -0,23 | 0,03 |
| ANP-Oligo 113 | AAAAAAAGCCCAAAAAAAA | -0,23 | 0,03 |
| ANP-Oligo 014 | AAAAAAAACACAAAAAAAA | -0,23 | 0,03 |
| ANP-Oligo 077 | AAAAAAACGCCAAAAAAAA | -0,23 | 0,03 |
| ANP-Oligo 126 | AAAAAAAGGCGAAAAAAAA | -0,23 | 0,03 |
| ANP-Oligo 125 | AAAAAAAGGCCAAAAAAAA | -0,23 | 0,03 |
| ANP-Oligo 050 | AAAAAAACAACAAAAAAAA | -0,23 | 0,03 |
| ANP-Oligo 015 | AAAAAAAACAGAAAAAAAA | -0,22 | 0,03 |
| ANP-Oligo 114 | AAAAAAAGCCGAAAAAAAA | -0,22 | 0,03 |
| ANP-Oligo 027 | AAAAAAAAGAGAAAAAAAA | -0,22 | 0,03 |
| ANP-Oligo 117 | AAAAAAAGCGGAAAAAAAA | -0,22 | 0,03 |
| ANP-Oligo 006 | AAAAAAAACGAAAAAAAAA | -0,22 | 0,03 |
| ANP-Oligo 005 | AAAAAAAACCAAAAAAAAA | -0,22 | 0,03 |
| ANP-Oligo 069 | AAAAAAACCGGAAAAAAAA | -0,22 | 0,03 |
| ANP-Oligo 066 | AAAAAAACCCGAAAAAAAA | -0,22 | 0,03 |
| ANP-Oligo 007 | AAAAAAAACUAAAAAAAAA | -0,22 | 0,03 |
| ANP-Oligo 060 | AAAAAAACAUGAAAAAAAA | -0,22 | 0,03 |
| ANP-Oligo 021 | AAAAAAAACGGAAAAAAAA | -0,22 | 0,03 |
| ANP-Oligo 042 | AAAAAAAAUCGAAAAAAAA | -0,22 | 0,03 |
| ANP-Oligo 086 | AAAAAAACUACAAAAAAAA | -0,22 | 0,03 |
| ANP-Oligo 064 | AAAAAAACCAUAAAAAAAA | -0,22 | 0,03 |
| ANP-Oligo 102 | AAAAAAAGACGAAAAAAAA | -0,22 | 0,03 |
| ANP-Oligo 068 | AAAAAAACCGCAAAAAAAA | -0,22 | 0,03 |
| ANP-Oligo 129 | AAAAAAAGGGGAAAAAAAA | -0,22 | 0,03 |
| ANP-Oligo 008 | AAAAAAAAGCAAAAAAAAA | -0,22 | 0,03 |
| ANP-Oligo 002 | AAAAAAAACAAAAAAAAAA | -0,22 | 0,03 |
| ANP-Oligo 038 | AAAAAAAAUACAAAAAAAA | -0,22 | 0,03 |
| ANP-Oligo 026 | AAAAAAAAGACAAAAAAAA | -0,22 | 0,03 |
| ANP-Oligo 116 | AAAAAAAGCGCAAAAAAAA | -0,22 | 0,03 |
| ANP-Oligo 054 | AAAAAAACACGAAAAAAAA | -0,22 | 0,03 |
| ANP-Oligo 057 | AAAAAAACAGGAAAAAAAA | -0,22 | 0,03 |
| ANP-Oligo 001 | AAAAAAAAAAAAAAAAAAA | -0,21 | 0,03 |
| ANP-Oligo 074 | AAAAAAACGACAAAAAAAA | -0,21 | 0,03 |
| ANP-Oligo 033 | AAAAAAAAGGGAAAAAAAA | -0,21 | 0,03 |
| ANP-Oligo 123 | AAAAAAAGGAGAAAAAAAA | -0,21 | 0,03 |
| ANP-Oligo 059 | AAAAAAACAUCAAAAAAAA | -0,21 | 0,03 |
| ANP-Oligo 110 | AAAAAAAGCACAAAAAAAA | -0,21 | 0,03 |
| ANP-Oligo 065 | AAAAAAACCCCAAAAAAAA | -0,21 | 0,03 |
| ANP-Oligo 055 | AAAAAAACACUAAAAAAAA | -0,21 | 0,03 |
| ANP-Oligo 122 | AAAAAAAGGACAAAAAAAA | -0,21 | 0,03 |
| ANP-Oligo 104 | AAAAAAAGAGCAAAAAAAA | -0,21 | 0,03 |
| ANP-Oligo 078 | AAAAAAACGCGAAAAAAAA | -0,21 | 0,03 |
| ANP-Oligo 032 | AAAAAAAAGGCAAAAAAAA | -0,21 | 0,04 |
| ANP-Oligo 030 | AAAAAAAAGCGAAAAAAAA | -0,21 | 0,03 |
| ANP-Oligo 004 | AAAAAAAAUAAAAAAAAAA | -0,21 | 0,03 |
| ANP-Oligo 105 | AAAAAAAGAGGAAAAAAAA | -0,21 | 0,03 |
| ANP-Oligo 161 | AAAAAAAUCCCAAAAAAAA | -0,21 | 0,02 |
| ANP-Oligo 061 | AAAAAAACAUUAAAAAAAA | -0,2 | 0,02 |
| ANP-Oligo 041 | AAAAAAAAUCCAAAAAAAA | -0,2 | 0,03 |
| ANP-Oligo 063 | AAAAAAACCAGAAAAAAAA | -0,2 | 0,04 |
| ANP-Oligo 072 | AAAAAAACCUGAAAAAAAA | -0,2 | 0,02 |
| ANP-Oligo 101 | AAAAAAAGACCAAAAAAAA | -0,2 | 0,04 |
| ANP-Oligo 056 | AAAAAAACAGCAAAAAAAA | -0,2 | 0,04 |
| ANP-Oligo 162 | AAAAAAAUCCGAAAAAAAA | -0,2 | 0,02 |
| ANP-Oligo 099 | AAAAAAAGAAGAAAAAAAA | -0,2 | 0,05 |
| ANP-Oligo 111 | AAAAAAAGCAGAAAAAAAA | -0,2 | 0,04 |
| ANP-Oligo 011 | AAAAAAAAUCAAAAAAAAA | -0,2 | 0,04 |
| ANP-Oligo 062 | AAAAAAACCACAAAAAAAA | -0,19 | 0,04 |
| ANP-Oligo 016 | AAAAAAAACAUAAAAAAAA | -0,19 | 0,03 |
| ANP-Oligo 177 | AAAAAAAUGGGAAAAAAAA | -0,19 | 0,03 |
| ANP-Oligo 019 | AAAAAAAACCUAAAAAAAA | -0,19 | 0,03 |
| ANP-Oligo 098 | AAAAAAAGAACAAAAAAAA | -0,19 | 0,05 |
| ANP-Oligo 052 | AAAAAAACAAUAAAAAAAA | -0,19 | 0,02 |
| ANP-Oligo 089 | AAAAAAACUCCAAAAAAAA | -0,18 | 0,05 |
| ANP-Oligo 067 | AAAAAAACCCUAAAAAAAA | -0,18 | 0,02 |
| ANP-Oligo 040 | AAAAAAAAUAUAAAAAAAA | -0,18 | 0,02 |
| ANP-Oligo 039 | AAAAAAAAUAGAAAAAAAA | -0,18 | 0,02 |
| ANP-Oligo 043 | AAAAAAAAUCUA,AAAAAAA | -0,18 | 0,02 |
| ANP-Oligo 073 | AAAAAAACCUUAAAAAAAA | -0,18 | 0,01 |
| ANP-Oligo 024 | AAAAAAAACUGAAAAAAAA | -0,17 | 0,02 |
| ANP-Oligo 028 | AAAAAAAAGAUAAAAAAAA | -0,17 | 0,01 |
| ANP-Oligo 103 | AAAAAAAGACUAAAAAAAA | -0,17 | 0,01 |
| ANP-Oligo 091 | AAAAAAACUCUAAAAAAAA | -0,17 | 0,01 |
| ANP-Oligo 158 | AAAAAAAUCACAAAAAAAA | -0,17 | 0,02 |
| ANP-Oligo 012 | AAAAAAAAUGAAAAAAAAA | -0,16 | 0,02 |
| ANP-Oligo 163 | AAAAAAAUCCUAAAAAAAA | -0,15 | 0,03 |
| ANP-Oligo 025 | AAAAAAAACUUAAAAAAAA | -0,15 | 0,02 |
| ANP-Oligo 112 | AAAAAAAGCAUAAAAAAAA | -0,15 | 0,01 |
| ANP-Oligo 070 | AAAAAAACCGUAAAAAAAA | -0,14 | 0,02 |
| ANP-Oligo 172 | AAAAAAAUGAUAAAAAAAA | -0,14 | 0,03 |
| ANP-Oligo 150 | AAAAAAAUACGAAAAAAAA | -0,14 | 0,04 |
| ANP-Oligo 146 | AAAAAAAUAACAAAAAAAA | -0,14 | 0,03 |
| ANP-Oligo 115 | AAAAAAAGCCUAAAAAAAA | -0,14 | 0,01 |
| ANP-Oligo 160 | AAAAAAAUCAUAAAAAAAA | -0,13 | 0,02 |
| ANP-Oligo 151 | AAAAAAAUACUAAAAAAAA | -0,13 | 0,03 |
| ANP-Oligo 013 | AAAAAAAAUUAAAAAAAAA | -0,13 | 0,03 |
| ANP-Oligo 090 | AAAAAAACUCGAAAAAAAA | -0,13 | 0,03 |
| ANP-Oligo 048 | AAAAAAAAUUGAAAAAAAA | -0,13 | 0,02 |
| ANP-Oligo 092 | AAAAAAACUGCAAAAAAAA | -0,13 | 0,02 |
| ANP-Oligo 093 | AAAAAAACUGGAAAAAAAA | -0,13 | 0,03 |
| ANP-Oligo 031 | AAAAAAAAGCUAAAAAAAA | -0,13 | 0,02 |
| ANP-Oligo 157 | AAAAAAAUAUUAAAAAAAA | -0,12 | 0,02 |
| ANP-Oligo 108 | AAAAAAAGAUGAAAAAAAA | -0,12 | 0,02 |
| ANP-Oligo 076 | AAAAAAACGAUAAAAAAAA | -0,12 | 0,03 |
| ANP-Oligo 165 | AAAAAAAUCGGAAAAAAAA | -0,11 | 0,03 |
| ANP-Oligo 087 | AAAAAAACUAGAAAAAAAA | -0,11 | 0,01 |
| ANP-Oligo 109 | AAAAAAAGAUUAAAAAAAA | -0,11 | 0,01 |
| ANP-Oligo 183 | AAAAAAAUUAGAAAAAAAA | -0,11 | 0,05 |
| ANP-Oligo 127 | AAAAAAAGGCUAAAAAAAA | -0,11 | 0,03 |
| ANP-Oligo 079 | AAAAAAACGCUAAAAAAAA | -0,1 | 0,02 |
| ANP-Oligo 088 | AAAAAAACUAUAAAAAAAA | -0,1 | 0,03 |
| ANP-Oligo 159 | AAAAAAAUCAGAAAAAAAA | -0,1 | 0,05 |
| ANP-Oligo 152 | AAAAAAAUAGCAAAAAAAA | -0,1 | 0,04 |
| ANP-Oligo 084 | AAAAAAACGUGAAAAAAAA | -0,1 | 0,03 |
| ANP-Oligo 184 | AAAAAAAUUAUAAAAAAAA | -0,1 | 0,03 |
| ANP-Oligo 175 | AAAAAAAUGCUAAAAAAAA | -0,1 | 0,03 |
| ANP-Oligo 164 | AAAAAAAUCGCAAAAAAAA | -0,09 | 0,04 |
| ANP-Oligo 149 | AAAAAAAUACCAAAAAAAA | -0,09 | 0,06 |
| ANP-Oligo 185 | AAAAAAAUUCCAAAAAAAA | -0,08 | 0,05 |
| ANP-Oligo 044 | AAAAAAAAUGCAAAAAAAA | -0,08 | 0,03 |
| ANP-Oligo 022 | AAAAAAAACGUAAAAAAAA | -0,08 | 0,02 |
| ANP-Oligo 049 | AAAAAAAAUUUAAAAAAAA | -0,08 | 0,02 |
| ANP-Oligo 147 | AAAAAAAUAAGAAAAAAAA | -0,07 | 0,06 |
| ANP-Oligo 120 | AAAAAAAGCUGAAAAAAAA | -0,07 | 0,03 |
| ANP-Oligo 107 | AAAAAAAGAUCAAAAAAAA | -0,07 | 0,05 |
| ANP-Oligo 118 | AAAAAAAGCGUAAAAAAAA | -0,07 | 0,03 |
| ANP-Oligo 170 | AAAAAAAUGACAAAAAAAA | -0,07 | 0,06 |
| ANP-Oligo 124 | AAAAAAAGGAUAAAAAAAA | -0,07 | 0,05 |
| ANP-Oligo 148 | AAAAAAAUAAUAAAAAAAA | -0,07 | 0,05 |
| ANP-Oligo 097 | AAAAAAACUUUAAAAAAAA | -0,07 | 0,02 |
| ANP-Oligo 156 | AAAAAAAUAUGAAAAAAAA | -0,06 | 0,04 |
| ANP-Oligo 045 | AAAAAAAAUGGAAAAAAAA | -0,06 | 0,06 |
| ANP-Oligo 171 | AAAAAAAUGAGAAAAAAAA | -0,05 | 0,08 |
| ANP-Oligo 173 | AAAAAAAUGCCAAAAAAAA | -0,05 | 0,05 |
| ANP-Oligo 174 | AAAAAAAUGCGAAAAAAAA | -0,05 | 0,07 |
| ANP-Oligo 153 | AAAAAAAUAGGAAAAAAAA | -0,05 | 0,05 |
| ANP-Oligo 100 | AAAAAAAGAAUAAAAAAAA | -0,04 | 0,02 |
| ANP-Oligo 169 | AAAAAAAUCUUAAAAAAAA | -0,04 | 0,05 |
| ANP-Oligo 155 | AAAAAAAUAUCAAAAAAAA | -0,03 | 0,04 |
| ANP-Oligo 010 | AAAAAAAAGUAAAAAAAAA | -0,03 | 0,04 |
| ANP-Oligo 036 | AAAAAAAAGUGAAAAAAAA | -0,02 | 0,04 |
| ANP-Oligo 082 | AAAAAAACGGUAAAAAAAA | -0,01 | 0,03 |
| ANP-Oligo 168 | AAAAAAAUCUGAAAAAAAA | -0,01 | 0,06 |

**Table3: Group 2 ssRNA-Oligonucleotides**

| Name | Sequence 5' → 3' | adjusted | SEM |
|---|---|---|---|
| | | IFN-α index | |
| ANP-Oligo 071 | AAAAAAACCUCAAAAAAAA | 0 | 0,05 |
| ANP-Oligo 182 | AAAAAAAUUACAAAAAAAA | 0,01 | 0,1 |
| ANP-Oligo 096 | AAAAAAACUUGAAAAAAAA | 0,02 | 0,06 |
| ANP-Oligo 085 | AAAAAAACGUUAAAAAAAA | 0,02 | 0,04 |
| ANP-Oligo 176 | AAAAAAAUGGCAAAAAAAA | 0,04 | 0,04 |
| ANP-Oligo 187 | AAAAAAAUUCUAAAAAAAA | 0,05 | 0,04 |
| ANP-Oligo 166 | AAAAAAAUCGUAAAAAAAA | 0,07 | 0,04 |
| ANP-Oligo 023 | AAAAAAAACUCAAAAAAAA | 0,08 | 0,06 |
| ANP-Oligo 193 | AAAAAAAUUUUAAAAAAAA | 0,13 | 0,08 |
| ANP-Oligo 188 | AAAAAAAUUGCAAAAAAAA | 0,14 | 0,04 |
| ANP-Oligo 132 | AAAAAAAGGUGAAAAAAAA | 0,18 | 0,04 |
| ANP-Oligo 106 | AAAAAAAGAGUAAAAAAAA | 0,21 | 0,07 |
| ANP-Oligo 095 | AAAAAAACUUCAAAAAAAA | 0,22 | 0,07 |
| ANP-Oligo 186 | AAAAAAAUUCGAAAAAAAA | 0,22 | 0,09 |
| ANP-Oligo 058 | AAAAAAACAGUAAAAAAAA | 0,22 | 0,09 |
| ANP-Oligo 167 | AAAAAAAUCUCAAAAAAAA | 0,23 | 0,07 |
| ANP-Oligo 189 | AAAAAAAUUGGAAAAAAAA | 0,24 | 0,12 |
| ANP-Oligo 047 | AAAAAAAAUUCAAAAAAAA | 0,27 | 0,09 |
| ANP-Oligo 135 | AAAAAAAGUAGAAAAAAAA | 0,3 | 0,04 |
| ANP-Oligo 154 | AAAAAAAUAGUAAAAAAAA | 0,32 | 0,03 |
| ANP-Oligo 136 | AAAAAAAGUAUAAAAAAAA | 0,36 | 0,07 |
| ANP-Oligo 134 | AAAAAAAGUACAAAAAAAA | 0,37 | 0,08 |
| ANP-Oligo 140 | AAAAAAAGUGCAAAAAAAA | 0,38 | 0,04 |
| ANP-Oligo 141 | AAAAAAAGUGGAAAAAAAA | 0,39 | 0,06 |
| ANP-Oligo 178 | AAAAAAAUGGUAAAAAAAA | 0,41 | 0,03 |
| ANP-Oligo 192 | AAAAAAAUUUGAAAAAAAA | 0,42 | 0,11 |
| ANP-Oligo 138 | AAAAAAAGUCGAAAAAAAA | 0,44 | 0,05 |
| ANP-Oligo 034 | AAAAAAAAGGUAAAAAAAA | 0,46 | 0,05 |
| ANP-Oligo 180 | AAAAAAAUGUGAAAAAAAA | 0,46 | 0,07 |
| ANP-Oligo 191 | AAAAAAAUUUCAAAAAAAA | 0,54 | 0,13 |
| ANP-Oligo 046 | AAAAAAAAUGUAAAAAAAA | 0,59 | 0,04 |
| ANP-Oligo 037 | AAAAAAAAGUUAAAAAAAA | 0,62 | 0,13 |
| ANP-Oligo 181 | AAAAAAAUGUUAAAAAAAA | 0,64 | 0,07 |
| ANP-Oligo 083 | AAAAAAACGUCAAAAAAAA | 0,68 | 0,15 |
| ANP-Oligo 094 | AAAAAAACUGUAAAAAAAA | 0,73 | 0,09 |
| ANP-Oligo 179 | AAAAAAAUGUCAAAAAAAA | 0,73 | 0,04 |
| ANP-Oligo 190 | AAAAAAAUUGUAAAAAAAA | 0,74 | 0,07 |
| ANP-Oligo 121 | AAAAAAAGCUUAAAAAAAA | 0,77 | 0,07 |
| ANP-Oligo 137 | AAAAAAAGUCCAAAAAAAA | 0,79 | 0,06 |
| ANP-Oligo 139 | AAAAAAAGUCUAAAAAAAA | 0,84 | 0,08 |
| ANP-Oligo 131 | AAAAAAAGGUCAAAAAAAA | 0,93 | 0,2 |
| ANP-Oligo 142 | AAAAAAAGUGUAAAAAAAA | 0,96 | 0,08 |
| ANP-Oligo 133 | AAAAAAAGGUUAAAAAAAA | 1,04 | 0,13 |
| ANP-Oligo 145 | AAAAAAAGUUUAAAAAAAA | 1,17 | 0,08 |
| ANP-Oligo 144 | AAAAAAAGUUGAAAAAAAA | 1,22 | 0,08 |
| ANP-Oligo 119 | AAAAAAAGCUCAAAAAAAA | 1,26 | 0,15 |
| ANP-Oligo 035 | AAAAAAAAGUCAAAAAAAA | 1,33 | 0,28 |
| ANP-Oligo 143 | AAAAAAAGUUCAAAAAAAA | 1,35 | 0,11 |

**Table 4: ssRNA oligonucleotides - Figure 7A**

| Name | Sequence 5' → 3' |
|---|---|
| ANP-Oligo 194 | AAGUCAAAAAAAAAAAAAA |
| ANP-Oligo 195 | AAAAAAGUCAAAAAAAAAA |
| ANP-Oligo 035 | AAAAAAAAGUCAAAAAAAA |
| ANP-Oligo 196 | AAAAAAAAAAGUCAAAAAA |
| ANP-Oligo 197 | AAAAAAAAAAAAAAGUCAA |

**Table 5: ssRNA oligonucleotides - Figure 7B**

| Name | Sequence 5' → 3' |
|---|---|
| ANP-Oligo 035 | AAAAAAAAGUCAAAAAAAA |
| ANP-Oligo 198 | AAAAAAAAGUCACAAAAAA |
| ANP-Oligo 199 | AAAAAAAAGUCAGAAAAAA |
| ANP-Oligo 200 | AAAAAAAAGUCAUAAAAAA |
| ANP-Oligo 083 | AAAAAAACGUCAAAAAAAA |
| ANP-Oligo 201 | AAAAAAACGUCACAAAAAA |
| ANP-Oligo 202 | AAAAAAACGUCAGAAAAAA |
| ANP-Oligo 203 | AAAAAAACGUCAUAAAAAA |
| ANP-Oligo 131 | AAAAAAAGGUCAAAAAAAA |
| ANP-Oligo 204 | AAAAAAAGGUCACAAAAAA |
| ANP-Oligo 205 | AAAAAAAGGUCAGAAAAAA |
| ANP-Oligo 206 | AAAAAAAGGUCAUAAAAAA |
| ANP-Oligo 179 | AAAAAAAUGUCAAAAAAAA |
| ANP-Oligo 207 | AAAAAAAUGUCACAAAAAA |
| ANP-Oligo 208 | AAAAAAAUGUCAGAAAAAA |
| ANP-Oligo 209 | AAAAAAAUGUCAUAAAAAA |

**Table 6: ssRNA oligonucleotides -- Figure 8A**

| Oligo-name | Sequence 5' → 3' |
|---|---|
| 9.2 sense | AGCUUAACCUGUCCUUCAA |
| L7A | AAAAAAACCUGUCCUUCAA |
| L8A | AAAAAAAACUGUCCUUCAA |
| L9A | AAAAAAAAAUGUCCUUCAA |
| L10A | AAAAAAAAAAGUCCUUCAA |
| L11A | AAAAAAAAAAAUCCUUCAA |
| L12A | AAAAAAAAAAAACCUUCAA |
| R9A | AGCUUAACCUAAAAAAAAA |
| R8A | AGCUUAACCUGAAAAAAAA |
| R7A | AGCUUAACCUGUAAAAAAA |
| R6A | AGCUUAACCUGUCAAAAAA |
| R5A | AGCUUAACCUGUCCAAAAA |
| R4A | AGCUUAACCUGUCCUAAAA |
| R3A | AGCUUAACCUGUCCUUAAA |

## Claims

1. A method for determining the immunostimulatory activity of a RNA oligonucleotide, comprising the steps of:
(a) complexing the RNA oligonucleotide with a complexation agent;
(b) contacting a cell with the complexed RNA oligonucleotide, wherein the cell expresses TLR7, TLR8, or both TLR7 and TLR8; and
(c) determining the amount of IFN-α produced by the cell of step (b), an increase of IFN-α production indicating immunostimulatory activity of the RNA oligonucleotide.

2. The method of claim 1, wherein the complexation agent is a polycationic peptide, preferably poly-L-arginine (poly-L-Arg).

3. The method of claim 1, wherein the cell is a peripheral blood mononuclear cell (PBMC), preferably a plasmacytoid dendritic cell (PDC).

4. A method for predicting the immunostimulatory activity of a RNA oligonucleotide, comprising the steps of:
(a) identifying all possible 3-nucleotide (3mer) motifs contained in the oligonucleotide;
(b) assigning an IFN-α point score for each individual 3mer motif;
(c) calculating the IFN-α score of the oligonucleotide by calculating the sum of the IFN-α point scores of individual 3mer motifs; and
(d) assigning to the oligonucleotide a high immunostimulatory activity if the IFN-α score is at least 23, an intermediate immunostimulatory activity if the IFN-α score is between -4 and 23, and a low immunostimulatory activity if the IFN-α score is at most -4, when n=6;
assigning to the oligonucleotide a high immunostimulatory activity if the IFN-α score is at least 26, an intermediate immunostimulatory activity if the IFN-α score is between -4 and 26, and a low immunostimulatory activity if the IFN-α score is at most -4, when n=7;
assigning to the oligonucleotide a high immunostimulatory activity if the IFN-α score is at least 28, an intermediate immunostimulatory activity if the IFN-α score is between -5 and 28, and a low immunostimulatory activity if the IFN-α score is at most -5, when n=8;
assigning to the oligonucleotide a high immunostimulatory activity if the IFN-α score is at least 30, an intermediate immunostimulatory activity if the IFN-α score is between -5 and 30, and a low immunostimulatory activity if the IFN-α score is at most -5, when n=9;
assigning to the oligonucleotide a high immunostimulatory activity if the IFN-α score is at least 1.4909 x n + 22.014, an intermediate immunostimulatory activity if the IFN-α score is between 0.005 x n² - 0.2671 x n - 3.5531 and 1.4909 x n + 22.014, and a low immunostimulatory activity if the IFN-α score is at most 0.005 x n² - 0.2671 x n - 3.5531, when n is greater than 9,
wherein n is the length of the oligonucleotide.

5. A method for preparing a RNA oligonucleotide having high immunostimulatory activity, comprising the steps of:
(a) providing candidate oligonucleotide sequence(s);
(b) identifying oligonucleotide sequence(s) with high immunostimulatory activity predicted according to the method of claim 4;
(c) preparing the RNA oligonucleotide(s) identified for high immunostimulatory activity in step (b); and
(d) optionally testing the immunostimulatory activity of the RNA oligonucleotide(s) prepared in step (c) according to the method of claim 1; and
(e) further optionally modifying the oligonucleotide(s) to optimize the immunostimulatory activity.

6. A method for preparing a RNA oligonucleotide having low immunostimulatory activity, comprising the steps of:
(a) providing candidate oligonucleotide sequence(s);
(b) identifying oligonucleotide sequence(s) with low immunostimulatory activity predicted according to the method of claim 4;
(c) preparing the RNA oligonucleotide(s) identified for low immunostimulatory activity in step (b); and
(d) optionally testing the RNA oligonucleotide(s) prepared in step (c) for the lack of immunostimulatory activity according to the method of claim 1; and
(e) further optionally modifying the oligonucleotide(s) to minimize the immunostimulatory activity.

7. A RNA oligonucleotide having immunostimulatory activity comprising at least one, preferably at least two, more preferably at least three, even more preferably at least four, of the 4-nucleotide (4mer) motifs selected from the group consisting of:
GUUC (No. 1), GUCA (No. 2), GCUC (No. 3), GUUG (No. 4), GUUU (No. 5),
GGUU (No. 6), GUGU (No. 7), GGUC (No. 8), GUCU (No. 9), GUCC (No. 10),
GCUU (No. 11), UUGU (No. 12), UGUC (No. 13), CUGU (No. 14), CGUC (No. 15),
UGUU (No. 16), GUUA (No. 17), UGUA (No. 18), UUUC (No. 19), UGUG (No. 20),
GGUA (No. 21), GUCG (No. 22), UUUG (No. 23), UGGU (No. 24), GUGG (No. 25),
GUGC (No. 26), GUAC (No. 27), GUAU (No. 28), UAGU (No. 29), GUAG (No. 30),
UUCA (No. 31), UUGG (No. 32), UCUC (No. 33), CAGU (No. 34), UUCG (No. 35),
CUUC (No. 36), GAGU (No. 37), GGUG (No. 38), UUGC (No. 39), UUUU (No. 40),
CUCA (No. 41), UCGU (No. 42), UUCU (No. 43), UGGC (No. 44), CGUU (No. 45),
CUUG (No. 46), UUAC (No. 47),
wherein the nucleotide sequences of the motifs are 5' → 3',
wherein the oligonucleotide is between 6 and 64, preferably between 12 and 50, more preferably between 14 and 40, even more preferably between 16 and 36, and most preferably between 18 and 25 nucleotides in length,
wherein at least one strand of the RNA oligonucleotide has an IFN-α score of at least 23 when n=6; at least 26 when n=7; at least 28 when n=8; at least 30 when n=9; at least 1.4909 x n + 22.014 when n is greater than 9,
wherein n is the length of the oligonucleotide,
and wherein the oligonucleotide is not 5'-UUGAUGUGUUUAGUCGCUA-3', 5'-GCACCACUAGUUGGUUGUC-3', 5'-GUUGUAGUUGUACUCCAGC-3', 5'-GCCCGUCUGUUGUGUGACUC-3', 5'-GUCUGUUGUGUG-3', 5'-GUUGUGGUUGUGGUUGUG-3'.

8. The oligonucleotide of claim 7, wherein the 4mer motifs are selected from the group consisting of number 1-19, preferably, number 1-6 of the 4mer motifs.

9. A RNA oligonucleotide having immunostimulatory activity comprising at least one, preferably at least two, more preferably at least three, even more preferably at least four, of the 4mer motifs selected from the group consisting of number 1-6 of the 4mer motifs, wherein the spacer nucleotides which are not part of any of the 4mer motif(s) are identical, and wherein the spacer nucleotide is selected from the group consisting of A, T, C, G and variants and derivatives thereof.

10. The oligonucleotide of any one of claims 7-9, wherein the oligonucleotide does not have gene silencing activity for any known mammalian gene.

11. The oligonucleotide of any one of claims 7-10, wherein the oligonucleotide is single-stranded.

12. The oligonucleotide of claim 11, wherein the oligonucleotide contains a self-complementary sequence and can form a hairpin structure.

13. The oligonucleotide of any one of claims 7-10, wherein the oligonucleotide is double-stranded.

14. The oligonucleotide of any one of claims 7-10, wherein the oligonucleotide is partially double-stranded.

15. The oligonucleotide of any one of claims 7-14, wherein the oligonucleotide is covalently linked to one or more lipophilic groups.

16. The oligonucleotide of claim 15, wherein the lipophilic group is cholesterol or a derivative thereof.

17. A RNA oligonucleotide having both gene silencing activity and high immunostimulatory activity, wherein at least one strand of the oligonucleotide has an IFN-α score of at least 1.4909 x n + 31.014,
wherein n is the length of the oligonucleotide,
wherein the oligonucleotide is an siRNA and n is between 14 and 25,
or wherein the oligonucleotide is an antisense and n is between 14 and 50.

18. A RNA oligonucleotide having gene silencing activity and low immunostimulatory activity, wherein all strand(s) of the oligonucleotide has(have) an IFN-α score of at
most 0.6075 x - 9.9484,
wherein n is the length of the oligonucleotide,
wherein the oligonucleotide is an siRNA and n is between 19 and 25,
or wherein the oligonucleotide is an antisense and n is between 18 and 50.

19. A pharmaceutical composition comprising one or more of the RNA oligonucleotides of any one of claims 7-18 and a pharmaceutically acceptable carrier.

20. The pharmaceutical composition of claim 19, further comprising a RNA complexation agent.

21. The pharmaceutical composition of claim 20, wherein the RNA complexation agent is a polycationic peptide, preferably poly-L-Arg.

22. The pharmaceutical composition of any one of claims 19-21, further comprising one or more further pharmaceutically active agents, wherein the further pharmaceutically active agent is selected from the group consisting of agents that are used to treat cancer, dermatological disorders, immune disorders, metabolic disorders, neurological disorders, ocular diseases, infections, and other hereditary and non-hereditary disorders in a mammal.

23. The pharmaceutical composition of any one of claims 19-22, wherein the composition is for administration selected from the group consisting of airway, oral, ocular, parenteral (including intraveneous, intradermal, intramuscular, intraperitoneal, and subcutaneous), rectal, vaginal and topical (including buccal and sublingual) administration.

24. Use of the RNA oligonucleotide of any one of claims 7-16 for the preparation of a pharmaceutical composition for inducing an immune response in a mammal.

25. Use of the RNA oligonucleotide of any one of claims 7-16 for the preparation of a pharmaceutical composition for preventing and/or treating a disorder selected from the group consisting of immune disorders, infections, and cancers in a mammal.

26. Use of the RNA oligonucleotide of claim 17 for the preparation of a pharmaceutical composition for preventing and/or treating a disorder selected from the group consisting of infections and cancers in a mammal.

27. Use of the RNA oligonucleotide of claim 18 for the preparation of a pharmaceutical composition for preventing and/or treating a disorder selected from the group consisting of cancer, dermatological disorders, immune disorders, metabolic disorders, neurological disorders, ocular diseases, infections, and other hereditary and non-hereditary disorders.

28. The use of claim 25 or 27, wherein the immune disorder is selected from the group consisting of allergy, autoimmune disorders, inflammatory disorders, and immunodeficiency.

29. The use of any one of claims 25-27, wherein the infection is selected from the group consisting of viral infections, bacterial infections, anthrax, parasitic infections, fungal infections and prion infection.

30. The use of claim 29, wherein the viral infection is selected from the group consisting of chornic hepatitis B, chornic hepatitis C, HIV infection, RSV infection, HSV infection, VSV infection, CMV infection and influenza infection.

31. The use of any one of claims 25-27, wherein the cancer is selected from the group consisting of hairy cell leukemia, chronic myelogenous leukemia, cutaneous T-cell leukemia, chronic myeloid leukemia, non-Hodgkin's lymphoma, multiple myeloma, follicular lymphoma, malignant melanoma, squamous cell carcinoma, renal cell carcinoma, prostate carcinoma, bladder cell carcinoma, breast carcinoma, ovarian carcinoma, non-small cell lung cancer, small cell lung cancer, hepatocellular carcinoma, basaliom, colon carcinoma, cervical dysplasia and AIDS-related Kaposi's sarcoma.

32. The use of any one of claims 24-31, wherein the pharmaceutical composition is for administration selected from the group consisting of airway, oral, ocular, parenteral (including intraveneous, intradermal, intramuscular, intraperitoneal, and subcutaneous), rectal, vaginal and topical (including buccal and sublingual) administration.

33. The use of any one of claims 24-32, wherein the pharmaceutical composition is for use in combination with one or more treatments of disorders selected from the group consisting of treatments for cancer, dermatological disorders, immune disorders, metabolic disorders, neurological disorders, ocular diseases, infections, and other hereditary and non-hereditary disorders in a mammal.

34. The use of any one of claims 24-33, wherein the mammal is human.

35. An in vitro method of inducing IFN-α production by a mammalian cell, comprising the steps of:
(a) complexing the RNA oligonucleotide of any one of claims 7-16 with a complexation agent; and
(b) contacting the cell with the complex prepared in step (a).

36. The method of claim 35, wherein the mammalian cell is selected from the group consisting of freshly isolated PBMC, freshly isolated PDC, and a cell line capable of producing IFN-α.

37. The method of claim 35 or 36, wherein the complexation agent is a polycationic peptide, preferably poly-L-Arg.

38. An in vitro method of activating mammalian dendritic cells comprising the steps of:
(a) complexing the RNA oligonucleotide of any one of claims 7-16 with a complexation agent;
(b) contacting dendritic cells isolated from a donor mammal with the complexed RNA oligonucleotide; and
(c) contacting the dendritic cells with an antigen.

39. Use of the in vitro activated dendritic cells prepared according to the method of claim 38 for the preparation of a medicament for inducing an immune response in a mammal, wherein the in vitro activated dendritic cells are for transfer into a recipient that is the same or different from the donor.
